(19) Europäisches Patentamt
European Patent Office
Office européen des brevets

(11) EP 2 778 237 A1

(12) **EUROPEAN PATENT APPLICATION**

(43) Date of publication:
**17.09.2014 Bulletin 2014/38**

(51) Int Cl.:
***C12Q 1/68*** *(2006.01)* *G06F 19/18* *(2011.01)*

(21) Application number: **14168194.0**

(22) Date of filing: **11.01.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **13.01.2011 US 201161432468 P**

(62) Document number(s) of the earlier application(s) in accordance with Art. 76 EPC:
**12733946.3 / 2 619 587**

(71) Applicant: **Industrial Technology Research Institute**
**Chutung Hsinchu 31040 (TW)**

(72) Inventors:
- **Wang, Ting-Huei**
**300 Hsinchu City (TW)**
- **Li, Yen-Peng**
**106 Taipei (CN)**
- **Kuo, Pei-Fen**
**242 Taipei (TW)**
- **Chen, Shih-Ya**
**439 Taichung (TW)**
- **Lin, Chia-Ju**
**231 Taipei (TW)**
- **Lee, Jenq-Chang**
**700 Tainan (TW)**
- **Lee, Angelina Huai-Lo**
**310 Hsinchu County (TW)**

(74) Representative: **Fuchs Patentanwälte Partnerschaft mbB**
**Patentanwälte**
**Westhafenplatz 1**
**60327 Frankfurt am Main (DE)**

Remarks:
This application was filed on 13-05-2014 as a divisional application to the application mentioned under INID code 62.

(54) **Biomarkers for recurrence prediction of colorectal cancer**

(57) Methods for determining the likelihood of colorectal cancer (CRC) recurrence in a subject that involve measuring the expression level of two or more micro ribonucleic acids (miRNAs) in a biological sample comprising CRC tumor cells from said subject and using the normalized, measured expression levels to determine the likelihood of colorectal cancer recurrence for said subject. In the methods, the normalized expression levels of specific miRNAs are weighted by their contribution to CRC recurrence to calculate the likelihood of CRC recurrence. Kits for measuring the expression level of specific miRNAs that can be used in determining the likelihood of CRC recurrence are also provided.

EP 2 778 237 A1

SEQUENCE LISTING

<110> INDUSTRIAL TECHNOLOGY RESEARCH INSTITUTE

<120> BIOMARKERS FOR RECURRENCE PREDICTION OF COLORECTAL CANCER

<130> 0941-2458PWO

<140> PCT/US2012/020949
<141> 2012-01-11

<150> US 61/432,468
<151> 2011-01-13

<160> 18

<170> PatentIn version 3.5

<210> 1
<211> 22
<212> RNA
<213> Artificial Sequence

<220>
<223> Synthetic: hsa-miR-363*

<400> 1
cggguggauc acgaugcaau uu 22

<210> 2
<211> 22
<212> RNA
<213> Artificial Sequence

<220>
<223> Synthetic: hsa-miR-1255b

<400> 2
cggaugagca aagaaagugg uu 22

<210> 3
<211> 19
<212> RNA
<213> Artificial Sequence

<220>
<223> Synthetic: hsa-miR-566

<400> 3
gggcgccugu gaucccaac 19

<210> 4
<211> 22
<212> RNA
<213> Artificial Sequence

<220>
<223> Synthetic: hsa-miR-1265

<400> 4

caggaugugg ucaaguguug uu 22

<210> 5
<211> 22
<212> RNA
<213> Artificial Sequence

<220>
<223> Synthetic: hsa-miR-127-5p

<400> 5
cugaagcuca gagggcucug au 22

<210> 6
<211> 22
<212> RNA
<213> Artificial Sequence

<220>
<223> Synthetic: hsa-miR-338-5p

<400> 6
aacaauaucc uggugcugag ug 22

<210> 7
<211> 22
<212> RNA
<213> Artificial Sequence

<220>
<223> Synthetic: hsa-miR-550a*

<400> 7
ugucuuacuc ccucaggcac au 22

<210> 8
<211> 21
<212> RNA
<213> Artificial Sequence

<220>
<223> Synthetic: hsa-miR-588

<400> 8
uuggccacaa uggguuagaa c 21

<210> 9
<211> 22
<212> RNA
<213> Artificial Sequence

<220>
<223> Synthetic: hsa-miR-651

<400> 9
uuuaggauaa gcuugacuuu ug 22

<210> 10
<211> 22
<212> RNA
<213> Artificial Sequence

<220>
<223> Synthetic: hsa-miR-223*

<400> 10
cguguauuug acaagcugag uu 22

<210> 11
<211> 22
<212> RNA
<213> Artificial Sequence

<220>
<223> Synthetic: hsa-miR-518b

<400> 11
caaagcgcuc cccuuuagag gu 22

<210> 12
<211> 21
<212> RNA
<213> Artificial Sequence

<220>
<223> Synthetic: hsa-miR-629

<400> 12
uggguuuacg uugggagaac u 21

<210> 13
<211> 22
<212> RNA
<213> Artificial Sequence

<220>
<223> Synthetic: hsa-miR-885-5p

<400> 13
uccauuacac uacccugccu cu 22

<210> 14
<211> 22
<212> RNA
<213> Artificial Sequence

<220>
<223> Synthetic: hsa-miR-139-3p

<400> 14
ucuacagugc acgugucucc ag 22

<210> 15
<211> 22
<212> RNA

```
<213>  Artificial Sequence

<220>
<223>  Synthetic: hsa-miR-655

<400>  15
auaauacaug guuaaccucu uu                                                22


<210>  16
<211>  19
<212>  RNA
<213>  Artificial Sequence

<220>
<223>  Synthetic: hsa-miR-1290

<400>  16
uggauuuuug gaucaggga                                                    19


<210>  17
<211>  22
<212>  RNA
<213>  Artificial Sequence

<220>
<223>  Synthetic: hsa-miR-450b-5p

<400>  17
uuuugcaaua uguuccugaa ua                                                22


<210>  18
<211>  19
<212>  RNA
<213>  Artificial Sequence

<220>
<223>  Synthetic: hsa-miR-1224-5p

<400>  18
gugaggacuc gggaggugg                                                    19
```

## Description

This application is a Divisional application of European patent application No. 12733946.3, filed January 11, 2012, and entitled "biomarkers for recurrence prediction of colorectal cancer", which claims priority under 35 U.S.C. § 119(e) to U.S. Provisional Application No. 61/432,468 filed on January 13, 2011.

TECHNICAL FIELD

The disclosure provides methods for predicting the recurrence of colorectal cancer in a subject.

BACKGROUND

Colorectal cancer (CRC) is cancer that originates in either the large intestine (colon) or the rectum. CRC is the third most common cancer in men and the second most common in women worldwide. In 2008, it was estimated that about 608,000 deaths worldwide could be attributed to CRC annually, accounting for 8% of all cancer deaths, and making CRC the fourth most common cause of death from cancer worldwide. CRC is the number two cause of cancer-related death in the United States and the European Union, accounting for 10% of all cancer-related deaths in the U.S. and the E.U. The American Cancer Society (ACS) estimates that there will be about 100,000 new cases of colon cancer and nearly 40,000 new cases of rectal cancer in the U.S. in 2011. ACS further estimates that there will be nearly 50,000 CRC related deaths in the U.S. in 2011.

Colon cancer and rectal cancer may represent the identical disease or similar diseases at the molecular level, but surgery for rectal cancer is more complicated than that for colon cancer due to issues of anatomy. Possibly for this reason, the rate of local recurrence following surgical removal of cancerous tissue is significantly higher for rectal cancer than for colon cancer, and therefore, approaches to treating the two cancers are significantly different.

Clinical tests that help oncologists in making well-reasoned treatment decisions are invaluable. Oncologists are repeatedly confronted with the decision of whether to treat or forego treatment of a patient with chemotherapeutic agents. Current therapeutic agents for cancer generally have modest efficacy accompanied with substantial toxicity. Thus, it would be useful for an oncologist to know the likelihood of metastatic recurrence in a patient that has undergone resection of a primary tumor in making treatment decisions. Armed with such information, high risk patients could be selected for chemotherapy and patients unlikely to have cancer recurrence could be spared unnecessary exposure to the adverse events associated with chemotherapy.

There are two classification systems used to track progression of colorectal cancer, the modified Duke's (or Astler-Coller) staging system (Stages A-D) (Astler V B, Coller F A., Ann Surg 1954; 139:846-52), and more recently TNM staging (Stages I-IV) as developed by the American Joint Committee on Cancer (AJCC Cancer Staging Manual, 6th Edition, Springer-Verlag, New York, 2002). Both systems evaluate tumor progression by measuring the spread of the primary tumor through layers of the colon or rectal wall to adjacent organs, lymph nodes and distant sites. Estimates of recurrence risk and treatment decisions in colon cancer are currently based primarily on tumor stage.

The decision whether to administer adjuvant chemotherapy is not straightforward. There are approximately 33,000 newly diagnosed Stage II colorectal cancers each year in the United States. Nearly all of these patients are treated by performing a surgical resection of the tumor and subsequent to resection about 40% of the surgical patients are treated with 5-fluorouracil (5-FU) based chemotherapy. The five-year survival rate for Stage II colon cancer patients treated with surgery alone is approximately 80%. Standard adjuvant treatment with 5-FU+leucovorin (leucovorin-mediated fluorouracil) yields an absolute improvement in the 5-year survival rate of only 2-4%, and such chemotherapy is associated with significant toxicity.

The benefit of chemotherapy in treating Stage III colon cancer is much more apparent than in treating Stage II colon cancer. A large proportion of the patients diagnosed with Stage III colon cancer receive 5-FU-based adjuvant chemotherapy. The reported absolute benefit of chemotherapy in Stage III colon cancer varies depending on the treatment regimen. An increase in survival rate for patients treated with 5-FU+leucovorin has been reported as being about 18%, and about 24% for patients treated with 5-FU+leucovorin+oxaliplatin. Current standard-of-care chemotherapy treatment for Stage III colon cancer patients is moderately effective, achieving an improvement in 5-year survival rate of from about 50% (surgery alone) to about 65% (5-FU+leucovorin) or 70% (5-FU+leucovorin+oxaliplatin). Treatment with 5-FU+leucovorin alone or in combination with oxaliplatin is accompanied by a range of adverse side-effects, including toxic death in approximately 1% of patients treated. It has not been established whether a subset of Stage III patients (overall untreated 5-year survival about 50%) exists for which recurrence risk resembles that observed for Stage II patients (overall untreated 5-year survival about 80%).

Staging of rectal tumors is carried out using similar criteria to those used for colon tumor staging. Stage II/III rectal tumors bear a reasonable correlation to Stage II/III colon tumors as to their state of progression. As noted above, the rate of local recurrence and other aspects of prognosis differ between rectal cancer and colon cancer, and these differences may arise from difficulties in accomplishing total resection of rectal tumors.

Thus, given the toxicity associated with existing chemotherapies information regarding the likelihood of recurrence of CRC following surgical resection would be useful to an oncologist in deciding whether such chemotherapy would be of likely benefit to the patient.

Clinical tests for cancer are generally single analyte tests. Single analyte tests fail to capture complex relationships that can occur between a number of different markers correlated with a particular cancer. Moreover, many existing cancer clinical tests are not quantitative, relying on immunohistochemistry. Immunohistochemistry results can differ between laboratories, in part because the reagents are not standardized, and in part because the interpretations are subjective and cannot be easily quantified.

Ribonucleic acid (RNA)-based tests have not often been used in clinical testing, because RNA in tissue samples tends to be easily degraded. However, RNA-based methods have the potential to permit simultaneous observation of the expression of multiple cancer markers from a small amount of material *(i.e.,* tissue or cells) taken from a cancer patient.

A microRNA (abbreviated miRNA) is a short ribonucleic acid (RNA) molecule found in all eukaryotic cells, except those of fungi, algae, and marine plants. A miRNA molecule has very few nucleotides (an average of 22) compared with other RNAs. miRNAs are post-transcriptional regulators that bind to complementary sequences on target messenger RNA transcripts (mRNAs), usually resulting in translational repression or target degradation and gene silencing. The human genome may encode over 1000 miRNAs, which may target about 60% of mammalian genes and are abundant in many human cell types.

miRNAs were not recognized as a distinct class of biological regulators with conserved functions until the early 2000s. Since then, miRNA research has revealed multiple roles in negative regulation (transcript degradation and sequestration, translational suppression) and possible involvement in positive regulation (transcriptional and translational activation). By affecting gene regulation, miRNAs are likely to be involved in most biological processes. Different sets of expressed miRNAs are found in different cell types and tissues. Aberrant expression of miRNAs has been implicated in numerous disease states.

## SUMMARY

Some aspects of the present invention are drawn to methods for determining the likelihood of colorectal cancer (CRC) recurrence in a subject. Such methods may include measuring the expression level of two or more micro ribonucleic acids (miRNAs) selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p in a biological sample comprising CRC tumor cells obtained from said subject. The measured expression levels may be used to calculate a recurrence score by a method including weighting the expression levels of the miRNAs by their contribution to CRC recurrence. The likelihood of colorectal cancer recurrence for the subject can then be determined using the recurrence score, in some embodiments of the present invention. Some of the claimed methods may further include preparing a report including the recurrence score and/or the determination of the likelihood of CRC recurrence made using the recurrence score.

Certain embodiments of the present invention are drawn to methods for determining the likelihood of colorectal cancer (CRC) recurrence in a subject. Such methods may include measuring the expression level of two or more micro ribonucleic acids (miRNAs) selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p in a biological sample comprising CRC tumor cells obtained from the subject. The measured expression levels may be normalized and used to calculate a recurrence score by a method including weighting the normalized expression levels of the miRNAs by their contribution to CRC recurrence. The likelihood of colorectal cancer recurrence for the subject can then be determined using the recurrence score, in some embodiments of the present invention. Some of the claimed methods may further include preparing a report including the recurrence score and/or the determination of the likelihood of CRC recurrence made using the recurrence score.

Certain embodiments of the invention are drawn to kits for predicting the recurrence of CRC in a subject. In some embodiments, a kit consists of at least two reverse transcription primers for specifically reverse transcribing at least two miRNAs selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p into cDNA.

In certain embodiments, a kit can comprise (a) at least two reverse transcription primers for specifically reverse transcribing at least two miRNAs selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p into cDNA; (b) at least two probes that specifically bind to cDNAs reverse transcribed from the miRNAs, wherein the probes are suitable for use in real-time polymerase chain reaction, for example, quantitative real time polymerase chain reaction (qRT-PCR or Q-PCR) for quantifying the miRNAs present; and (c) a reverse transcription primer for specifically reverse transcribing at least one noncoding RNA and a probe that specifically binds a cDNA reverse transcribed from the at least one noncoding RNA, wherein the probe is suitable for use in real time polymerase chain reaction, for example, quantitative real time polymerase chain reaction (qRT-PCR or Q-PCR) for quantifying the noncoding RNA present.

Some embodiments of the invention are drawn to methods for determining the likelihood of the recurrence of colorectal cancer in a subject involving collecting at the time of colorectal cancer tumor removal surgery from the subject (a) a sample of a colorectal cancer tumor and (b) a paired sample of non-tumorous tissue that is of the same type of tissue out of which the tumor was formed. Total ribonucleic acid (RNA) can then be extracted from each of samples (a) and (b). Using the extracted total RNA reverse transcription and quantitative real-time polymerase chain reaction (qRT-PCR or Q-PCR) can be performed for each of samples (a) and (b) to determine the normalized level of expression of each of the miRNAs of hsa-miR-1224-5p; hsa-miR-518b; hsa-miR-629; hsa-miR-885-5p; hsa-miR-139-3p; and hsa-miR-223*. The expression level of the miRNAs can be normalized relative to level of expression of noncoding RNAs (ncRNAs) RNU6B and RNU44 in the samples. Given the normalized expression levels, in certain aspects of the present invention, the probability that the subject will have a recurrence of colorectal cancer may be calculated according to logistic regression analysis.

Certain embodiments of the present invention are drawn to methods for determining the likelihood of the recurrence of colorectal cancer in a subject comprising (a) providing a sample from the subject containing small RNA; (b) detecting expression of each of the miRNAs of hsa-miR-1224-5p; hsa-miR-518b; hsa-miR-629; hsa-miR-885-5p; hsa-miR-139-3p; hsa-miR-223*; hsa-miR-655; hsa-miR-1290; and hsa-miR-450b-5p; and (c) calculating the probability that the subject will have a recurrence of colorectal cancer according to logistic regression analysis.

Some embodiments of the present invention are drawn to methods for determining the likelihood of the recurrence of colorectal cancer in a subject comprising (a) providing a sample from the subject containing small RNA; (b) detecting expression of each of the miRNAs of hsa-miR-1224-5p; hsa-miR-518b; hsa-miR-629; hsa-miR-885-5p; hsa-miR-139-3p; and hsa-miR-223*; and (c) calculating the probability that the subject will have a recurrence of colorectal cancer according to logistic regression analysis.

BRIEF DESCRIPTION OF THE DRAWINGS

Figure 1 is a schematic of one embodiment of the invention.

Figure 2-1 is a diagram showing an Area Under the Receiver Operating Characteristic (AUROC) curve and disease-free survival analysis of a CRC recurrence prediction model. In order to achieve Negative Predictive Value (NPV)=1 and Sensitivity=1.00, 6 out of 19 candidates were selected to build a predictive model using a logistic regression method. Figure 2-1a is results obtained from 65 patients in a training dataset, while the cut-off value of recurrence score set of 0.1830; Figure 2-1b shows results obtained from 15 patients in a testing dataset, while the cut-off value of recurrence score set of 0.1830 .

Figure 3-1 is a diagram showing an AUROC curve and disease-free survival analysis of a CRC recurrence prediction model. In order to achieve Positive Predictive Value (PPV)=1 and Specificity=1.00, 6 out of 19 candidates were selected to build a predictive model using a logistic regression method. Figure 3-1a shows results obtained from 65 patients in a training dataset, while the cut-off value of recurrence score set of 0.2331; Figure 3-1b shows results obtained from 15 patients in a testing dataset, while the cut-off value of recurrence score set of 0.2331.

Figures 4-49 present data derived from qPCR, normalized using RNU6B and RNU44; ("tumor": normalized qPCR Ct derived from tumor tissue; "normal": normalized qPCR Ct derived from paired non-tumorous tissue). Figures 50-54 present data derived from qPCR, without normalized. ("tumor": raw qPCR Ct derived from tumor tissue; "normal":raw qPCR Ct derived from paired non-tumorous tissue). Figures 55-60 present data derived from microarray experiments, each candidate represent: tumor tissue array intensity/ normal tissue array intensity). The specifics of Figures 4-60 are detailed below.

Figure 4 is a diagram showing an AUROC curve and disease free analysis of a CRC recurrence prediction model of one embodiment of the present invention.

Figure 5 is a diagram showing an AUROC curve and disease free analysis of a CRC recurrence prediction model of one embodiment of the present invention.

Figure 6 is a diagram showing an AUROC curve and disease free analysis of a CRC recurrence prediction model of one embodiment of the present invention.

Figure 7 is a diagram showing an AUROC curve and disease free analysis of a CRC recurrence prediction model of one embodiment of the present invention.

Figure 8 is a diagram showing an AUROC curve and disease free analysis of a CRC recurrence prediction model of one embodiment of the present invention.

Figure 9 is a diagram showing an AUROC curve and disease free analysis of a CRC recurrence prediction model of one embodiment of the present invention.

Figure 10 is a diagram showing an AUROC curve and disease free analysis of a CRC recurrence prediction model of one embodiment of the present invention.

Figure 11 is a diagram showing an AUROC curve and disease free analysis of a CRC recurrence prediction model of one embodiment of the present invention.

Figure 12 is a diagram showing an AUROC curve and disease free analysis of a CRC recurrence prediction model of one embodiment of the present invention.

Figure 13 is a diagram showing an AUROC curve and disease free analysis of a CRC recurrence prediction model of one embodiment of the present invention.

Figure 14 is a diagram showing an AUROC curve and disease free analysis of a CRC recurrence prediction model of one embodiment of the present invention.

Figure 15 is a diagram showing an AUROC curve and disease free analysis of a CRC recurrence prediction model of one embodiment of the present invention.

Figure 16 is a diagram showing an AUROC curve and disease free analysis of a CRC recurrence prediction model of one embodiment of the present invention.

Figure 17 is a diagram showing an AUROC curve and disease free analysis of a CRC recurrence prediction model of one embodiment of the present invention.

Figure 18 is a diagram showing an AUROC curve and disease free analysis of a CRC recurrence prediction model of one embodiment of the present invention.

Figure 19 is a diagram showing an AUROC curve and disease free analysis of a CRC recurrence prediction model of one embodiment of the present invention.

Figure 20 is a diagram showing an AUROC curve and disease free analysis of a CRC recurrence prediction model of one embodiment of the present invention.

Figure 21 is a diagram showing an AUROC curve and disease free analysis of a CRC recurrence prediction model of one embodiment of the present invention.

Figure 22 is a diagram showing an AUROC curve and disease free analysis of a CRC recurrence prediction model of one embodiment of the present invention.

Figure 23 is a diagram showing an AUROC curve and disease free analysis of a CRC recurrence prediction model of one embodiment of the present invention.

Figure 24 is a diagram showing an AUROC curve and disease free analysis of a CRC recurrence prediction model of one embodiment of the present invention.

Figure 25 is a diagram showing an AUROC curve and disease free analysis of a CRC recurrence prediction model of one embodiment of the present invention.

Figure 26 is a diagram showing an AUROC curve and disease free analysis of a CRC recurrence prediction model of one embodiment of the present invention.

Figure 27 is a diagram showing an AUROC curve and disease free analysis of a CRC recurrence prediction model of one embodiment of the present invention.

Figure 28 is a diagram showing an AUROC curve and disease free analysis of a CRC recurrence prediction model of one embodiment of the present invention.

Figure 29 is a diagram showing an AUROC curve and disease free analysis of a CRC recurrence prediction model of one embodiment of the present invention.

Figure 30 is a diagram showing an AUROC curve and disease free analysis of a CRC recurrence prediction model of one embodiment of the present invention.

Figure 31 is a diagram showing an AUROC curve and disease free analysis of a CRC recurrence prediction model of one embodiment of the present invention.

Figure 32 is a diagram showing an AUROC curve and disease free analysis of a CRC recurrence prediction model of one embodiment of the present invention.

Figure 33 is a diagram showing an AUROC curve and disease free analysis of a CRC recurrence prediction model of one embodiment of the present invention.

Figure 34 is a diagram showing an AUROC curve and disease free analysis of a CRC recurrence prediction model of one embodiment of the present invention.

Figure 35 is a diagram showing an AUROC curve and disease free analysis of a CRC recurrence prediction model of one embodiment of the present invention.

Figure 36 is a diagram showing an AUROC curve and disease free analysis of a CRC recurrence prediction model of one embodiment of the present invention.

Figure 37 is a diagram showing an AUROC curve and disease free analysis of a CRC recurrence prediction model of one embodiment of the present invention.

Figure 38 is a diagram showing an AUROC curve and disease free analysis of a CRC recurrence prediction model of one embodiment of the present invention.

Figure 39 is a diagram showing an AUROC curve and disease free analysis of a CRC recurrence prediction model of one embodiment of the present invention.

Figure 40 is a diagram showing an AUROC curve and disease free analysis of a CRC recurrence prediction model of one embodiment of the present invention.

Figure 41 is a diagram showing an AUROC curve and disease free analysis of a CRC recurrence prediction model of one embodiment of the present invention.

Figure 42 is a diagram showing an AUROC curve and disease free analysis of a CRC recurrence prediction model of one embodiment of the present invention.

Figure 43 is a diagram showing an AUROC curve and disease free analysis of a CRC recurrence prediction model of one embodiment of the present invention.

Figure 44 is a diagram showing an AUROC curve and disease free analysis of a CRC recurrence prediction model of one embodiment of the present invention.

Figure 45 is a diagram showing an AUROC curve and disease free analysis of a CRC recurrence prediction model of one embodiment of the present invention.

Figure 46 is a diagram showing an AUROC curve and disease free analysis of a CRC recurrence prediction model of one embodiment of the present invention.

Figure 47 is a diagram showing an AUROC curve and disease free analysis of a CRC recurrence prediction model of one embodiment of the present invention.

Figure 48 is a diagram showing an AUROC curve and disease free analysis of a CRC recurrence prediction model of one embodiment of the present invention.

Figure 49 is a diagram showing an AUROC curve and disease free analysis of a CRC recurrence prediction model of one embodiment of the present invention.

Figure 50 is a diagram showing an AUROC curve and disease free analysis of a CRC recurrence prediction model of one embodiment of the present invention.

Figure 51 is a diagram showing an AUROC curve and disease free analysis of a CRC recurrence prediction model of one embodiment of the present invention.

Figure 52 is a diagram showing an AUROC curve and disease free analysis of a CRC recurrence prediction model of one embodiment of the present invention.

Figure 53 is a diagram showing an AUROC curve and disease free analysis of a CRC recurrence prediction model of one embodiment of the present invention.

Figure 54 is a diagram showing an AUROC curve and disease free analysis of a CRC recurrence prediction model of one embodiment of the present invention.

Figure 55 is a diagram showing an AUROC curve and disease free analysis of a CRC recurrence prediction model of one embodiment of the present invention.

Figure 56 is a diagram showing an AUROC curve and disease free analysis of a CRC recurrence prediction model of one embodiment of the present invention.

Figure 57 is a diagram showing an AUROC curve and disease free analysis of a CRC recurrence prediction model of one embodiment of the present invention.

Figure 58 is a diagram showing an AUROC curve and disease free analysis of a CRC recurrence prediction model of one embodiment of the present invention.

Figure 59 is a diagram showing an AUROC curve and disease free analysis of a CRC recurrence prediction model of one embodiment of the present invention.

Figure 60 is a diagram showing an AUROC curve and disease free analysis of a CRC recurrence prediction model of one embodiment of the present invention.

## DETAILED DESCRIPTION

In the following detailed description, for purposes of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the description.

One skilled in the art will recognize many methods and materials similar or equivalent to those described herein, which could be used in the practice of the present invention. Indeed, the present invention is in no way limited to the methods and materials described. For purposes of the present invention, the following terms are defined below.

"MicroRNA" and "miRNA" as used herein includes microRNAs registered in the miRBase (microRNA database mirbase.org). miRBase is the primary online repository for all microRNA sequences and annotation. The current release (miRBase 17) contains over 16000 microRNA gene loci in over 150 species, and over 19 000 distinct mature microRNA sequences. "MicroRNA" and "miRNA" as used herein include both precursor miRNAs and mature miRNA products.

The term "tumor," as used herein, refers to all neoplastic cell growth and proliferation, whether malignant or benign, and all pre-cancerous and cancerous cells and tissues.

The terms "cancer" and "cancerous" refer to or describe the physiological condition in mammals that is typically characterized by unregulated cell growth. Colorectal cancer is one type of cancer. The term "colorectal cancer" is used in the broadest sense and refers to (1) all stages and all forms of cancer arising from epithelial cells of the large intestine and/or rectum and/or (2) all stages and all forms of cancer affecting the lining of the large intestine and/or rectum. Colorectal cancer (CRC) is cancer that originates in either the large intestine (colon) or the rectum. Colon cancer and rectal cancer may represent the identical disease or similar diseases at the molecular level.

In the staging systems used for classification of colorectal cancer, the colon and rectum are treated as one organ. According to the tumor, node, and metastasis (TNM) staging system of the American Joint Committee on Cancer (AJCC) (Greene et al. (eds.), AJCC Cancer Staging Manual. 6th Ed. New York, N.Y.: Springer; 2002), the various stages of colorectal cancer are defined as follows:

Tumor: T1: tumor invades submucosa; T2: tumor invades muscularis propria; T3: tumor invades through the muscularis propria into the subserose, or into the pericolic or perirectal tissues; T4: tumor directly invades other organs or structures, and/or perforates.

Node: N0: no regional lymph node metastasis; N1: metastasis in 1 to 3 regional lymph nodes; N2: metastasis in 4 or more regional lymph nodes.

Metastasis: M0: mp distant metastasis; M1: distant metastasis present.

Stage groupings: Stage I: T1 N0 M0; T2 N0 M0; Stage II: T3 N0 M0; T4 N0 M0; Stage III: any T, N1-2; M0; Stage IV: any T, any N, M1.

According to the Modified Duke Staging System, the various stages of colorectal cancer are defined as follows:

Stage A: the tumor penetrates into the mucosa of the bowel wall but not further. Stage B: tumor penetrates into and through the muscularis propria of the bowel wall; Stage C: tumor penetrates into but not through muscularis propria of the bowel wall, there is pathologic evidence of colorectal cancer in the lymph nodes; or tumor penetrates into and through the muscularis propria of the bowel wall, there is pathologic evidence of cancer in the lymph nodes; Stage D: tumor has spread beyond the confines of the lymph nodes, into other organs, such as the liver, lung or bone.

Prognostic factors are those variables related to the natural history of colorectal cancer, which influence the recurrence rates and outcome of patients once they have developed colorectal cancer. Clinical parameters that have been associated with a worse prognosis include, for example, lymph node involvement, and high grade tumors. Prognostic factors are frequently used to categorize patients into subgroups with different baseline relapse risks.

The term "prediction" is used herein to refer to the likelihood that a patient will have a recurrence of CRC. The predictive methods of the present invention can be used clinically to make treatment decisions by choosing the most appropriate treatment modalities for any particular patient.

The term "subject" or "patient" refers to a mammal being treated. In an embodiment of the present invention the mammal is a human.

The term "differentially expressed biomarker" refers to a biomarker *(i.e.,* miRNA, among others) whose expression is activated to a higher or lower level in a subject suffering from a disease, specifically cancer, such as CRC, relative to its expression in a normal or control subject or relative to noncancerous tissue taken from the subject. The term also includes biomarkers whose expression is activated to a higher or lower level at different stages of the same disease. Such differences may be evidenced by a change in precursor miRNAs or mature miRNAs.

Certain embodiments of the present invention are drawn to methods for determining the likelihood of colorectal cancer (CRC) recurrence in a subject comprising measuring the expression level of two or more micro ribonucleic acids (miRNAs) selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p in a biological sample comprising CRC tumor cells obtained from said subject. The measured expression levels can be normalized and a recurrence score can be calculated by a method comprising weighting the normalized expression levels of the miRNAs by contribution to CRC recurrence. The likelihood of CRC recurrence in the subject can be determined using the recurrence score. In some embodiments of the present invention, the biological sample can be fresh or frozen tumor tissue from the subject or cells taken from such a tissue sample. The biological sample may be a paired non-tumoral tissue in some embodiments. The biological sample contains small RNAs in certain embodiments of the present invention.

Certain embodiments of the present invention are drawn to methods for determining the likelihood of the recurrence of colorectal cancer in a subject comprising: collecting at the time of colorectal tumor removal surgery from the subject (a) a sample of a colorectal tumor and (b) a paired sample of non-tumorous tissue that is of the same type of tissue out of which the tumor was formed. Total RNA is extracted from extracting total ribonucleic acid (RNA) from each of samples (a) and (b) and reverse transcription and quantitative real-time polymerase chain reaction (qRT-PCR) are performed with the extracted RNA for each of samples (a) and (b). The normalized level of expression of each of the miRNAs of hsa-miR-1224-5p; hsa-miR-518b; hsa-miR-629; hsa-miR-885-5p; hsa-miR-139-3p; and hsa-miR-223* are determined and the expression levels for the miRNAs can be normalized relative to the level of expression of noncoding RNAs (ncRNAs) RNU6B and RNU44 in the samples. The probability that the subject will have a recurrence of colorectal cancer can be calculated according to the equation:

$$\text{Recurrence score} = \exp(\ y)/(1 + \exp(\ y))$$

The recurrence score can be determined according to an equation derived from statistical analyses described below.

In certain embodiments of the invention, the expression levels of the miRNAs may be measured by a method including (a) reverse transcription of miRNA and a quantitative real-time polymerase chain reaction (qRT-PCR or Q-PCR) or (b) microarray analysis. Further, in some embodiments the measured expression levels of miRNAs may be normalized relative to the expression levels of one or more noncoding ribonucleic acids (ncRNAs) or normalized to total input amount of RNA. The individual contribution of each miRNA expression level measured may be weighted separately in calculating the recurrence score and/or the likelihood of CRC recurrence for a subject, in certain embodiments of the present invention. In certain aspects of the claimed methods, the normalized expression levels of specified miRNAs can be analyzed using Kaplan-Meier survival curves.

The term "subject" or "patient" refers to a mammal being treated. The subject can be a mammal in the claimed methods, and the mammal may be a human in certain claimed methods. In some aspects of the claimed methods, the biological sample used in determining miRNA expression levels can be fresh tumor tissue, for instance, fresh CRC tumor tissue. The fresh tumor tissue can be obtained during surgical resection or from a biopsy performed on a subject in some embodiments of the present invention.

Fresh or frozen samples of colorectal cancer tissues together and, optionally, paired non-tumoral tissues can be obtained from subjects for use in the present invention. The paired non-tumoral tissues can be of the same type of tissue out of which a cancerous CRC tumor was formed. In some aspects of the present invention, the samples used are frozen samples of a CRC tumor and a paired non-tumoral tissue from which CRC tumor was formed.

The tissue sample(s) can be taken at the time of surgery/resection of CRC in the subject. Alternatively, the sample(s) can be obtained by biopsy, such as an aspiration biopsy.

The methods of the claimed invention can include extracted RNA from a tissue sample (cancerous or noncancerous) from a subject. The first step for determining expression levels of miRNAs is the isolation of RNA from a target sample. The RNA can be extracted by methods known in the art. Total RNA can be extracted from a tissue sample or cells.

The starting material is typically total RNA isolated from human tumors or tumor cell lines, and corresponding normal tissues or cell lines, respectively. Thus RNA can be isolated from a primary tumor of the colon or rectum, or tumor cell lines. If the source of RNA is a primary tumor, RNA can be extracted, for example, from frozen or fresh tissue samples.

General methods for RNA (including, mRNA, miRNA, noncoding RNA (ncRNA), ribosomal RNA (rRNA), among others) extraction are well known in the art and are disclosed in standard textbooks of molecular biology, including Ausubel et al., Current Protocols of Molecular Biology, John Wiley and Sons (1997). In particular, RNA isolation can be performed using a purification kit, buffer set and protease from commercial manufacturers, such as Qiagen, Valencia, CA, USA, according to the manufacturer's instructions. For example, total RNA from cells in culture can be isolated using Qiagen miRNeasy mini-columns or using MasterPure™ RNA Purification Kit (EPICENTRE®, Madison, Wis.), or RNA can also be extracted using guanidinium thiocyanate-phenol-chloroform (Chomczynski and Sacchi, "Single-step method of RNA isolation by acid guanidinium thiocyanate-phenol-chloroform extraction," Anal Biochem, April 1987, Vol. 162, No. 1, pages 156-159). For example, the RNA can be extracted using a TRIzol®-based method (Invitrogen, Carlsbad, CA, USA) or a TRI Reagent®-based method (Sigma-Aldrich, St. Louis, MO, USA). Alternatively, RNA can be extracted from the tissues using a method without phenol: chloroform, *mir*Vana™ miRNA Isolation method (Ambion, Austin, TX, USA). Such methods involve disruption of cells or tissue with guanidinium thiocyanate and treatment with an ethanol solution and application to an RNA-binding glass fiber filter. The bound RNA is eluted from the filter following washing to remove proteins, DNA and other contaminants.

MicroRNAs (miRNAs) are released from long hairpin-containing miRNA precursors (pre-miRNAs) as 20-24 nucleotide single-stranded mature miRNAs and enter and guide the RNA-induced silencing complex (RISC) to identify target messages for silencing through either direct mRNA cleavage or translational repression (Bartel, D.P., "MicroRNAs: genomics, biogenesis, mechanism, and function, Cell, 2004, Vol. 116, pages 281-297; Ambros, V., "The functions of animal microRNAs," 2004, Nature Vol. 431, pages 350-355). Such miRNA-mediated gene silencing has been predicted to regulate various developmental, metabolic, and cellular processes.

The expression levels of specific miRNAs are determined in methods of the present invention to predict the likelihood of recurrence of colorectal cancer in a subject. In some aspects of the present invention expression levels are measured for two or more micro ribonucleic acids (miRNAs) selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p in a biological sample *(i.e.,* tumor sample, cancer cells, paired tissue sample, among others) obtained from said subject.

In some aspects of the present invention expression levels are measured for two or more micro ribonucleic acids (miRNAs) selected from the group consisting of hsa-miR-1224-5p; hsa-miR-518b; hsa-miR-629; hsa-miR-885-5p; hsa-miR-139-3p; hsa-miR-223*; hsa-miR-655, hsa-miR-1290, and hsa-miR-450b-5p.

In certain aspects of the present invention expression levels are measured for two or more micro ribonucleic acids (miRNAs) selected from the group consisting of hsa-miR-1224-5p; hsa-miR-518b; hsa-miR-629; hsa-miR-885-5p; hsa-miR-139-3p; and hsa-miR-223*.

Methods of expression profiling that can be used in the present invention include methods based on hybridization analysis of polynucleotides and proteomics-based methods that are known in the art. Methods known in the art for the quantification of miRNA expression in a sample include northern blotting and PCR-based methods, such as reverse transcription polymerase chain reaction (RT-PCR) (Weis et al., Trends in Genetics 8:263-264 (1992)) and quantitative real time polymerase (Q-PCR/qRT-PCR).

Many miRNA detection systems have recently been developed, such as mirVana™ miRNA Detection (Ambion, Austin, TX, USA), the invader assay-based detection (Allawi et al., "Quantitation of microRNAs using a modified Invader assay, 2004, RNA, Vol. 10, pages 1309-1322), mirMASA™ miRNA profiling (Genaco Biomedical Products, Huntsville, AL, USA) (Barad et al., "MicroRNA expression detected by oligonucleotide microarrays: system establishment and expression profiling in human tissues," 2004, Genome Res, Vol. 14, pages 2486-2494), and modified microarrays (Miska et al., "Microarray analysis of microRNA expression in the developing mammalian brain," 2004, Genome Biol, Vol. 5, page R68; Babak et al., "Probing microRNAs with microarrays: tissue specificity and functional interference," 2004, RNA, Vol. 10, pages 1813-1819). A sensitive real-time PCR method has been developed for quantifying the expression of pre-miRNAs (Schmittgen et al., "A high-throughput method to monitor the expression of microRNA precursors," 2004 Nucleic Acids Res, Vol. 32, page e43).

In various embodiments of the invention, various technological approaches are available for determination of expression levels of the disclosed biomarkers, including, without limitation, reverse transcription, qRT-PCR, and microarrays. In some aspects of the present invention the expression levels of the miRNAs are measured by a method comprising reverse transcription (RT-PCR) of miRNA and a quantitative real-time polymerase chain reaction (qRT-PCR).

Reverse transcription PCR (RT-PCR) can be used in determining RNA *(i.e.,* miRNA, ncRNA, *etc.*) levels in various samples. The results can be used to compare gene expression patterns between sample sets, for example in normal and tumor tissues.

As RNA cannot serve as a template for PCR, the first step in gene expression profiling by RT-PCR is the reverse transcription of the RNA template into cDNA, followed by its exponential amplification in a PCR reaction. The two most commonly used reverse transcriptases are avilo myeloblastosis virus reverse transcriptase (AMV-RT) and Moloney murine leukemia virus reverse transcriptase (MMLV-RT). The reverse transcription step is typically primed using specific primers, random hexamers, or oligo-dT primers, depending on the circumstances and the goal of expression profiling. For example, extracted RNA can be reverse-transcribed using a GeneAmp RNA PCR kit (Perkin Elmer, CA, USA), following the manufacturer's instructions. The derived cDNA can then be used as a template in the subsequent PCR reaction.

Although the PCR step can use a variety of thermostable DNA-dependent DNA polymerases, it typically employs the Taq DNA polymerase, which has a 5'-3' nuclease activity but lacks a 3'-5' proofreading endonuclease activity. Thus, TaqMan®PCR typically utilizes the 5'-nuclease activity of Taq or Tth polymerase to hydrolyze a hybridization probe bound to its target amplicon, but any enzyme with equivalent 5' nuclease activity can be used. Two oligonucleotide primers *(i.e.,* primers to a specific miRNA or ncRNA, among others) are used to generate an amplicon typical of a PCR reaction.

In certain embodiments, a third oligonucleotide or probe, is designed to specifically detect a nucleotide sequence located between the two PCR primers. The probe is non-extendible by Taq DNA polymerase enzyme, and is labeled with a reporter fluorescent dye and a quencher fluorescent dye. Any laser-induced emission from the reporter dye is quenched by the quenching dye when the two dyes are located close together as they are on the probe. During the amplification reaction, the Taq DNA polymerase enzyme cleaves the probe in a template-dependent manner. The resultant probe fragments disassociate in solution, and signal from the released reporter dye is free from the quenching effect of the second fluorophore. One molecule of reporter dye is liberated for each new molecule synthesized, and detection of the unquenched reporter dye provides the basis for quantitative interpretation of the data.

Known methods for determination of expression levels of microRNAs, such as Illumina® Small RNA Array System (Illumina, San Diego, CA, USA) and/or TaqMan® MicroRNA Assays (Applied Biosystems, Life Technologies Corp., Carlsbad, CA, USA) can be used in some aspects of the present invention. TaqMan® Small RNA Assays are preformulated primer and probe sets designed to detect and quantify mature microRNAs (miRNAs), small interfering RNAs (siRNAs), and other small RNAs using Applied Biosystems real-time PCR instruments. The assays can detect and quantify small RNAs in 1 to 10 ng of total RNA with a dynamic range of greater than six logs. When used for microRNA analysis, the assays can discriminate mature miRNA sequences from their precursors. TaqMan® MicroRNA Assays are predesigned assays that are available for the majority of content found in the miRBase miRNA sequence repository. These assays can be used for targeted quantification, screening, and validation of miRNA profiling results.

TaqMan®RT-PCR can be performed using commercially available equipment, such as, for example, ABI PRISM 7500™ Sequence Detection System™ (Perkin-Elmer-Applied Biosystems, Foster City, Calif., USA), or Lightcycler (Roche Molecular Biochemicals, Mannheim, Germany).

A system that can be used for qRT-PCR can consist of a thermocycler, laser, charge-coupled device (CCD), camera and computer. The system can amplify samples in a 96-well format on a thermocycler. During amplification, laser-induced fluorescent signal is collected in real-time through fiber optics cables for all 96 wells, and detected at the CCD. The system includes software for running the instrument and for analyzing the data.

5'-Nuclease assay data can initially be expressed as Ct, or the threshold cycle. As discussed above, fluorescence values are recorded during every cycle and represent the amount of product amplified to that point in the amplification reaction. The point when the fluorescent signal is first recorded as statistically significant is the threshold cycle ($C_t$).

Another variation of the RT-PCR technique is real time quantitative PCR (Q-PCR or qRT-PCR), which measures PCR product accumulation through a dual-labeled fluorogenic probe (*i.e.*, TaqMan®probe). Real time PCR is compatible both with quantitative competitive PCR, where internal competitor for each target sequence is used for normalization, and with quantitative comparative PCR using a normalization gene contained within the sample, or a housekeeping gene for RT-PCR. For further details see, *e.g.,* Held et al., Genome Research 6:986-994 (1996).

To minimize errors and the effect of sample-to-sample variation, RT-PCR is usually performed using an internal standard. The ideal internal standard is expressed at a constant level among different tissues, and is unaffected by the experimental treatment.

When determining expression levels of microRNAs, variation in the amount of starting material, sample collection, RNA preparation and quality, and reverse transcription (RT-PCR) efficiency can contribute to quantification errors. Normalization to endogenous control genes can be used to correct for potential RNA input or RT-PCR efficiency biases. Thus, the expression level of the miRNAs can be normalized relative to level of expression of noncoding RNAs (ncRNAs), certain miRNAs or ribosomal RNAs (rRNAs). Noncoding RNAs that can be used for normalization purposes include RNU24, RNU66, RNU19, RNU38B, RNU49, Z30, RNU48, RNU43, U18, RNU58B, RNU58A, RPL21, U54, HY3, U75, RNU68, RNU44, U47 and RNU6B, among others. miRNAs that can be used for normalization purposes include hsa-miR-26b, hsa-miR-92, hsa-miR-92N, hsa-miR-423, hsa-miR-374 and hsa-miR-16, among others. Alternatively, 18S rRNAs can be used for normalization in some aspects of the invention. Preferably, ncRNAs expression levels and total input RNA are used for normalization. Alternatively, normalization can be based on the mean or median signal (Ct) of all of the assayed genes or a large subset thereof (global normalization approach).

In certain embodiments of the present invention, the measured expression levels of miRNAs of interest are normalized relative to the expression levels of one or more noncoding ribonucleic acids (ncRNAs). In some aspects of the present invention, expression levels of RNU6B, RNU44, and/or RNU 48 are used to determine normalized expression levels of miRNAs of interest. In certain aspects of the present invention, expression levels of RNU6B, and/or RNU44 are used to determine normalized expression levels of miRNAs of interest.

In some embodiments of the present invention, the measured expression levels of miRNAs of interest are normalized relative to total amount of RNA, the expression levels of one or more miRNAs, or the expression levels of one or more 18S rRNAs. In some aspects of the present invention expression levels of hsa-miR-16 and/or hsa-miR-92 may be used to determine normalized expression levels of miRNAs of interest. In other embodiments of the present invention, the measured levels of miRNAs are not normalized and the raw qPCR Ct results may be used in calculating the recurrence score.

Differential biomarker/RNA expression can also be identified, or confirmed using the microarray technique. Thus, the expression profile of colorectal cancer-associated biomarkers can be measured in tissue/cells, using microarray technology. In this method, polynucleotide sequences of interest (including cDNAs and oligonucleotides) are plated, or arrayed, on a microchip substrate. The arrayed sequences are then hybridized with specific DNA probes from cells or tissues of interest. Just as in the RT-PCR method, the source of mRNA typically is total RNA isolated from human tumors or tumor cell lines, and corresponding normal tissues or cell lines. Thus, RNA can be isolated from a variety of primary tumors or tumor cell lines. If the source of mRNA is a primary tumor, mRNA can be extracted from a tissue sample or cells.

In a specific embodiment of the microarray technique, PCR amplified inserts of cDNA clones are applied to a substrate in a dense array. Preferably at least 10,000 nucleotide sequences are applied to the substrate. The microarrayed genes, immobilized on the microchip at 10,000 elements each, are suitable for hybridization under stringent conditions. Fluorescently labeled cDNA probes can be generated through incorporation of fluorescent nucleotides by reverse transcription of RNA extracted from tissues of interest. Labeled cDNA probes applied to the chip hybridize with specificity to each spot of DNA on the array. After stringent washing to remove non-specifically bound probes, the chip is scanned by confocal laser microscopy or by another detection method, such as a CCD camera. Quantitation of hybridization of each arrayed element allows for assessment of corresponding mRNA abundance. With dual color fluorescence, separately labeled cDNA probes generated from two sources of RNA are hybridized pair wise to the array. The relative abundance of the transcripts from the two sources corresponding to each specified gene is thus determined simultaneously. The miniaturized scale of the hybridization affords a convenient and rapid evaluation of the expression pattern for large numbers of genes. Such methods have been shown to have the sensitivity required to detect rare transcripts, which are expressed at a few copies per cell, and to reproducibly detect at least approximately two-fold differences in the expression levels (Schena et al., Proc. Natl. Acad. Sci. USA 93(2):106-149 (1996)). Microarray analysis can be performed by commercially available equipment, following manufacturer's protocols, such as by using the Affymetrix GenChip technology, or Incyte's microarray technology.

The materials for use in the methods of the present invention are suited for preparation of kits produced in accordance with well-known procedures. The invention thus provides kits comprising agents, which can include biomarker-specific or biomarker-selective probes and/or primers, for quantitating the expression of the disclosed biomarkers (*i.e.,* miRNAs) for predicting likelihood of recurrence of CRC in a subject. Such kits can optionally contain reagents for the extraction of RNA from tumor samples or cells. In addition, the kits can optionally comprise the reagent(s) with an identifying description or label or instructions relating to their use in the methods of the present invention. The kits can comprise containers (including microtiter plates suitable for use in an automated implementation of the method), each with one or more of the various reagents (typically in concentrated form) utilized in the methods, including, for example, pre-fabricated microarrays, buffers, the appropriate nucleotide triphosphates (*e.g.*, dATP, dCTP, dGTP and dTTP; or rATP, rCTP, rGTP and UTP), reverse transcriptase, DNA polymerase, RNA polymerase, and one or more probes and primers of the present invention (*e.g.*, appropriate length poly(T) or random primers linked to a promoter reactive with the RNA polymerase). Mathematical algorithms used to estimate or quantify prognostic or predictive information are also properly potential components of kits.

Thus, a kit of the present invention can comprise (a) at least two reverse transcription primers for specifically reverse transcribing at least two miRNAs selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p into cDNA; (b) at least two probes that specifically bind to cDNAs reverse transcribed from the miRNAs, wherein the probes are suitable for use in quantitative real time polymerase chain reaction (qRT-PCR) for quantifying the miRNAs present; and a reverse transcription primer for specifically reverse transcribing at least one noncoding RNA and a probe that specifically binds a cDNA reverse transcribed from the at least one noncoding RNA, wherein the probe is suitable for use in quantitative real time polymerase chain reaction (qRT-PCR) for quantifying the noncoding RNA present.

A predictive model for calculating the likelihood of recurrence of CRC can be derived by methods including recruiting colorectal patients having undergone surgical resection and performing statistical analyses (*i.e.,* T-test, Wilcoxon Signed-Rank Test, Logistic Regression, Receiver Operating Characteristic (ROC) analysis, Cox proportional hazards model, Pearson Correlation analysis and Kaplan-Meier estimator, among others) of normalized expression levels of a variety of known miRNAs in cancerous tissue/cells and paired noncancerous tissue/cells from the patients, while taking into account the recurrence rate of CRC in the patients over a given period of time.

In some aspects of the present invention, recruited CRC patients having undergone surgical resectioning can be randomly assigned to a training dataset and a testing dataset. Each dataset can be sorted into two risk groups. "Low risk" being defined as disease free longer than three years after surgery and "high risk" being defined as relapse less than three years after surgery. Samples of colorectal cancer tissues together with the paired non-tumoral tissues can be obtained from all subjects, and the expression levels of a group of miRNAs and other biomarkers for determining normalized expression levels can be determined. The miRNAs, ncRNAs, or rRNAs (ncRNAs and rRNAs measured for normalization) measured can be any known in the art. At least 754 human miRNAs are known in the art.

In certain embodiments of the present invention the normalized expression levels of at least two of the following miRNAs are considered in statistical analyses used to derive a predictive model of the likelihood of recurrence of CRC in a patient: hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p.

In some embodiments the normalized expression levels of the following miRNAs are considered in statistical analyses used to derive a predictive model of the likelihood of recurrence of CRC in a patient: hsa-miR-1224-5p, hsa-miR-518b, hsa-miR-629, hsa-miR-885-5p, hsa-miR-139-3p, and hsa-miR-223*.

Subsequently, the expression levels can be subjected to statistical analysis to derive a predictive model/equation to predict the likelihood of recurrence of CRC in a patient. Thus, in some aspects of the present invention, the T-test and Wilcoxon Signed-Rank Test can be applied to expression level data for two defined patient groups (Low-risk, High-risk), to decide the significant biomarker (*e.g.*, miRNA) candidates (P-value<0.05) in the gene expression data. The Pearson Correlation analysis can be used to calculate the correlation between recurrent time and gene expression.

To better distinguish subjects with high risk or low risk, a logistic regression analysis and a ROC curve analysis may be used for analysis of the Ct value from RT-PCR data. The Kaplan-Meier method and the Cox proportional hazard regression model may also be used.

Combining candidate genes (qPCR data) and clinical factors, the logistic regression analysis may be used to model a response variable and multiple predictors. The ROC curves may be generated for each of the criteria by plotting sensitivity against 1-specificity. The process computes estimated sensitivity and specificity of each observation and also calculates predictive probability for each case by logistic regression analysis. An Area under Curve (AUROC) may be calculated to show the performance of the model.

An event may be defined as the time to recurrence or death in the period of study. All cases may be censored at loss to follow-up or at the end of study period (five years).The Kaplan-Meier method may be used to calculate the disease free survival (DFS) rate and to plot survival curves between two defined groups (high and low risk). The log-rank test may be used for comparing two survival distributions of two groups. In view of such analyses, candidates and clinical factors may be selected for preparing a model. The candidate genes may be identified using a Cox proportional hazard regression model to study the interaction between gene expression levels from subjects with low risk compared to subjects with high risk. The Hazard Ratio (HR) may also be calculated by a Cox proportional hazards model.

A T-test is known in the art and is a statistical hypothesis test in which the test statistic follows a Student's t distribution if the null hypothesis is supported. It can be applied when the test statistic would follow a normal distribution if the value of a scaling term in the test statistic is known. When the scaling term is unknown and is replaced by an estimate based on the data, the test statistic (under certain conditions) follows a Student's t distribution. The T-test may be used to assess whether the means of two groups are statistically different from each other.

The Wilcoxon Signed-Rank Test (Wilcoxon, "Individual comparisons by ranking methods," 1945, Biometrics Bulletin, Vol. 1, No. 6, pages 80-83; Siegel, "Non-parametric statistics for behavioral sciences," 1956, McGraw-Hill, New York, New York, pages 75-83) is a non-parametric statistical hypothesis test that may be used when comparing two related samples or repeated measurements on a single sample to assess whether their population means differ (*i.e.*, a paired difference test).

The Pearson product-moment correlation coefficient (Pearson Correlation analysis) can be used to measure the correlation (linear dependence) between two variables X and Y, giving a value between +1 and -1 inclusive. It can be used as a measure of the strength of linear dependence between two variables.

Combining candidate genes and clinical factors, a logistic regression method can be used to build a predictive model. An effect of the model can be confirmed by Receiver Operating Characteristic (ROC) analysis. The Kaplan-Meier method can be performed to compare the survival rate for patients between two groups. The candidate biomarkers/genes *(i.e.,* miRNAs, among others) can be identified using a Cox proportional hazard regression model to study the interaction effects between gene expression levels from patients with low-risk compared to patients with high-risk. The Hazard ratio (Risk ratio) for candidates can be different when measured in high-risk patients versus low-risk patients.

In signal detection theory, a receiver operating characteristic (ROC), or simply ROC curve, is a graphical plot of the sensitivity, or true positive rate, vs. false positive rate (1 - specificity or 1 - true negative rate), for a binary classifier system as its discrimination threshold is varied. The ROC can also be represented equivalently by plotting the fraction of true positives out of the positives (TPR = true positive rate) vs. the fraction of false positives out of the negatives (FPR = false positive rate). Also known as a Relative Operating Characteristic curve, because it is a comparison of two operating characteristics (TPR & FPR) as the criterion changes. ROC analysis provides tools to select possibly optimal models and to discard suboptimal ones independently from (and prior to specifying) the cost context or the class distribution.

The Kaplan-Meier estimator, (Kaplan and Meier, "Nonparametric estimation from incomplete observations," 1958, J. Amer Statist Assn, Vol. 53, pages 457-481) also known as the product limit estimator, is an estimator for estimating the survival function from life-time data. It can be used to measure the fraction of patients living for a certain amount of time after treatment. A plot of the Kaplan-Meier estimate of the survival function is a series of horizontal steps of declining magnitude which, when a large enough sample is taken, approaches the true survival function for that population. The value of the survival function between successive distinct sampled observations is assumed to be constant. Some embodiments of the present invention can comprise analyzing the normalized expression levels of specific miRNAs correlated with CRC recurrence using Kaplan-Meier survival curves.

Whereas the Kaplan-Meier method with log-rank test is useful for comparing survival curves in two or more groups, Cox proportional-hazards regression allows analyzing the effect of several risk factors on survival. (Cox, "Regression Models and Life-Tables," 1972, J of the Royal Stat Soc, Series B (Methodological), Vol. 34, No. 2, pages 187-220.) The probability of the endpoint (death, or any other event of interest, *e.g.*, recurrence of CRC) is designated as the hazard.

The statistical methods discussed above can be performed using known computer programs for statistical analysis.

Two exemplary recurrence predicting models of the present invention for predicting the likelihood of recurrence of CRC in a patient are as follows:

*Recurrence Predicting Model 1:*

*Recurrence score=exp(y)/(1+exp(y)*

*Where y:*

$$y = 315.8 - 26.3641 \times (hsa\text{-}mir\text{-}1224\text{-}5p(Tumor)) - 3.1687 \times (hsa\text{-}mir\text{-}1224\text{-}5p(Normal)) \\ -3.8282 \times (hsa\text{-}miR\text{-}518b(Tumor)) + 2.9126 \times (hsa\text{-}miR\text{-}629(Tumor)) + 9.4863 \times (hsa\text{-}miR\text{-}629(Normal)) \\ -30.1097 \times (hsa\text{-}miR\text{-}885\text{-}5p(Normal)) - 6.9425 \times (hsa\text{-}mir\text{-}139\text{-}3p(Tumor)) - 2.0399 \times (hsa\text{-}mir\text{-}139\text{-}3p(Normal)) \\ +0.5164 \times (hsa\text{-}miR\text{-}223^*(Tumor)) + 3.1883 \times (hsa\text{-}miR\text{-}223^*(Normal)) \\ +3.2598 \times \{(hsa\text{-}mir\text{-}1224\text{-}5p(Tumor)) \times (hsa\text{-}miR\text{-}885\text{-}5p(Normal))\} \\ +0.6281 \times \{(hsa\text{-}mir\text{-}139\text{-}3p(Tumor)) \times (hsa\text{-}miR\text{-}223^*(Tumor))\} \\ -1.3465 \times \{(hsa\text{-}miR\text{-}629(Normal)) \times (hsa\text{-}miR\text{-}223^*(Tumor))\} ......[\text{formula}37]$$

*Recurrence Predicting Model 2*

*Recurrence score=exp(y)/(1+exp(y)*

*Where y:*

$$y = 10.1195 - 0.3503 \times (hsa\text{-}mir_1224\text{-}5p(Tumor)) + 0.9442 \times (hsa\text{-}mir_1224\text{-}5p(Normal)) \\ +8.7184 \times (hsa\text{-}miR\text{-}518b(Tumor)) \\ +2.2476 \times (hsa\text{-}miR\text{-}629(Tumor)) - 1.4460 \times (hsa\text{-}miR\text{-}629(Normal)) - 0.2093 \times (hsa\text{-}miR\text{-}885\text{-}5p(Normal)) \\ -4.9632 \times (hsa\text{-}miR\text{-}223^*(Tumor)) + 0.5182 \times (hsa\text{-}miR\text{-}223^*(Normal)) - 2.5481 \times (hsa\text{-}mir\text{-}139\text{-}3p(Tumor)) \\ -2.6980 \times (hsa\text{-}mir\text{-}139\text{-}3p(Normal)) - 1.8716 \times (hsa\text{-}miR\text{-}655(Tumor)) + 1.9734 \times (hsa\text{-}miR\text{-}1290(Normal)) \\ -2.4953 \times (hsa\text{-}miR\text{-}450b\text{-}5p(Tumor)) ......fomula50$$

In the predictive models above, (tumor) refers to expression of the specified miRNA in at tumor sample or sample of cancer cells and (normal) refers to expression of the specified miRNA in a sample from noncancerous tissue that is of the same type of tissue out of which the tumor was formed.

These are but two exemplary predictive models according to the present invention. Other predictive models can be developed using the methods disclosed herein. Other predictive models can employ more or few microRNAs to predict the likelihood of recurrence of CRC in a patient.

Thus, RNA can be extracted from a tumor sample or cancerous cells from a CRC patient. The normalized expression levels of specific miRNAs used in the predictive models can be determined and using these expression levels the probability of recurrence of CRC can be calculated.

The methods provided by the present invention can also be automated in whole or in part.

The methods of the present invention are suited for the preparation of reports summarizing the predictions resulting from the methods of the present invention. The invention thus provides for methods of creating reports and the reports resulting therefrom. The report can include a summary of the expression levels of the RNA transcripts for certain biomarkers *(i.e.,* miRNAs, among others) in the cells obtained from the patient's tumor tissue. In some embodiments of the present invention a report is prepared the recurrence score and/or a determination of the likelihood of CRC recurrence made using the recurrence score. The report can include a prediction that said subject has an increased likelihood of recurrence of CRC. The report can include a recommendation for treatment modality such as surgery alone or surgery in combination with chemotherapy. The report can be presented in electronic format or on paper.

All aspects of the present invention may also be practiced such that a limited number of additional genes that are co-expressed with the disclosed genes, for example as evidenced by high Pearson correlation coefficients, are included in a predictive test in addition to and/or in place of disclosed biomarkers.

Having described the invention, the same will be more readily understood through reference to the following Examples, which are provided by way of illustration, and are not intended to limit the invention in any way.

EXAMPLES

Example 1

Patients and Tissue Specimens

Eighty colorectal patients underwent surgical resection and were recruited from the National Cheng-Kung University Hospital, Tainan, Taiwan. These patients were randomly assigned to a training dataset (n=65) and a testing dataset (n=15). Characteristics of the patients are summarized in Table 1. Each dataset consisted of two risk groups. "Low risk" was defined as disease free after surgery and "high risk" was defined as relapse less than three years after surgery. Frozen samples of colorectal cancer tissues together with the paired non-tumoral tissues were obtained from all subjects.

Table 1: Summary of Clinical Information

| **Patient Characteristics** | | | | |
|---|---|---|---|---|
| | **Training set (n=65)** | | **Testing set (n=15)** | |
| | **High Risk (n=29)** | **Low Risk (n=36)** | **High Risk (n=6)** | **Low Risk (n=9)** |
| Age, mean (SD) | 63.55(12.44) | 64.17(11.60) | 66.37(5.12) | 66.78(8.94) |
| Gender, M/F | 14 / 15 | 20 / 16 | 3 / 3 | 6 / 3 |
| Stage (II/III) | 11 / 18 | 11 / 25 | 1 / 5 | 5 / 4 |

More detailed information regarding the patients in the study are provided in Tables 2-4 below.

| **Table 2. Patient Characteristics** | | |
|---|---|---|
| | **Patients (n=80)** | |
| | **High Risk (n=35)** | **Low Risk (n=45)** |
| Age, mean (SD) | 64.03 (11.51) | 64.69 (11.08) |
| Male, n (%) | 17 (0.49) | 26 (0.58) |
| Stage II, n (%) | 12 (0.34) | 16 (0.36) |
| Diagnosis, n (%) | | |
| A-colon cancer | 6 (0.17) | 9 (0.20) |
| T-colon cancer | 0 (0.00) | 2 (0.04) |
| D-colon cancer | 2 (0.06) | 3 (0.07) |
| S-colon cancer | 10 (0.29) | 18 (0.40) |
| Rectal cancer | 12 (0.34) | 12 (0.27) |
| Colon cancer | 1 (0.03) | 1 (0.02) |
| N/A | 4 (0.11) | 0 (0.00) |
| **mean (SD)** | | |

| Table 3. Patient Characteristics | | |
|---|---|---|
| | Training set (n=65) | Testing set (n=15) |
| Age, mean (SD) | 63.89(11.89) | 66.62(7.32) |
| Male, n (%) | 34 (0.52) | 9 (0.60) |
| Stage II, n (%) | 22 (0.34) | 6 (0.40) |
| Diagnosis, n (%) | | |
| A-colon cancer | 11 (0.17) | 4 (0.27) |
| T-colon cancer | 2 (0.03) | 0 (0.00) |
| D-colon cancer | 5 (0.08) | 0 (0.00) |
| S-colon cancer | 21 (0.32) | 7 (0.47) |
| Rectal cancer | 21 (0.32) | 3 (0.20) |
| Colon cancer | 2 (0.03) | 0 (0.00) |
| N/A | 3 (0.05) | 1 (0.07) |
| **mean (SD)** | | |

**Table 4. Patient Characteristics**

| | Training set (n=65) | | Testing set (n=15) | |
|---|---|---|---|---|
| | High Risk (n=29) | Low Risk (n=36) | High Risk (n=6) | Low Risk (n=9) |
| Age, mean (SD) | 63.55(12.44) | 64.17(11.60) | 66.37(5.12) | 66.78(8.94) |
| Male, n(%) | 14 (0.48) | 20 (0.56) | 3 (0.50) | 6 (0.67) |
| Stage II, n (%) | 11 (0.38) | 11 (0.31) | 1 (0.17) | 5 (0.56) |
| Diagnosis, n (%) | | | | |
| A-colon cancer | 5 (0.17) | 7 (0.19) | 1 (0.17) | 3 (0.33) |
| T-colon cancer | 0 (0.00) | 2 (0.06) | 0 (0.00) | 0 (0.00) |
| D-colon cancer | 2 (0.07) | 3 (0.08) | 0 (0.00) | 0 (0.00) |
| S-colon cancer | 8 (0.28) | 13 (0.36) | 2 (0.33) | 5 (0.56) |
| Rectal cancer | 10 (0.34) | 11 (0.31) | 2 (0.33) | 1 (0.11) |
| Colon cancer | 1 (0.03) | 1 (0.03) | 0 (0.00) | 0 (0.00) |
| N/A | 3 (0.10) | 0 (0.00) | 1 (0.17) | 0 (0.00) |
| **mean (SD)** | | | | |

MicroRNA Profiling

Total RNA were extracted using a TRIzol®-based method from all study subjects. For the discovery phase, 30 potential candidates were identified using Illumina® Small RNA Array System Human MI_V2 (human microarray version 2; including 1146 probes) (Illumina, San Diego, CA, USA). Subsequently, custom TaqMan® small RNA Assays (Applied Biosystems, Life Technologies Corp., Carlsbad, CA, USA) were used for validation and algorithm development. A microarray was used to select significant differential small RNA candidates, which included about 30 small RNA candidates. TaqMan® Human MicroRNA Assays were used to further validate the 30 candidates selected. Using TaqMan® MicroRNA Assays, 19 miRNA candidates demonstrated differential expression between high/low risk groups. The overall development process is summarized in Figure 1. The expression level of each miRNA was represented by a threshold cycle (Ct) value. The Ct value of each miRNA was then normalized by RNU6B and RNU44 expression levels, which are commonly used as internal controls for miRNA quantification assays. Finally, the normalized miRNA expression levels were represented as dCt.

Statistical Analysis

According to the two defined groups (Low-risk, High-risk), the T-test and Wilcoxon Signed-Rank Test were utilized to decide the significant candidates (P-value<0.05) in the gene expression data. The Pearson Correlation analysis was used to calculate the correlation between recurrent time and gene expression.

Combining candidate genes and clinical factors, a logistic regression method was used to build a predictive model. An effect of the model was confirmed by Receiver Operating Characteristic (ROC) analysis. The Kaplan-Meier method was conducted to compare the survival rate for patients between two groups. The candidate genes were identified using a Cox proportional hazard regression model to study the interaction effects between gene expression levels from patients with low-risk compared to patients with high-risk. The Hazard ratio (Risk ratio) for candidates changed when measured in high-risk patients versus low-risk patients.

To better distinguish subjects with high risk or low risk, the logistic regression analysis and the ROC curve analysis were considered to analysis Ct value from RT-PCR data. In addition, the aim was to better understand the recurrence of cancer progression. The Kaplan-Meier method and the Cox proportional hazard regression model were also used.

Combining candidate genes (qPCR data) and clinical factors, the logistic regression analysis was used to model a response variable and multiple predictors. The ROC curves were generated for each of the criteria by plotting sensitivity against 1-specificity. The process computes estimated sensitivity and specificity of each observation and also calculates predictive probability for each case by logistic regression analysis. An Area under Curve (AUC) was calculated to show the performance of the model.

The event was defined as the time to recurrence or death in the period of study. All cases were censored at loss to follow-up or at the end of study period (five years).The Kaplan-Meier method was conducted to calculate the disease free survival(DFS) rate and to plot survival curves between two defined groups(high risk , low risk).The log-rank test was used for comparison of two survival distributions of two groups. In consideration of previously candidates and clinical factor were selected for the model. The candidate genes could be identified using Cox proportional hazard regression model to study the interaction effect between gene expression levels from subjects with low risk compared to subjects with high risk. The Hazard Ratio (HR) was also calculated by a Cox proportional hazards model.

<u>Results</u>

Expression levels of colorectal cancer (CRC) recurrence biomarkers were measured, including 18 miRNAs: hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-223*, hsa-miR-518b, hsa-miR-629, hsa-miR-885-5p, hsa-miR-139-3p, hsa-miR-655, hsa-miR-1290, hsa-miR-450b-5p, and hsa-miR-1224-5p. The expression levels for the two reference non-coding RNAs RNU6B and RNU44 were also measured to normalize the expression levels measured for the CRC recurrence biomarkers. The sequence information for the measured CRC biomarkers (microRNAs) is summarized in Table 5.

Table 5: Sequences of CRC recurrent biomarkers

| miRBase ID | Target Sequence | SEQ ID |
|---|---|---|
| hsa-miR-363* | CGGGUGGAUCACGAUGCAAUUU | SEQ ID NO:1 |
| hsa-miR-1255b | CGGAUGAGCAAAGAAAGUGGUU | SEQ ID NO:2 |
| hsa-miR-566 | GGGCGCCUGUGAUCCCAAC | SEQ ID NO:3 |
| hsa-miR-1265 | CAGGAUGUGGUCAAGUGUUGUU | SEQ ID NO:4 |
| hsa-miR-127-5p | CUGAAGCUCAGAGGGCUCUGAU | SEQ ID NO:5 |
| hsa-miR-338-5p | AACAAUAUCCUGGUGCUGAGUG | SEQ ID NO:6 |
| hsa-miR-550a* | UGUCUUACUCCCUCAGGCACAU | SEQ ID NO:7 |
| hsa-miR-588 | UUGGCCACAAUGGGUUAGAAC | SEQ ID NO:8 |
| hsa-miR-651 | UUUAGGAUAAGCUUGACUUUUG | SEQ ID NO:9 |
| hsa-miR-223* | CGUGUAUUUGACAAGCUGAGUU | SEQ ID NO:10 |
| hsa-miR-518b | CAAAGCGCUCCCCUUUAGAGGU | SEQ ID NO:11 |
| hsa-miR-629 | UGGGUUUACGUUGGGAGAACU | SEQ ID NO:12 |
| hsa-miR-885-5p | UCCAUUACACUACCCUGCCUCU | SEQ ID NO:13 |
| hsa-miR-139-3p | UCUACAGUGCACGUGUCUCCAG | SEQ ID NO:14 |
| hsa-miR-655 | AUAAUACAUGGUUAACCUCUUU | SEQ ID NO:15 |
| hsa-miR-1290 | UGGAUUUUUGGAUCAGGGA | SEQ ID NO:16 |
| hsa-miR-450b-5p | UUUUGCAAUAUGUUCCUGAAUA | SEQ ID NO:17 |

(continued)

| miRBase ID | Target Sequence | SEQ ID |
|---|---|---|
| hsa-miR-1224-5p | GUGAGGACUCGGGAGGUGG | SEQ ID NO:18 |

Two recurrence predicting models developed from the statistical analyses performed are detailed below. In the predictive models below, (tumor) refers to expression of the specified miRNA in at tumor sample or sample of cancer cells and (normal) refers to expression of the specified miRNA in a sample from noncancerous tissue that is of the same type of tissue out of which the tumor was formed.

*Recurrence Predicting Model 1*

Based on statistical analysis of the expression levels in tumor tissue and matched tissue in the study patient population, the following predictive model was developed:

$$Recurrence\ score=exp(y)/(1+exp(y))$$

*Where y:*

$$y = 315.8-26.3641\times(hsa\text{-}mir\text{-}1224\text{-}5p(Tumor))-3.1687\times(hsa\text{-}mir\text{-}1224\text{-}5p(Normal))$$
$$-3.8282\times(hsa\text{-}miR\text{-}518b(Tumor))+2.9126\times(hsa\text{-}miR\text{-}629(Tumor))+9.4863\times(hsa\text{-}miR\text{-}629(Normal))$$
$$-30.1097\times(hsa\text{-}miR\text{-}885\text{-}5p(Normal))-6.9425\times(hsa\text{-}mir\text{-}139\text{-}3p(Tumor))-2.0399\times(hsa\text{-}mir\text{-}139\text{-}3p(Normal))$$
$$+0.5164\times(hsa\text{-}miR\text{-}223^*(Tumor))+3.1883\times(hsa\text{-}miR\text{-}223^*(Normal))$$
$$+3.2598\times\{(hsa\text{-}mir\text{-}1224\text{-}5p(Tumor))\times(hsa\text{-}miR\text{-}885\text{-}5p(Normal))\}$$
$$+0.6281\times\{(hsa\text{-}mir\text{-}139\text{-}3p(Tumor))\times(hsa\text{-}miR\text{-}223^*(Tumor))\}$$
$$-1.3465\times\{(hsa\text{-}miR\text{-}629(Normal))\times(hsa\text{-}miR\text{-}223^*(Tumor))\}......[formula37]$$

*Recurrence Predicting Model 2*

Based on statistical analysis of the expression levels in tumor tissue and matched tissue in the study patient population, the following second predictive model was developed:

$$Recurrence\ score=exp(y)/(1+exp(y))$$

*Where y:*

$$y=10.1195-0.3503\times(hsa\text{-}mir_1224\text{-}5p(Tumor))+0.9442\times(hsa\text{-}mir_1224\text{-}5p(Normal))$$
$$+8.7184\times(hsa\text{-}miR\text{-}518b(Tumor))$$
$$+2.2476\times(hsa\text{-}miR\text{-}629(Tumor))-1.4460\times(hsa\text{-}miR\text{-}629(Normal))-0.2093\times(hsa\text{-}miR\text{-}885\text{-}5p(Normal))$$
$$-4.9632\times(hsa\text{-}miR\text{-}223^*(Tumor))+0.5182\times(hsa\text{-}miR\text{-}223^*(Normal))-2.5481\times(hsa\text{-}mir\text{-}139\text{-}3p(Tumor))$$
$$-2.6980\times(hsa\text{-}mir\text{-}139\text{-}3p(Normal))-1.8716\times(hsa\text{-}miR\text{-}655(Tumor))+1.9734\times(hsa\text{-}miR\text{-}1290(Normal))$$
$$-2.4953\times(hsa\text{-}miR\text{-}450b\text{-}5p(Tumor))......fomula50$$

Results of the statistical analyses performed and relied on in developing recurrence models are found in Figures 2-60. Figures 2-60 demonstrate the combination of specific microRNA candidates, which could reach AUROC>0.85 (which is of clinical usefulness). These are examples of the present invention and are not intended to encompass all possible embodiments.

While the present invention has been described with reference to what are considered to be the specific embodiments, it is to be understood that the invention is not limited to such embodiments. To the contrary, the invention is intended to cover various modifications and equivalents included within the spirit and scope of the appended claims.

It will be apparent to those skilled in the art that various modifications and variations can be made to the disclosed embodiments. It is intended that the specification and examples be considered as exemplary only, with a true scope of the disclosure being indicated by the following claims and their equivalents.

**Claims**

1. A method for determining the likelihood of colorectal cancer (CRC) recurrence in a subject comprising:

   measuring the expression level of six micro ribonucleic acids (miRNAs) selected from the group consisting of hsa-miR-1224-5p, hsa-miR-518b, hsa-miR-629, hsa-miR-885-5p, hsa-miR-139-3p, and hsa-miR-223* in a biological sample comprising CRC tumor cells obtained from said subject,
   calculating a recurrence score by a method comprising weighting the expression levels of the miRNAs by contribution to CRC recurrence, and
   determining the likelihood of colorectal cancer recurrence for said subject using the recurrence score.

2. The method of claim 1, further comprising measuring an expression level of one or more micro ribonucleic acids (miRNAs) selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-655, hsa-miR-1290, and hsa-miR-450b-5p.

3. The method of claim 1 or 2, wherein the subject is a human.

4. The method of claim 1 or 2, wherein the expression levels of the miRNAs are measured by a method comprising (a) reverse transcription of miRNA and a quantitative real-time polymerase chain reaction (RT-PCR) or (b) microarray analysis.

5. The method o of claim 1 or 2, wherein the measured expression levels are normalized relative to (a) the expression levels of one or more noncoding ribonucleic acids (ncRNAs), (b) total amount of RNA, (c) the expression levels of one or more miRNAs, or (d) the expression levels of one or more 18S rRNAs.

6. The method of claim 1 or 2, wherein the measured expression levels are not normalized and raw qPCR Ct results are used in calculating the recurrence score.

7. The method of claim 5, wherein the measured expression levels are normalized relative to the expression levels of one or more noncoding ribonucleic acids (ncRNAs) and the noncoding RNA (ncRNA) is selected from transcripts of RNU6B, RNU44, and RNU48.

8. The method of claim 5, wherein the measured expression levels are normalized relative to the expression levels of hsa-miR-16 and/or hsa-miR-92.

9. The method of claim 1 or 2, wherein the individual contribution of each miRNA expression level measured is weighted separately in the calculating step.

10. The method of claim 1 or 2, wherein said biological sample is a fresh sample of a CRC tumor, a frozen sample of a CRC tumor or a paired non-tumoral tissue.

11. The method of claim 1 or 2, wherein said biological sample is a sample containing small RNAs.

12. The method of claim 1 or 2, further comprising preparing a report comprising the recurrence score and/or the determination of the likelihood of CRC recurrence made using the recurrence score.

13. The method of claim 1 or 2, wherein said recurrence score is calculated by logistic regression analysis.

14. The method of claim 1 or 2, wherein said recurrence score is calculated by the following formula:

$$\text{Recurrence score} = \exp(y)/(1 + \exp(y)),$$

wherein y is a formula combined multiple predictors recurrence score.

15. The method of claim 1 or 2, wherein said recurrence score is determined according to one of the equations shown in Figures 4-60.

16. A kit for predicting the recurrence of colorectal cancer in a subject consisting of:

reverse transcription primers for specifically reverse transcribing six miRNAs of hsa-miR-1224-5p, hsa-miR-518b, hsa-miR-629, hsa-miR-885-5p, hsa-miR-139-3p, and hsa-miR-223* into cDNA.

17. A kit of claim 16, further comprising one or more reverse transcription primers for specifically reverse transcribing one or more miRNAs selected from the group consisting of hsa-miR-363*, hsa-miR-1255b, hsa-miR-566, hsa-miR-1265, hsa-miR-127-5p, hsa-miR-338-5p, hsa-miR-550a*, hsa-miR-588, hsa-miR-651, hsa-miR-655, hsa-miR-1290, and hsa-miR-450b-5p.

18. A kit of claim 16 or 17, further comprising probes that specifically bind to cDNAs reverse transcribed from the miRNAs, wherein the probes are suitable for use in real time polymerase chain reaction (RT-PCR) for quantifying the miRNAs present.

**19.** A kit of claim 16 or 17, further comprising a reverse transcription primer for specifically reverse transcribing at least one noncoding RNA and a probe that specifically binds a cDNA reverse transcribed from the at least one noncoding RNA, wherein the probe is suitable for use in real time polymerase chain reaction (RT-PCR) for quantifying the noncoding RNA present.

**20.** A method for determining the likelihood of the recurrence of colorectal cancer in a subject comprising:

collecting at the time of colorectal tumor removal surgery from the subject (a) a sample of a colorectal tumor and (b) a paired sample of non-tumorous tissue that is of the same type of tissue out of which the tumor was formed;
extracting total ribonucleic acid (RNA) from each of samples (a) and (b);
performing reverse transcription and quantitative real-time polymerase chain reaction (RT-PCR) with the extracted RNA for each of samples (a) and (b) to determine the normalized level of expression of each of the miRNAs of hsa-miR-1224-5p; hsa-miR-518b; hsa-miR-629; hsa-miR-885-5p; hsa-miR-139-3p; and hsa-miR-223*, wherein the expression level is normalized relative to level of expression of noncoding RNAs (ncRNAs) RNU6B and RNU44 in the samples; and
calculating the probability that the subject will have a recurrence of colorectal cancer according to recurrence score

$$\text{Recurrence score} = \exp(y)/(1 + \exp(y)),$$

wherein y is determined according to one of the equations of Figures 4-60,
wherein (tumor) refers to expression of the specified miRNA in sample (a) and (normal) refers to expression of the specified miRNA in sample (b) taken from non-tumorous tissue that is of the same type of tissue out of which the tumor was formed.

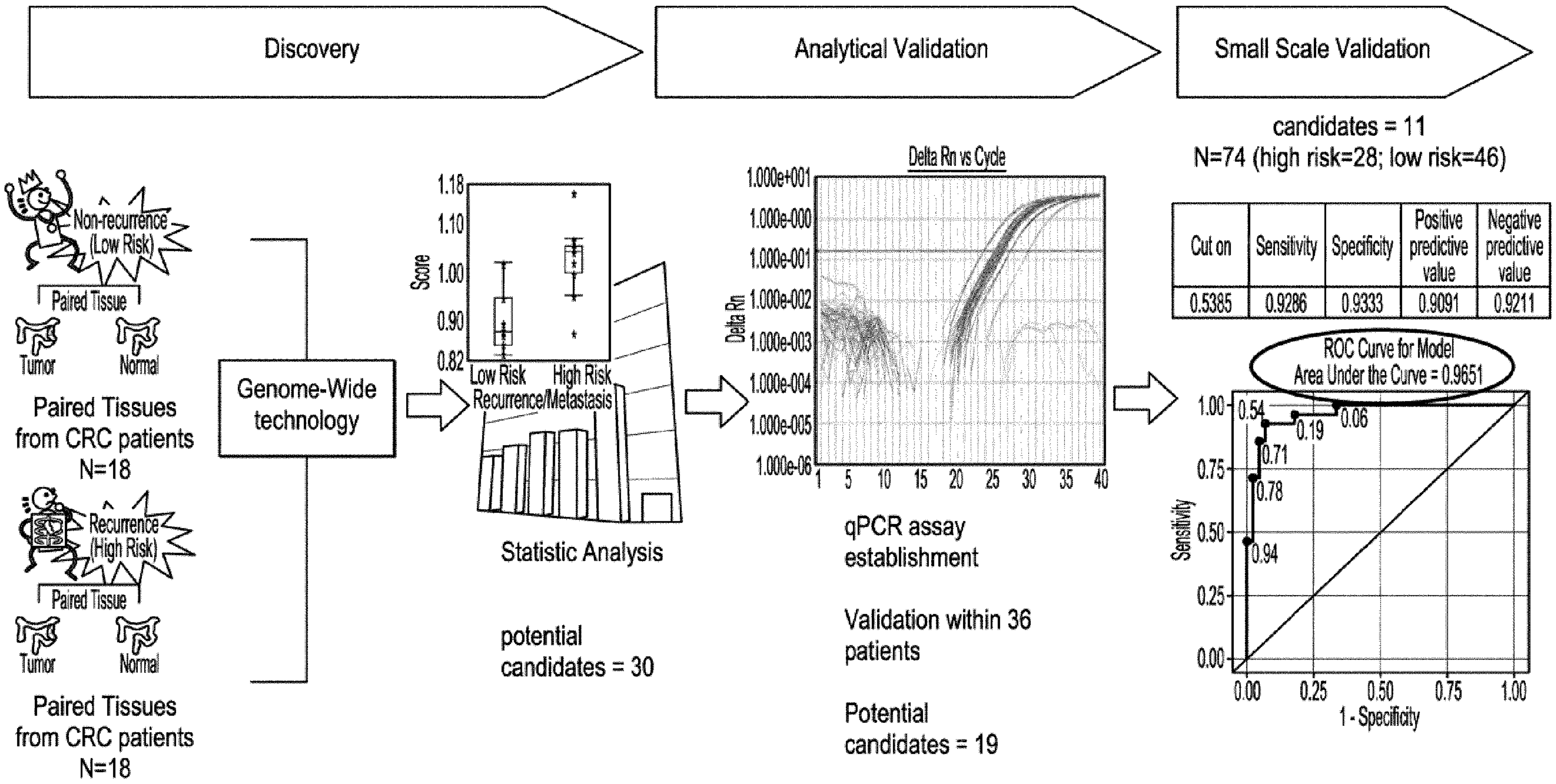


Summary of the CRC recurrence biomarker discovery process

Fig.1

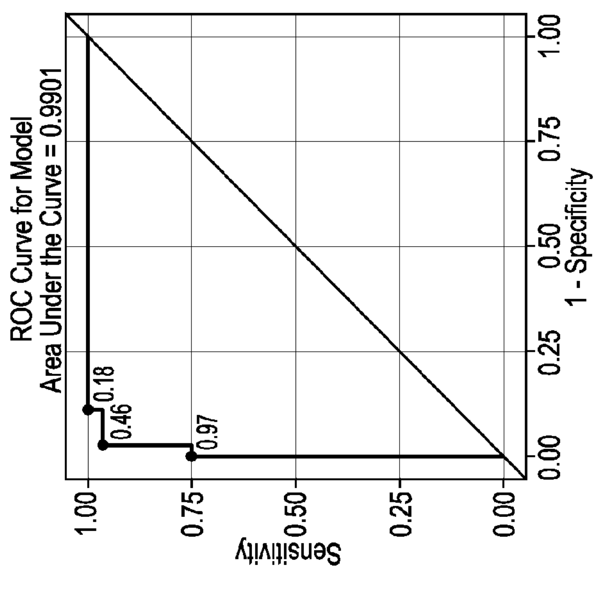


Fig.2-1

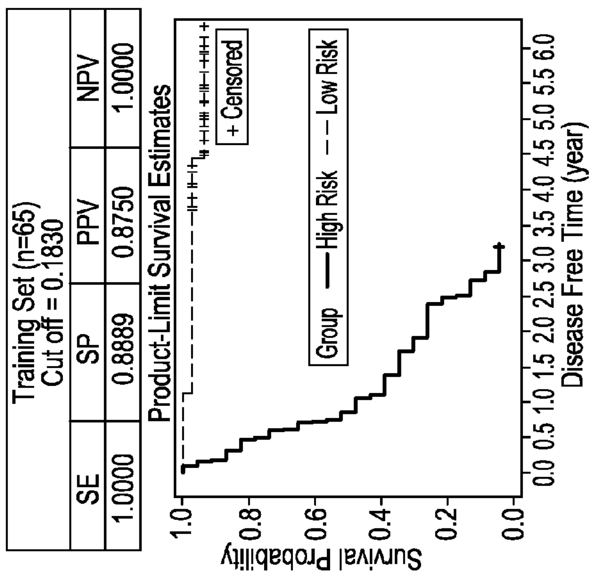


Fig.2-1a

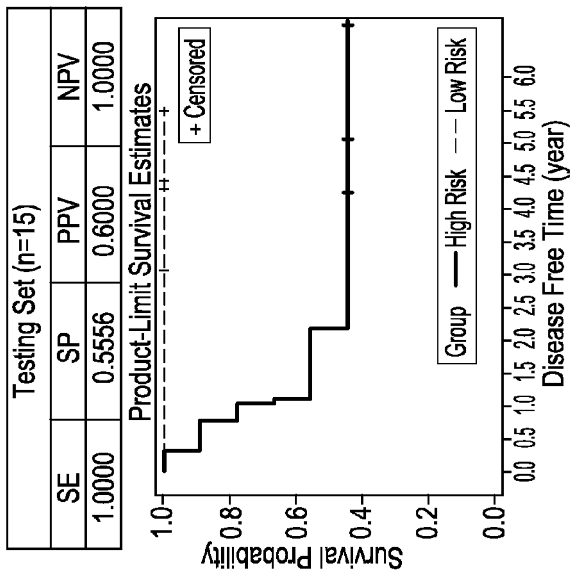


Fig.2-1b

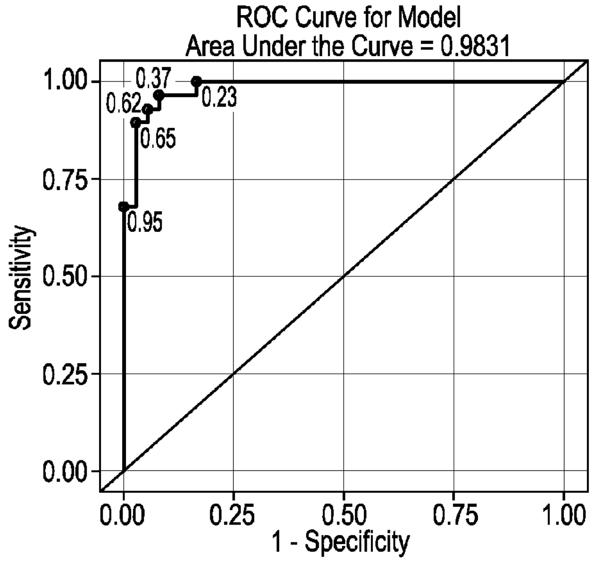


Fig.3-1

| Training Set (n=65) Cut off = 0.2331 | | | |
|---|---|---|---|
| SE | SP | PPV | NPV |
| 0.75 | 1.0000 | 1.0000 | 0.8372 |

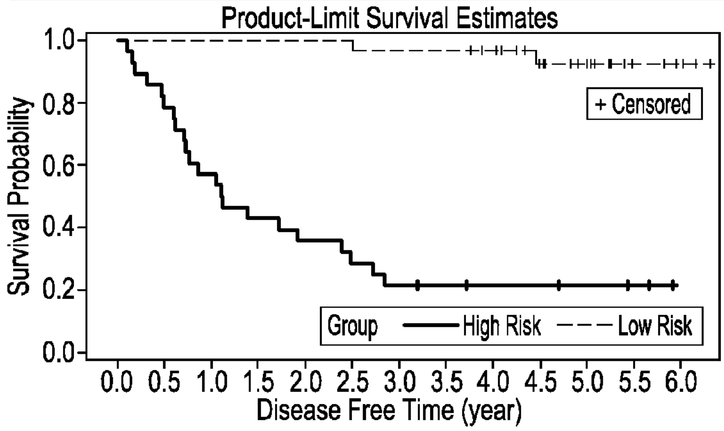


Fig.3-1a

| Testing Set (n=15) | | | |
|---|---|---|---|
| SE | SP | PPV | NPV |
| 0.5556 | 1.0000 | 1.0000 | 0.6 |

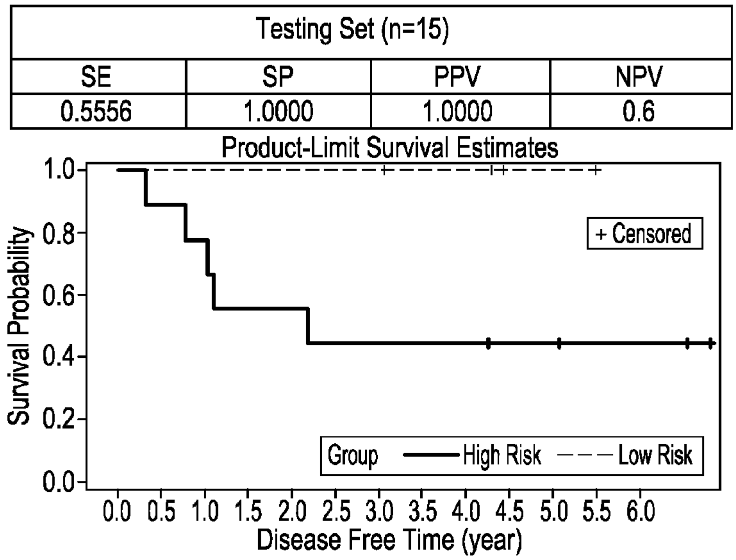


Fig.3-1b

Recurrence score = exp( y)/(1 + exp( y)); in which y

$$y = 16.8612 - 0.6640 \times (hsa\text{-}miR\text{-}518b(Tumor)) - 1.0130 \times (hsa\text{-}miR\text{-}885\text{-}5p(Normal)) \text{......[formula1]}$$

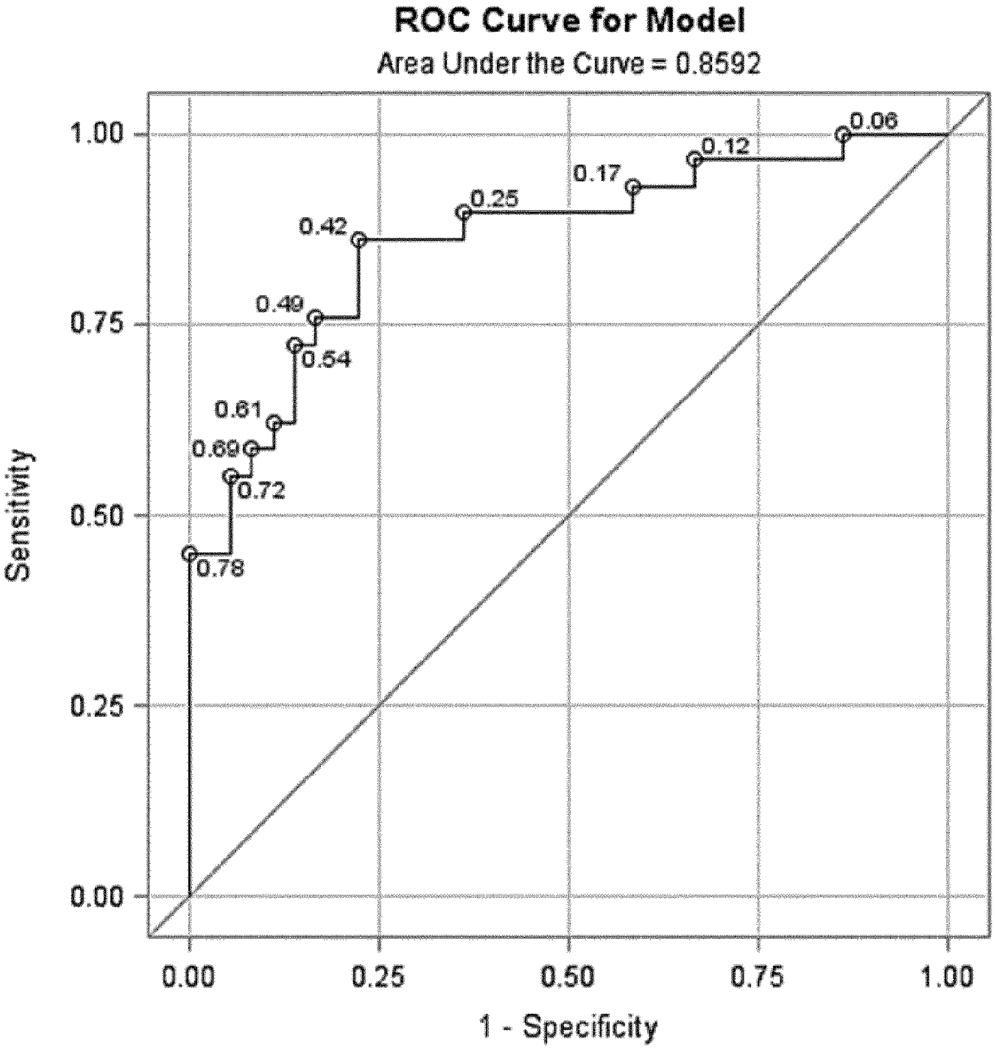


Fig.4

Recurrence score = exp( y)/(1 + exp( y)); in which y

$$y = 25.6050 - 0.8781 \times (hsa\text{-}miR\text{-}629(Normal)) - 1.1826 \times (hsa\text{-}miR\text{-}885\text{-}5p(Normal)) - 0.3721 \times (hsa\text{-}mir\text{-}139\text{-}3p(Tumor)) \quad ......[formula2]$$

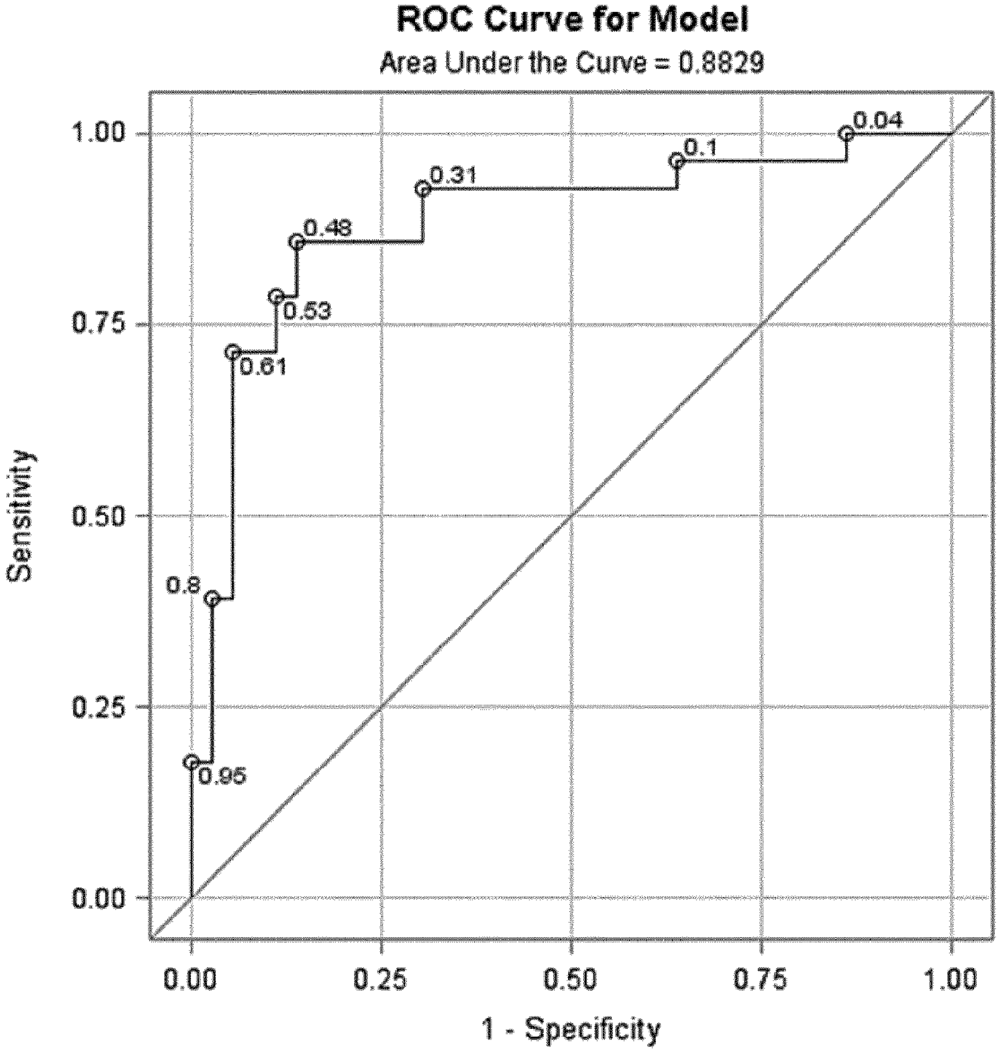


Fig.5

Recurrence score = exp( y)/(1 + exp( y)); in which y

$$y = 25.7065 - 0.4375 \times (hsa\text{-}miR\text{-}518b(Tumor)) - 0.6637 \times (hsa\text{-}miR\text{-}629(Normal)) - 0.9932 \times (hsa\text{-}miR\text{-}885\text{-}5p(Normal)) - 0.3170 \times (hsa\text{-}mir\text{-}139\text{-}3p(Tumor)) \quad \text{......[formula3]}$$

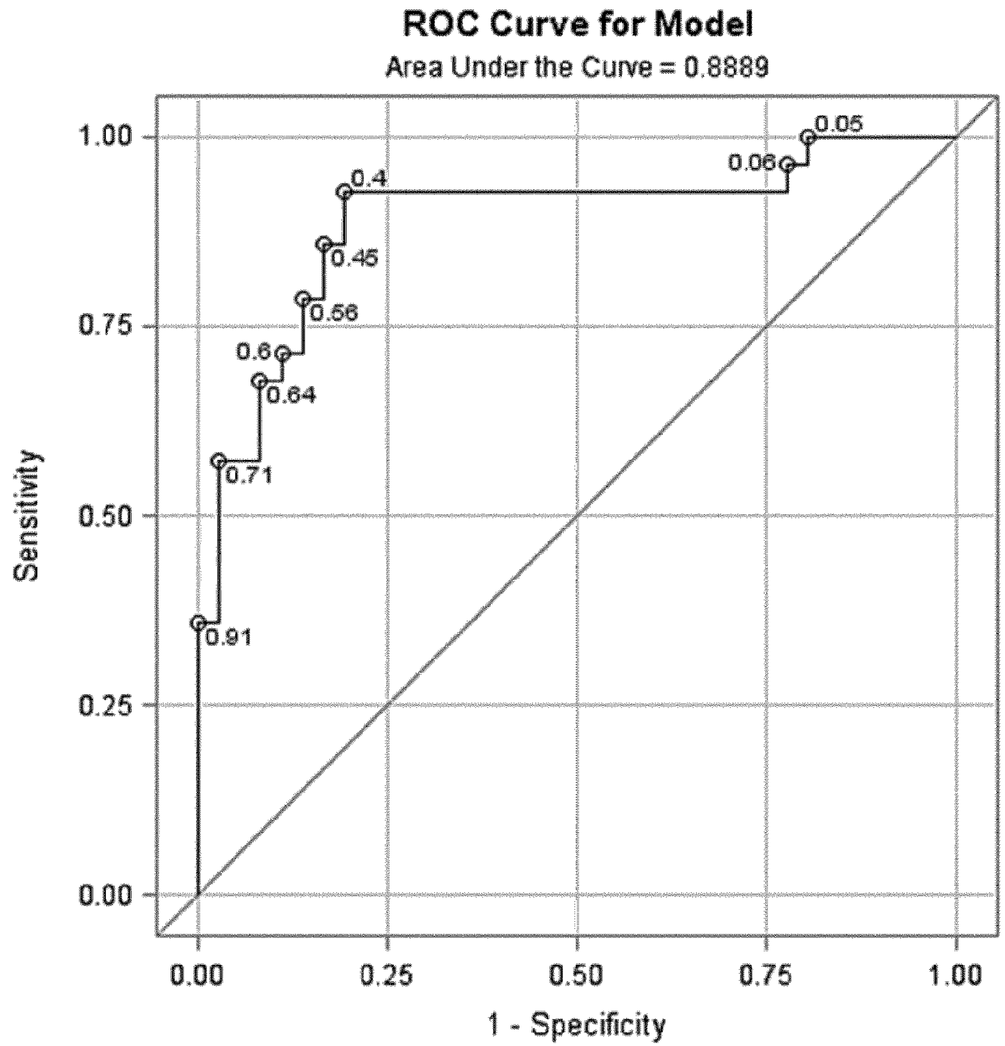


Fig.6

Recurrence score = exp( y)/(1 + exp( y)); in which y

$$y = 26.1881 - 0.3445 \times (hsa\text{-}miR\text{-}518b(Tumor)) - 0.7718 \times (hsa\text{-}miR\text{-}629(Normal)) - 0.8979 \times (hsa\text{-}miR\text{-}885\text{-}5p(Normal)) - 0.2791 \times (hsa\text{-}mir\text{-}139\text{-}3p(Tumor)) - 0.1839 \times (hsa\text{-}miR\text{-}223^{*}(Tumor)) \quad ......[formula4]$$

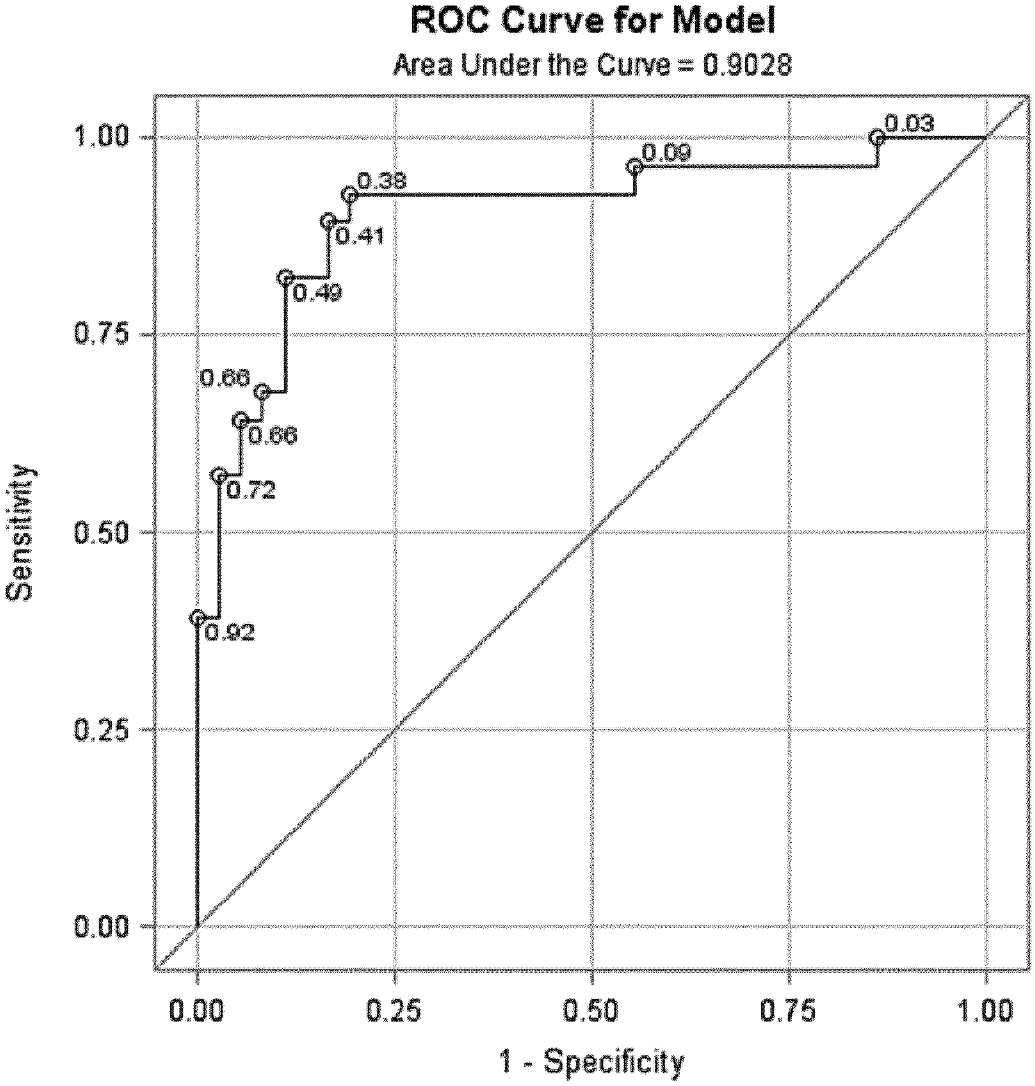


Fig.7

Recurrence score = exp( y)/(1 + exp( y)); in which y

$$y = 56.4146 - 2.9645 \times (hsa\text{-}miR\text{-}518b(Tumor)) - 0.7736 \times (hsa\text{-}miR\text{-}629(Normal))$$
$$-0.8220 \times (hsa\text{-}miR\text{-}885\text{-}5p(Normal)) - 0.2785 \times (hsa\text{-}mir\text{-}139\text{-}3p(Tumor)) - 3.1742 \times (hsa\text{-}miR\text{-}223^{*}(Tumor))$$
$$+0.2511 \times \{(hsa\text{-}miR\text{-}518b(Tumor)) \times (hsa\text{-}miR\text{-}223^{*}(Tumor))\} \text{ ......[formula5]}$$

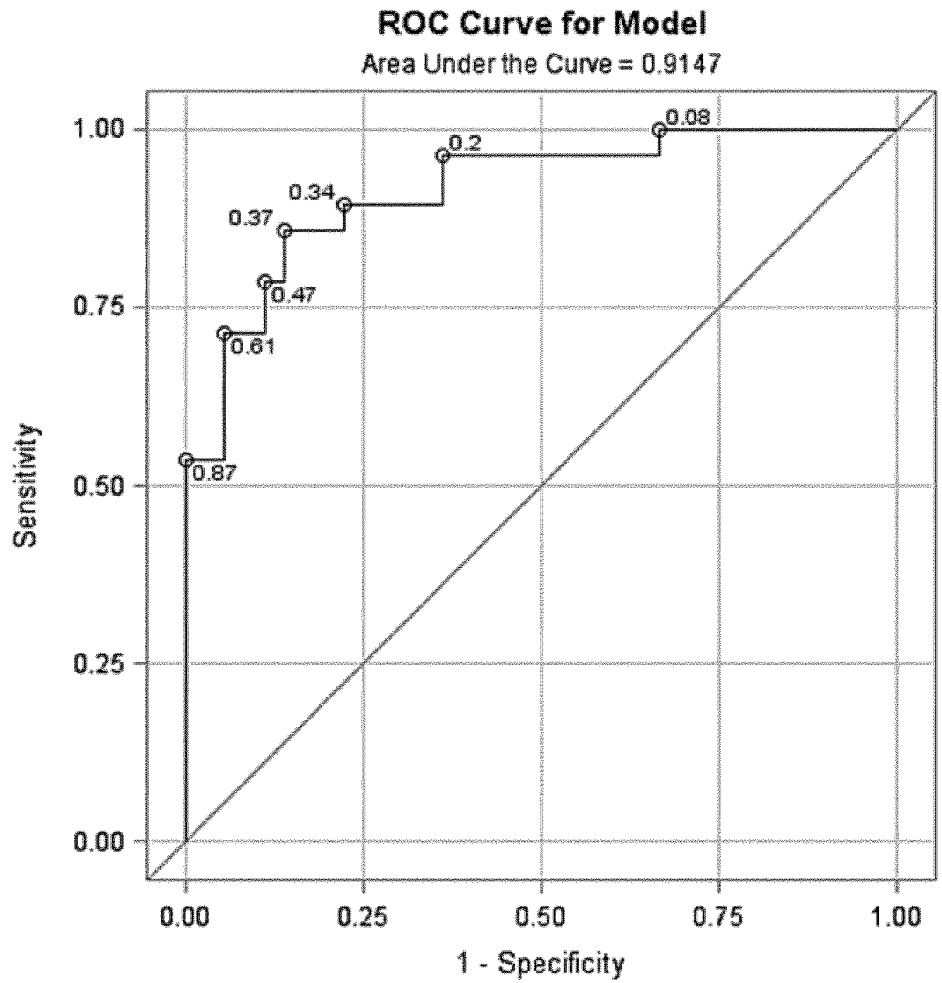


Fig.8

Recurrence score = exp( y)/(1 + exp( y)); in which y

$$y = 20.5432 + 0.4233 \times (hsa\text{-}miR\text{-}518b(Tumor)) + 2.9291 \times (hsa\text{-}miR\text{-}629(Normal))$$
$$-0.8806 \times (hsa\text{-}miR\text{-}885\text{-}5p(Normal)) - 0.2476 \times (hsa\text{-}mir\text{-}139\text{-}3p(Tumor))$$
$$-3.6438 \times (hsa\text{-}miR\text{-}223^*(Tumor)) + 0.2861 \times \{(hsa\text{-}miR\text{-}518b(Tumor) \times (hsa\text{-}miR\text{-}223^*(Tumor))\}$$
$$-0.3426 \times \{(hsa\text{-}miR\text{-}518b(Tumor) \times (hsa\text{-}miR\text{-}629(Normal))\} \ldots\ldots [\text{formula6}]$$

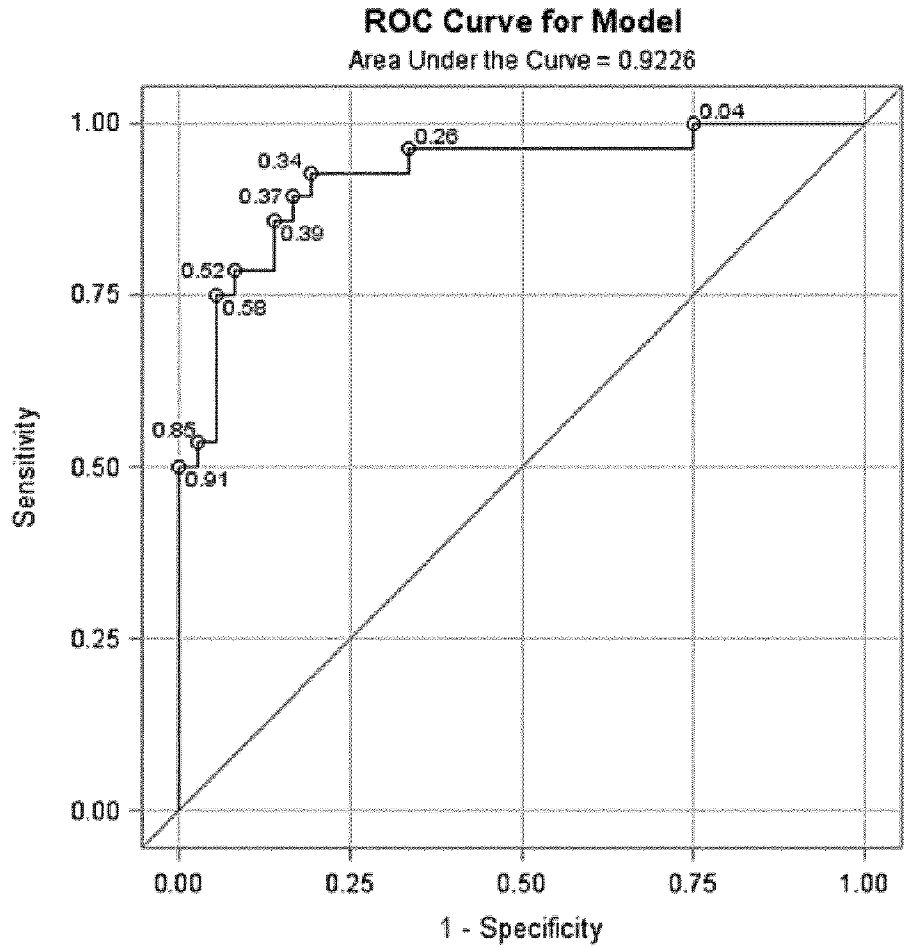


Fig.9

Recurrence score = exp( y)/(1 + exp( y)); in which y

$$y = -44.9363 + 0.5873 \times (hsa\text{-}miR\text{-}518b(Tumor)) + 10.5237 \times (hsa\text{-}miR\text{-}629(Normal))$$
$$-1.4451 \times (hsa\text{-}miR\text{-}885\text{-}5p(Normal)) - 0.2524 \times (hsa\text{-}mir\text{-}139\text{-}3p(Tumor))$$
$$+ 2.7393 \times (hsa\text{-}miR\text{-}223*(Tumor)) + 0.3606 \times \{(hsa\text{-}miR\text{-}518b(Tumor) \times (hsa\text{-}miR\text{-}223*(Tumor))\}$$
$$-0.4288 \times \{(hsa\text{-}miR\text{-}518b(Tumor) \times (hsa\text{-}miR\text{-}629(Normal))\}$$
$$-0.6967 \times \{(hsa\text{-}miR\text{-}629(Normal) \times (hsa\text{-}miR\text{-}223*(Tumor))\} \text{......[formula7]}$$

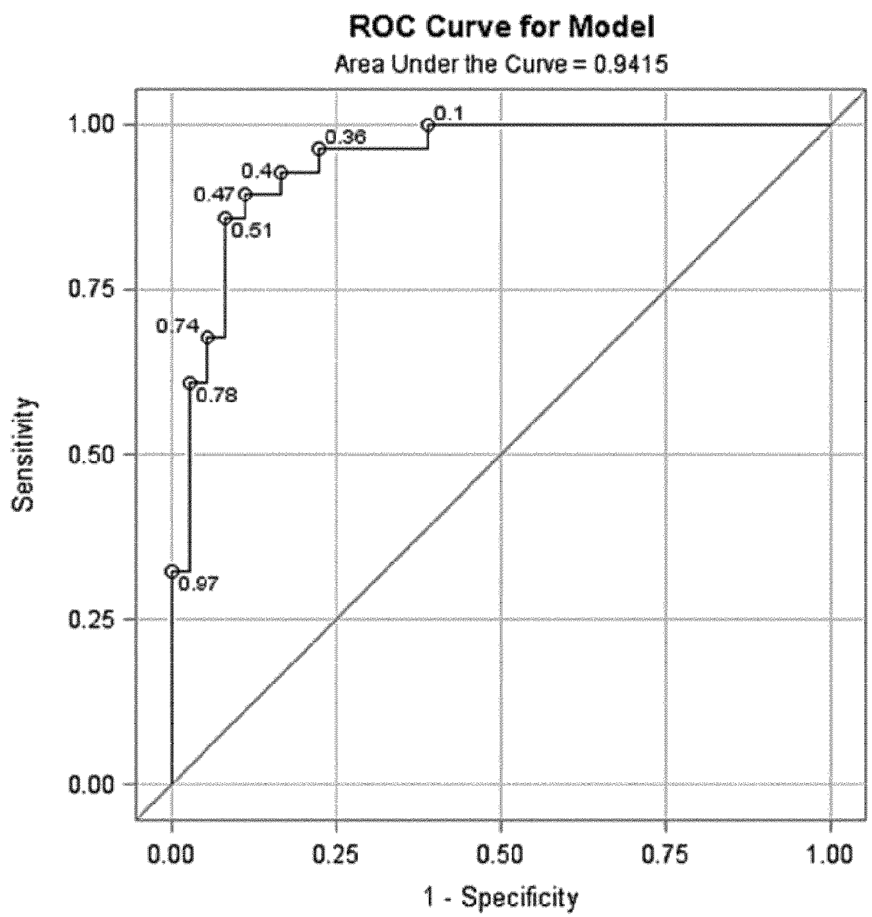


Fig.10

Recurrence score = exp( y)/(1 + exp( y)); in which y

$$y = -45.9840 + 0.5968 \times (hsa\text{-}miR\text{-}518b(Tumor)) + 11.4389 \times (hsa\text{-}miR\text{-}629(Normal))$$
$$-1.4526 \times (hsa\text{-}miR\text{-}885\text{-}5p(Normal)) - 1.1848 \times (hsa\text{-}mir\text{-}139\text{-}3p(Tumor))$$
$$+ 3.4236 \times (hsa\text{-}miR\text{-}223^{*}(Tumor)) + 0.3123 \times \{(hsa\text{-}miR\text{-}518b(Tumor) \times (hsa\text{-}miR\text{-}223^{*}(Tumor)))\}$$
$$-0.4994 \times \{(hsa\text{-}miR\text{-}518b(Tumor) \times (hsa\text{-}miR\text{-}629(Normal))\}$$
$$-0.7047 \times \{(hsa\text{-}miR\text{-}629(Normal) \times (hsa\text{-}miR\text{-}223^{*}(Tumor))\}$$
$$+ 0.0785 \times \{(hsa\text{-}miR\text{-}518b(Tumor) \times (hsa\text{-}mir\text{-}139\text{-}3p(Tumor))\} \ldots\ldots [formula8]$$

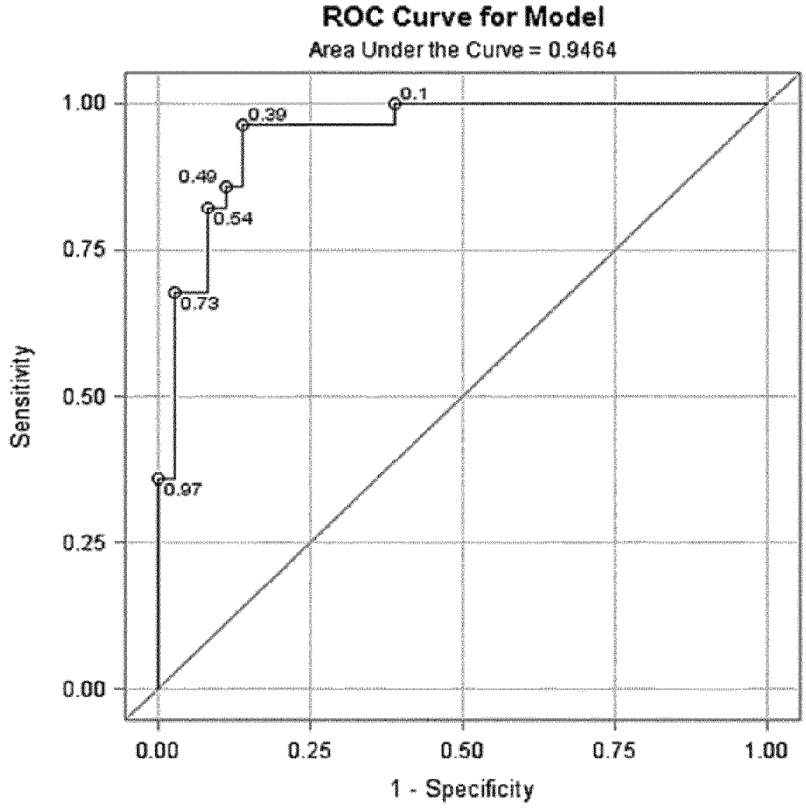


Fig.11

Recurrence score = exp( y)/(1 + exp( y)); in which y

$$y = -8.0836 + 0.5135 \times (hsa\text{-}miR\text{-}518b(Tumor)) + 10.0536 \times (hsa\text{-}miR\text{-}629(Normal))$$
$$-5.0937 \times (hsa\text{-}miR\text{-}885\text{-}5p(Normal)) - 2.6573 \times (hsa\text{-}mir\text{-}139\text{-}3p(Tumor)) + 2.8590 \times (hsa\text{-}miR\text{-}223^{*}(Tumor))$$
$$+ 0.3322 \times \{(hsa\text{-}miR\text{-}518b(Tumor) \times (hsa\text{-}miR\text{-}629(Normal))\}$$
$$-0.4003 \times \{(hsa\text{-}miR\text{-}518b(Tumor) \times (hsa\text{-}miR\text{-}223^{*}(Tumor))\}$$
$$-0.6740 \times \{(hsa\text{-}miR\text{-}629(Normal) \times (hsa\text{-}miR\text{-}223^{*}(Tumor))\}$$
$$+ 0.2530 \times \{(hsa\text{-}miR\text{-}885\text{-}5p(Normal) \times (hsa\text{-}mir\text{-}139\text{-}3p(Tumor))\} \ldots\ldots [formula9]$$

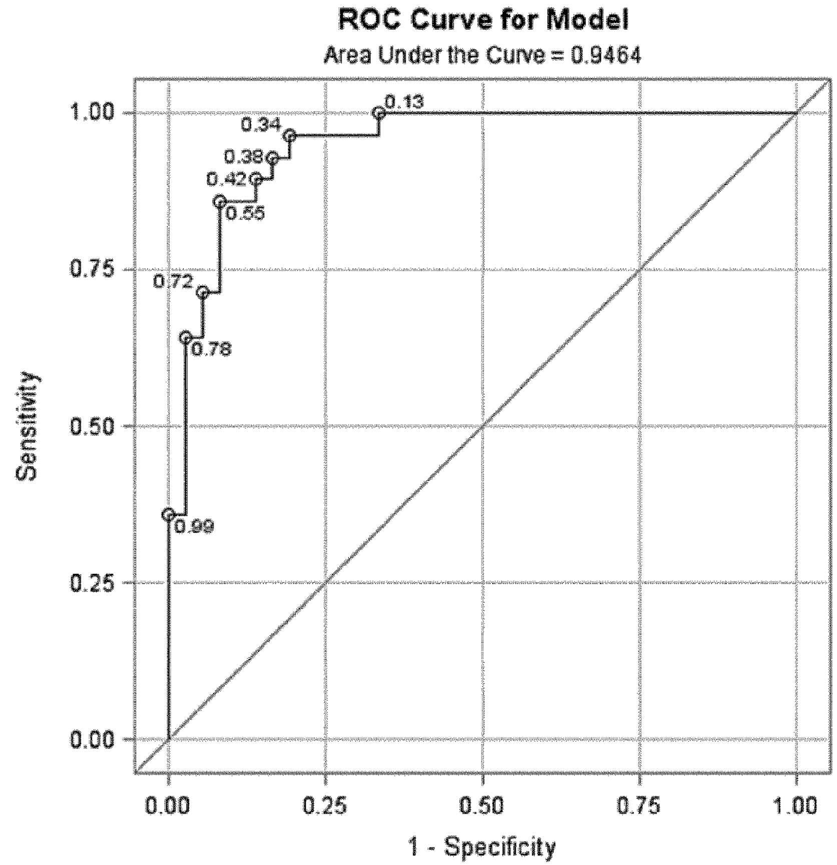


Fig.12

Recurrence score = exp( y)/(1 + exp( y)); in which y

$$y = 25.0054 - 0.8010 \times (hsa\text{-}miR\text{-}518b(Tumor)) + 0.5236 \times (hsa\text{-}miR\text{-}518b(Normal)) + 0.4554 \times (hsa\text{-}miR\text{-}629(Tumor)) - 1.1600 \times (hsa\text{-}miR\text{-}629(Normal)) - 1.0224 \times (hsa\text{-}miR\text{-}885\text{-}5p(Normal)) - 0.3805 \times (hsa\text{-}mir\text{-}139\text{-}3p(Tumor)) \ldots\ldots [formula10]$$

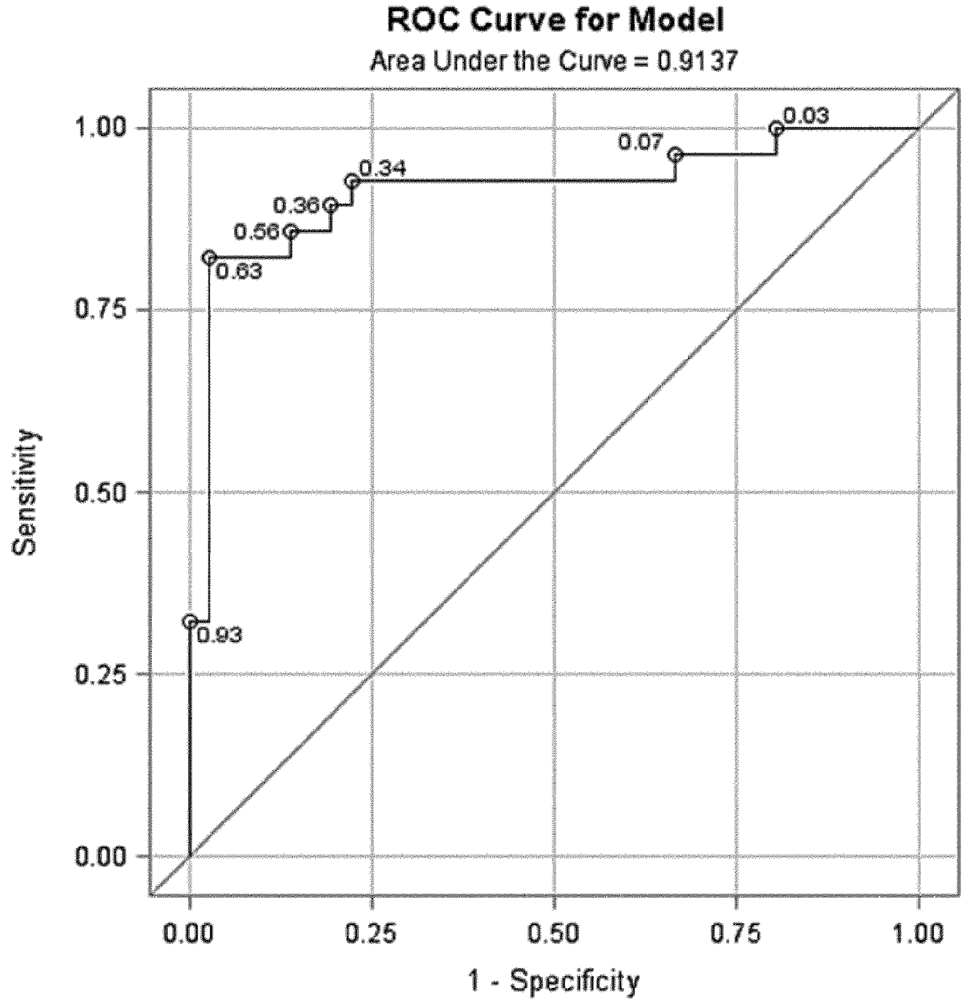


Fig.13

Recurrence score = exp( y)/(1 + exp( y)); in which y

$$y = -61.6705 + 6.8619 \times (hsa\text{-}miR\text{-}518b(Tumor)) + 0.6035 \times (hsa\text{-}miR\text{-}518b(Normal)) + 0.4214 \times (hsa\text{-}miR\text{-}629(Tumor)) + 7.2344 \times (hsa\text{-}miR\text{-}629(Normal)) - 1.0410 \times (hsa\text{-}miR\text{-}885\text{-}5p(Normal)) - 0.3520 \times (hsa\text{-}mir\text{-}139\text{-}3p(Tumor)) - 0.7476 \times \{(hsa\text{-}miR\text{-}518b(Tumor)) \times (hsa\text{-}miR\text{-}629(Normal))\} \ldots\ldots [formula 11]$$

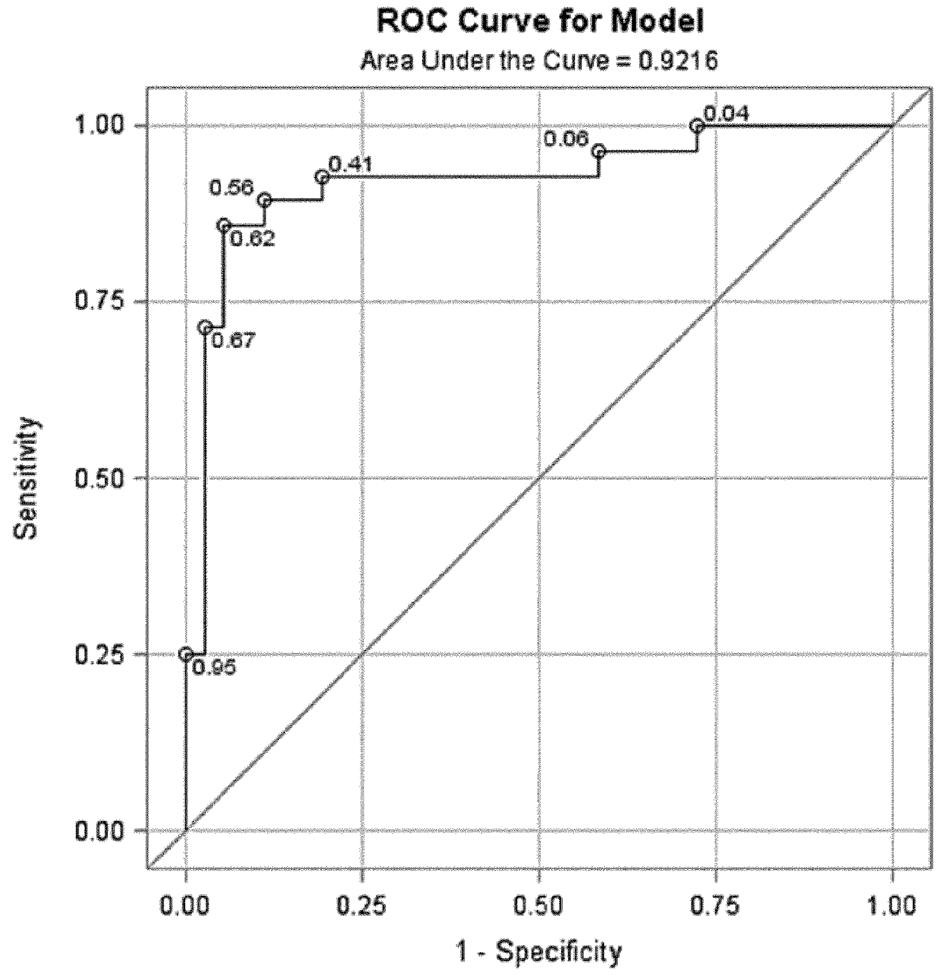


Fig.14

Recurrence score = exp( y)/(1 + exp( y)); in which y

$$y = -8.3254 - 0.7934 \times (hsa\text{-}miR\text{-}518b(Tumor)) + 3.4682 \times (hsa\text{-}miR\text{-}518b(Normal)) + 0.5218 \times (hsa\text{-}miR\text{-}629(Tumor)) + 2.0833 \times (hsa\text{-}miR\text{-}629(Normal)) - 1.0455 \times (hsa\text{-}miR\text{-}885\text{-}5p(Normal)) - 0.4284 \times (hsa\text{-}mir\text{-}139\text{-}3p(Tumor)) - 0.2842 \times \{(hsa\text{-}miR\text{-}518b(Normal)) \times (hsa\text{-}miR\text{-}629(Normal))\} \text{......[formula12]}$$

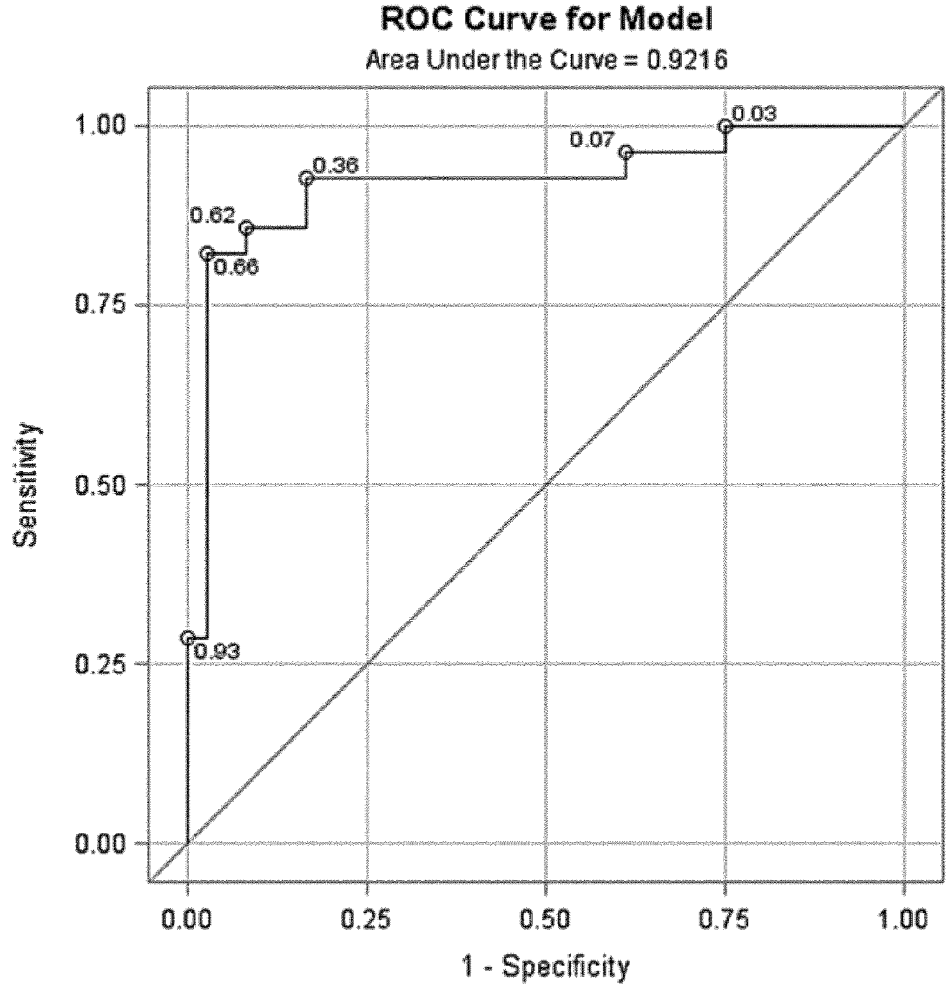


Fig.15

Recurrence score = exp( y)/(1 + exp( y)); in which y

$$y = -9.2695 + 17.0029 \times (hsa\text{-}miR\text{-}518b(Tumor)) + 0.6271 \times (hsa\text{-}miR\text{-}518b(Normal)) - 13.5446 \times (hsa\text{-}miR\text{-}629(Tumor))$$
$$+ 2.9234 \times (hsa\text{-}miR\text{-}629(Normal)) - 1.8225 \times (hsa\text{-}miR\text{-}885\text{-}5p(Normal)) - 0.4412 \times (hsa\text{-}mir\text{-}139\text{-}3p(Tumor))$$
$$-1.7187 \times \{(hsa\text{-}miR\text{-}518b(Tumor)) \times (hsa\text{-}miR\text{-}629(Normal))\}$$
$$+ 1.3433 \times \{(hsa\text{-}miR\text{-}629(Tumor)) \times (hsa\text{-}miR\text{-}629(Normal))\}$$ ......[formula13]

Fig.16

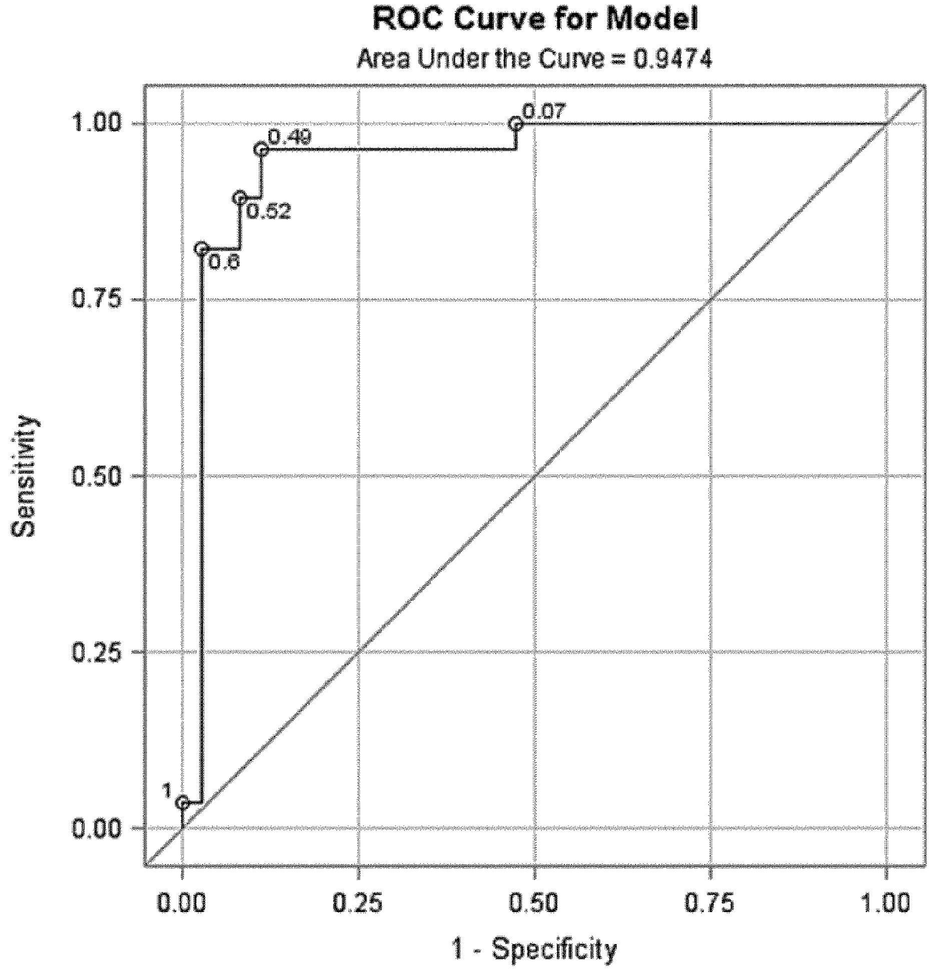

Recurrence score = exp( y)/(1 + exp( y)); in which y

$$y = 30.1620 + 15.5901 \times (hsa\text{-}miR\text{-}518b(Tumor)) + 0.8399 \times (hsa\text{-}miR\text{-}518b(Normal))$$
$$-15.4014 \times (hsa\text{-}miR\text{-}629(Tumor)) + 5.8744 \times (hsa\text{-}miR\text{-}629(Normal))$$
$$-9.4485 \times (hsa\text{-}miR\text{-}885\text{-}5p(Normal)) - 0.4926 \times (hsa\text{-}mir\text{-}139\text{-}3p(Tumor))$$
$$-2.1819 \times \{(hsa\text{-}miR\text{-}518b(Tumor)) \times (hsa\text{-}miR\text{-}629(Normal))\}$$
$$+1.5321 \times \{(hsa\text{-}miR\text{-}629(Tumor)) \times (hsa\text{-}miR\text{-}629(Normal))\}$$
$$+0.6491 \times \{(hsa\text{-}miR\text{-}518b(Tumor)) \times (hsa\text{-}miR\text{-}518b(Normal))\} ......[formula14]$$

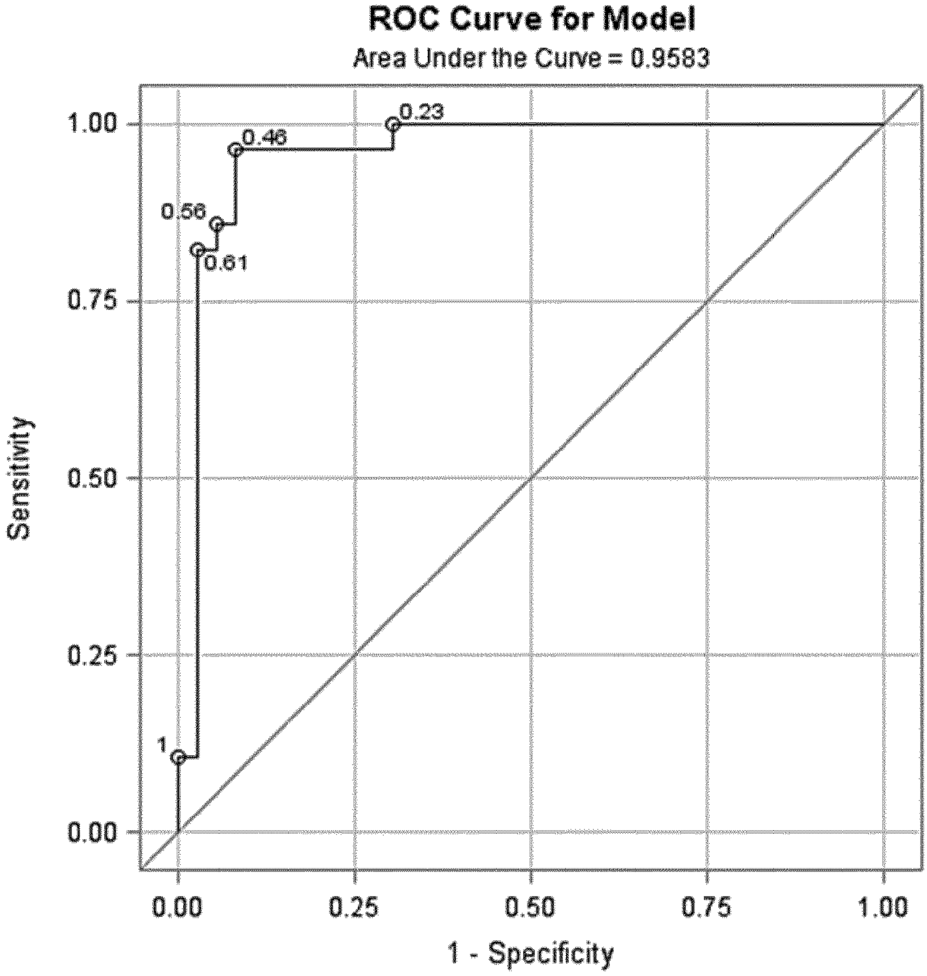


Fig.17

Recurrence score = exp( y)/(1 + exp( y)); in which y

$$y = 29.5776 + 16.6038 \times (hsa\text{-}miR\text{-}518b(Tumor)) - 2.0593 \times (hsa\text{-}miR\text{-}518b(Normal))$$
$$-16.3607 \times (hsa\text{-}miR\text{-}629(Tumor)) + 7.9768 \times (hsa\text{-}miR\text{-}629(Normal))$$
$$-8.0058 \times (hsa\text{-}miR\text{-}885\text{-}5p(Normal)) - 0.4868 \times (hsa\text{-}mir\text{-}139\text{-}3p(Tumor))$$
$$-2.4573 \times \{(hsa\text{-}miR\text{-}518b(Tumor)) \times (hsa\text{-}miR\text{-}629(Normal))\}$$
$$+1.6249 \times \{(hsa\text{-}miR\text{-}629(Tumor)) \times (hsa\text{-}miR\text{-}629(Normal))\}$$
$$+0.2507 \times \{(hsa\text{-}miR\text{-}518b(Tumor)) \times (hsa\text{-}miR\text{-}518b(Normal))\}$$
$$+0.5228 \times \{(hsa\text{-}miR\text{-}518b(Tumor)) \times (hsa\text{-}miR\text{-}885\text{-}5p(Normal))\} ......[formula15]$$

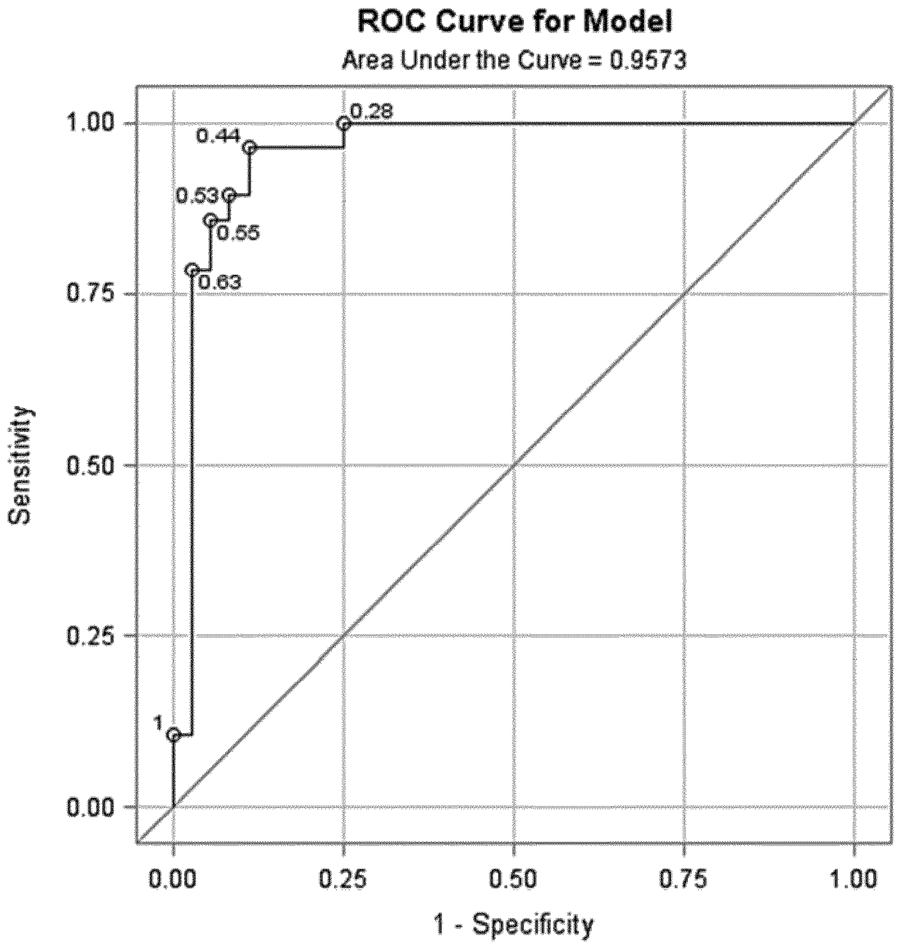


Fig.18

Recurrence score = exp( y)/(1 + exp( y)); in which y

$$y = 15.8547 + 19.7928 \times (hsa\text{-}miR\text{-}518b(Tumor)) - 4.0096 \times (hsa\text{-}miR\text{-}518b(Normal))$$
$$-14.4251 \times (hsa\text{-}miR\text{-}629(Tumor)) + 10.5888 \times (hsa\text{-}miR\text{-}629(Normal)) - 6.8641 \times (hsa\text{-}miR\text{-}885\text{-}5p(Normal))$$
$$-3.7748 \times (hsa\text{-}mir\text{-}139\text{-}3p(Tumor)) - 2.4644 \times \{(hsa\text{-}miR\text{-}518b(Tumor)) \times (hsa\text{-}miR\text{-}629(Normal))\}$$
$$+ 1.4285 \times \{(hsa\text{-}miR\text{-}629(Tumor)) \times (hsa\text{-}miR\text{-}629(Normal))\}$$
$$+ 0.4009 \times \{(hsa\text{-}miR\text{-}518b(Tumor)) \times (hsa\text{-}miR\text{-}518b(Normal))\}$$
$$+ 0.3535 \times \{(hsa\text{-}miR\text{-}885\text{-}5p(Normal)) \times (hsa\text{-}mir\text{-}139\text{-}3p(Tumor))\} ......[formula16]$$

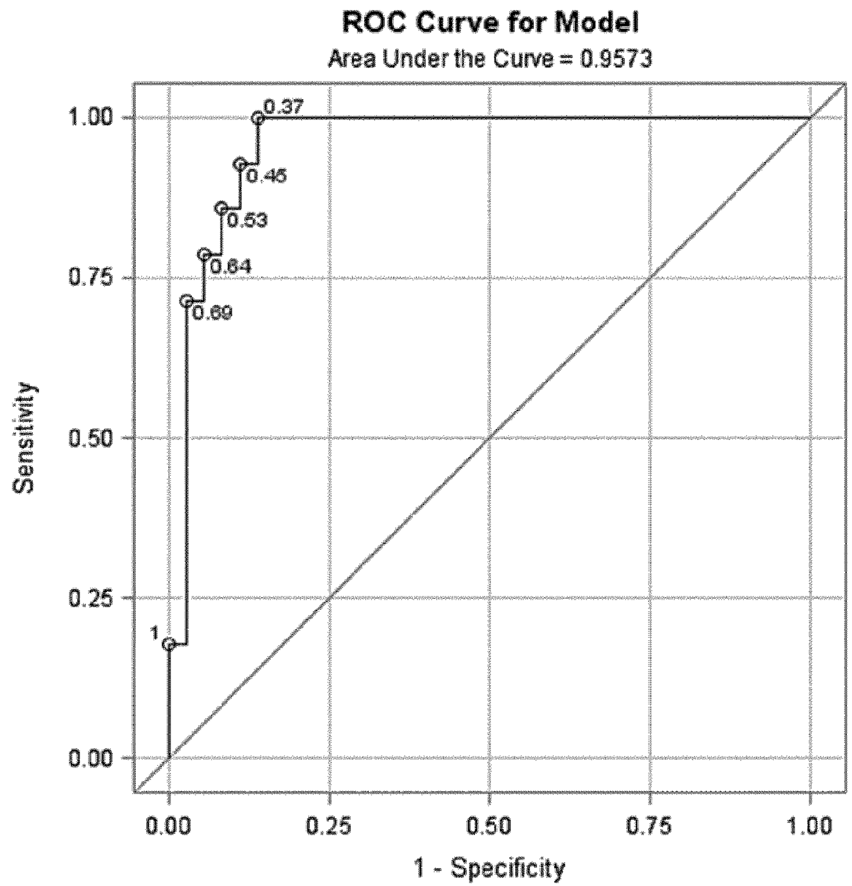


Fig.19

Recurrence score = exp( y)/(1 + exp( y)); in which y

$$y = 25.2101 - 0.8181 \times (hsa\text{-}miR\text{-}518b(Tumor)) + 0.5577 \times (hsa\text{-}miR\text{-}518b(Normal)) + 0.4613 \times (hsa\text{-}miR\text{-}629(Tumor)) - 1.1740 \times (hsa\text{-}miR\text{-}629(Normal)) - 1.0339 \times (hsa\text{-}miR\text{-}885\text{-}5p(Normal)) - 0.3634 \times (hsa\text{-}mir\text{-}139\text{-}3p(Tumor)) - 0.0461 \times (hsa\text{-}mir\text{-}139\text{-}3p(Normal)) \ldots\ldots [formula17]$$

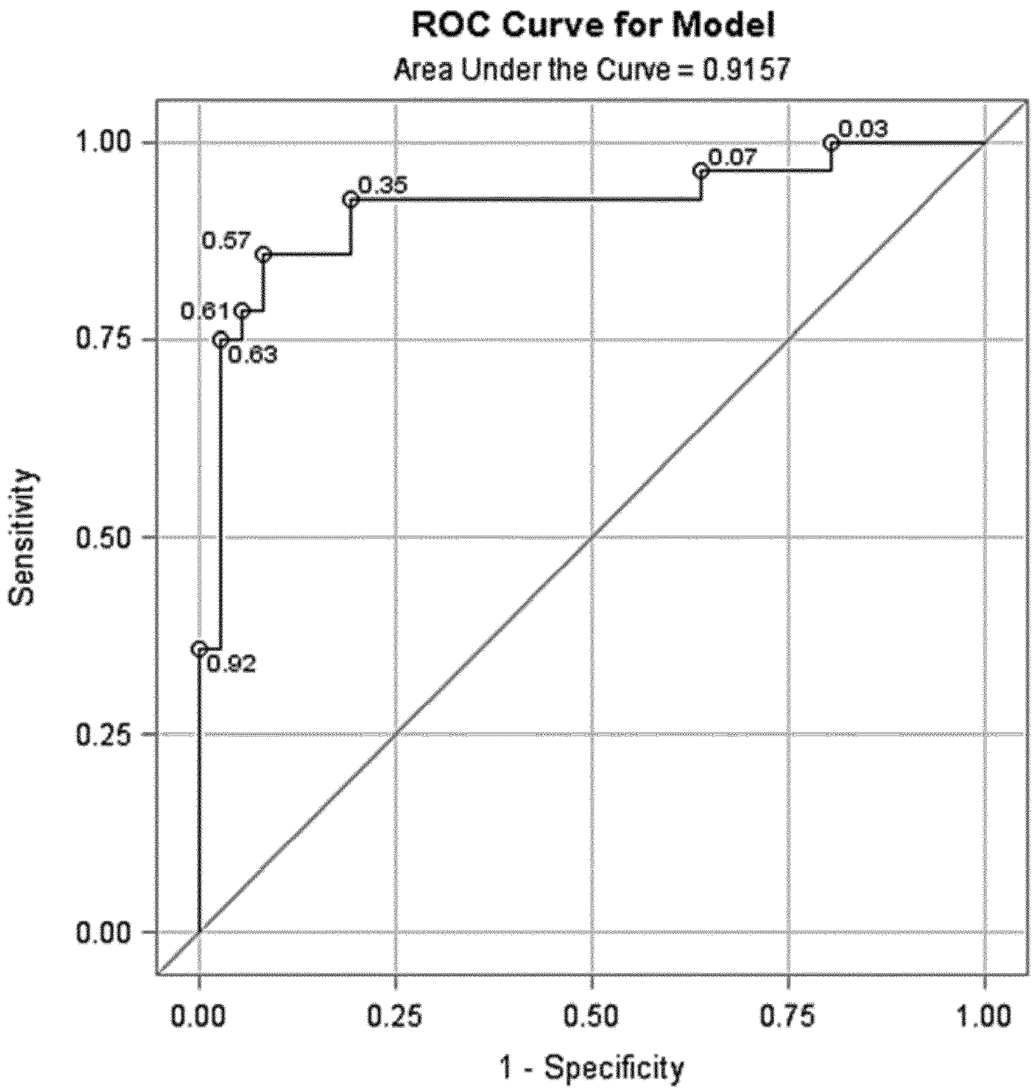


Fig.20

Recurrence score = exp( y)/(1 + exp( y)); in which y

$$y = -61.7241 + 6.8625 \times (hsa\text{-}miR\text{-}518b(Tumor)) + 0.6350 \times (hsa\text{-}miR\text{-}518b(Normal)) + 0.4261 \times (hsa\text{-}miR\text{-}629(Tumor)) + 7.2459 \times (hsa\text{-}miR\text{-}629(Normal)) - 1.0519 \times (hsa\text{-}miR\text{-}885\text{-}5p(Normal)) - 0.3365 \times (hsa\text{-}mir\text{-}139\text{-}3p(Tumor)) - 0.0403 \times (hsa\text{-}mir\text{-}139\text{-}3p(Normal)) - 0.7493 \times \{(hsa\text{-}miR\text{-}518b(Tumor)) \times (hsa\text{-}miR\text{-}629(Normal))\}$$ ......[formula18]

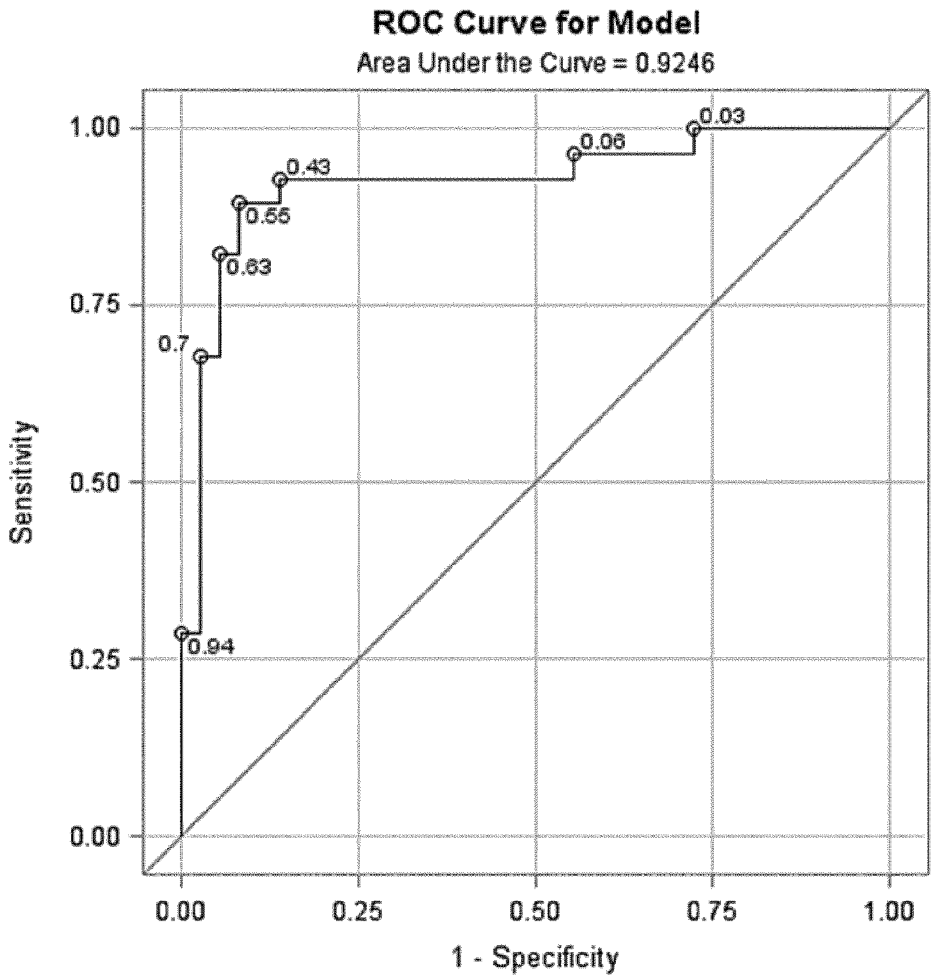


Fig.21

Recurrence score = exp( y)/(1 + exp( y)); in which y

$$y = -8.9599 + 17.2942 \times (hsa\text{-}miR\text{-}518b(Tumor)) + 0.6839 \times (hsa\text{-}miR\text{-}518b(Normal))$$
$$-13.8554 \times (hsa\text{-}miR\text{-}629(Tumor)) + 2.9395 \times (hsa\text{-}miR\text{-}629(Normal)) - 1.8605 \times (hsa\text{-}miR\text{-}885\text{-}5p(Normal))$$
$$-0.4172 \times (hsa\text{-}mir\text{-}139\text{-}3p(Tumor)) - 0.0740 \times (hsa\text{-}mir\text{-}139\text{-}3p(Normal))$$
$$-1.7502 \times \{(hsa\text{-}miR\text{-}518b(Tumor) \times (hsa\text{-}miR\text{-}629(Normal)))\}$$
$$+ 1.3733 \times \{(hsa\text{-}miR\text{-}629(Tumor)) \times (hsa\text{-}miR\text{-}629(Normal))\} \ldots\ldots[formula19]$$

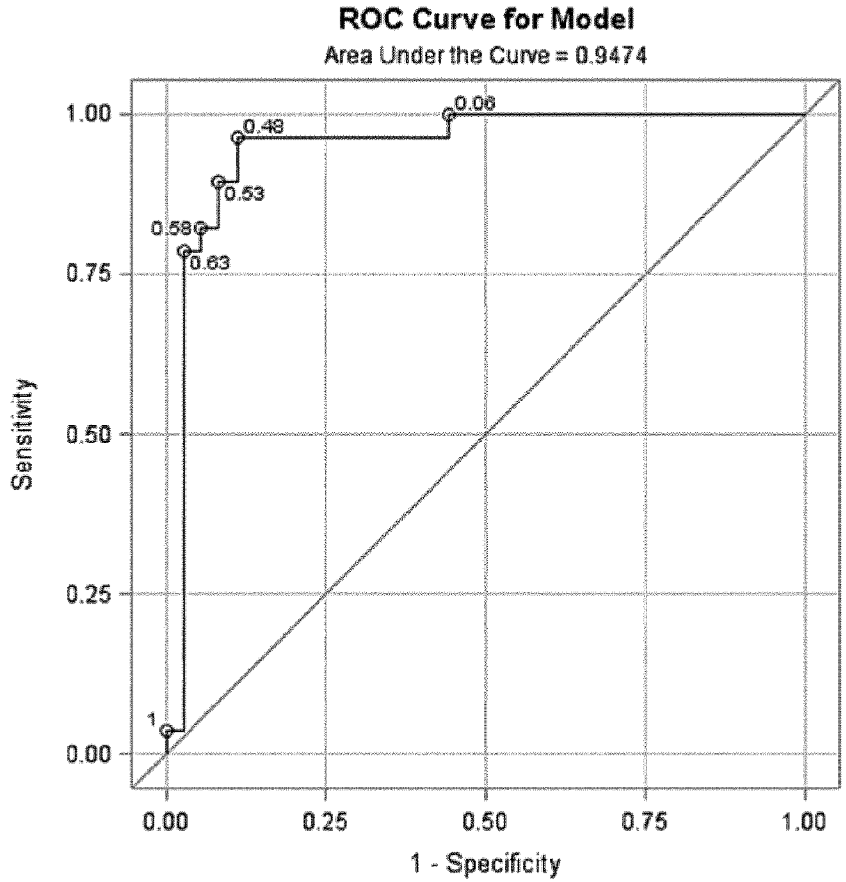


Fig.22

Recurrence score = exp( y)/(1 + exp( y)); in which y

$$y = 31.7485 + 15.9189 \times (hsa\text{-}miR\text{-}518b(Tumor)) + 0.9124 \times (hsa\text{-}miR\text{-}518b(Normal))$$
$$-15.8373 \times (hsa\text{-}miR\text{-}629(Tumor)) + 5.9588 \times (hsa\text{-}miR\text{-}629(Normal)) - 9.6770 \times (hsa\text{-}miR\text{-}885\text{-}5p(Normal))$$
$$-0.4717 \times (hsa\text{-}mir\text{-}139\text{-}3p(Tumor)) - 0.0887 \times (hsa\text{-}mir\text{-}139\text{-}3p(Normal))$$
$$-2.2334 \times \{(hsa\text{-}miR\text{-}518b(Tumor) \times (hsa\text{-}miR\text{-}629(Normal)))\}$$
$$+1.5743 \times \{(hsa\text{-}miR\text{-}629(Tumor)) \times (hsa\text{-}miR\text{-}629(Normal))\}$$
$$+0.6653 \times \{(hsa\text{-}miR\text{-}518b(Tumor)) \times (hsa\text{-}miR\text{-}885\text{-}5p(Normal))\} \ldots\ldots [formula20]$$

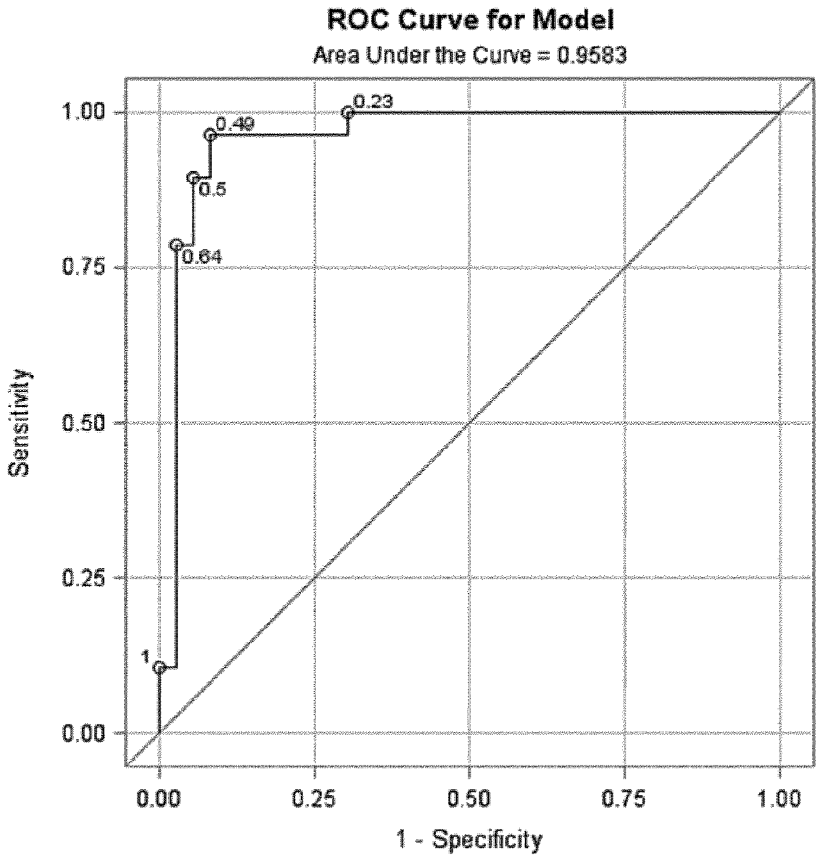


Fig.23

Recurrence score = exp( y)/(1 + exp( y)); in which y

$$y = 128.6 + 21.7977 \times (hsa\text{-}miR\text{-}518b(Tumor)) + 1.4314 \times (hsa\text{-}miR\text{-}518b(Normal))$$
$$-23.0029 \times (hsa\text{-}miR\text{-}629(Tumor)) + 7.1152 \times (hsa\text{-}miR\text{-}629(Normal)) - 20.8193 \times (hsa\text{-}miR\text{-}885\text{-}5p(Normal))$$
$$-0.5185 \times (hsa\text{-}mir\text{-}139\text{-}3p(Tumor)) - 7.2706 \times (hsa\text{-}mir\text{-}139\text{-}3p(Normal))$$
$$-3.0939 \times \{(hsa\text{-}miR\text{-}518b(Tumor) \times (hsa\text{-}miR\text{-}629(Normal)))\}$$
$$+ 2.2659 \times \{(hsa\text{-}miR\text{-}629(Tumor)) \times (hsa\text{-}miR\text{-}629(Normal))\}$$
$$+ 0.9353 \times \{(hsa\text{-}miR\text{-}518b(Tumor)) \times (hsa\text{-}miR\text{-}885\text{-}5p(Normal))\}$$
$$+ 0.7224 \times \{(hsa\text{-}miR\text{-}885\text{-}5p(Normal)) \times (hsa\text{-}mir\text{-}139\text{-}3p(Normal))\} \ldots\ldots [formula21]$$

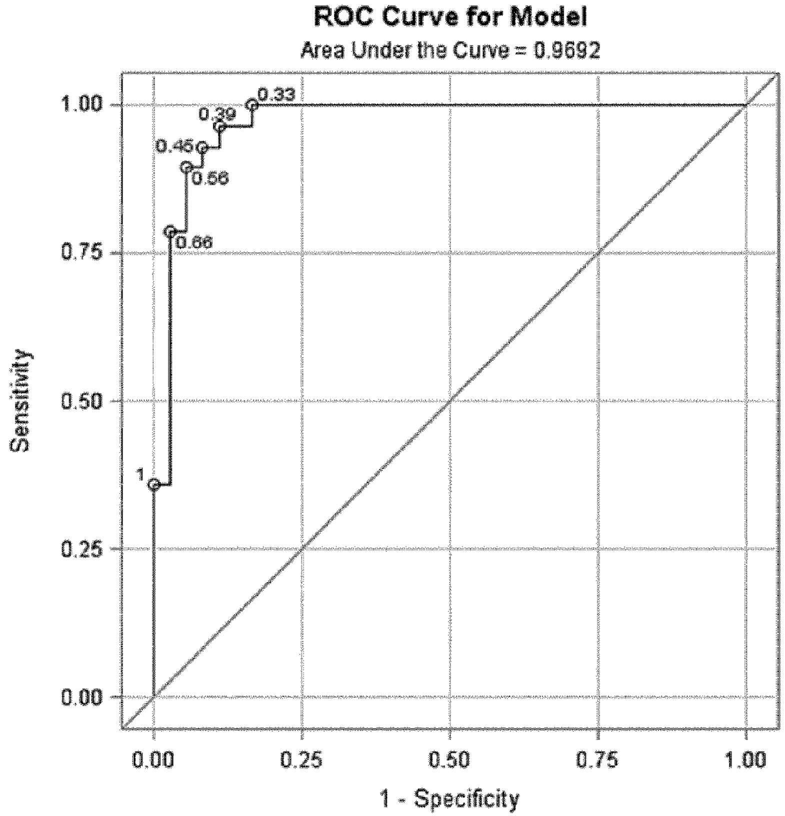


Fig.24

Recurrence score = exp( y)/(1 + exp( y)); in which y

$$y = 26.2637 - 0.7199 \times (hsa\text{-}miR\text{-}518b(Tumor)) + 0.6026 \times (hsa\text{-}miR\text{-}518b(Normal)) + 0.3850 \times (hsa\text{-}miR\text{-}629(Tumor)) - 1.2722 \times (hsa\text{-}miR\text{-}629(Normal)) - 0.9709 \times (hsa\text{-}miR\text{-}885\text{-}5p(Normal)) - 0.2920 \times (hsa\text{-}mir\text{-}139\text{-}3p(Tumor)) - 0.1035 \times (hsa\text{-}mir\text{-}139\text{-}3p(Normal)) - 0.1892 \times (hsa\text{-}miR\text{-}223^{*}(Tumor))$$ ......[formula22]

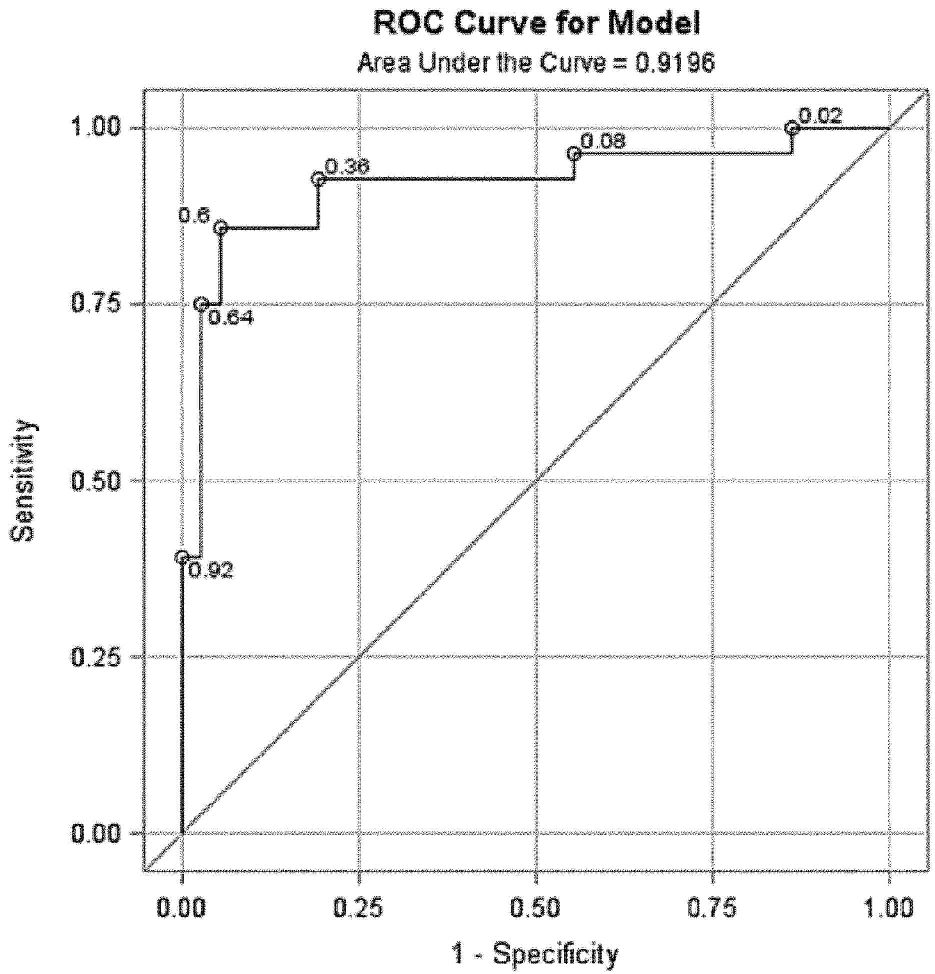


Fig.25

Recurrence score = exp( y)/(1 + exp( y)); in which y

$$y = -68.7954 + 7.6940 \times (hsa\text{-}miR\text{-}518b(Tumor)) + 0.6949 \times (hsa\text{-}miR\text{-}518b(Normal)) + 0.3207 \times (hsa\text{-}miR\text{-}629(Tumor)) + 7.9639 \times (hsa\text{-}miR\text{-}629(Normal)) - 0.9677 \times (hsa\text{-}miR\text{-}885\text{-}5p(Normal)) - 0.2519 \times (hsa\text{-}mir\text{-}139\text{-}3p(Tumor)) - 0.1122 \times (hsa\text{-}mir\text{-}139\text{-}3p(Normal)) - 0.2193 \times (hsa\text{-}miR\text{-}223*(Tumor)) - 0.8210 \times \{(hsa\text{-}miR\text{-}518b(Tumor) \times (hsa\text{-}miR\text{-}629(Normal))\} \text{......[formula23]}$$

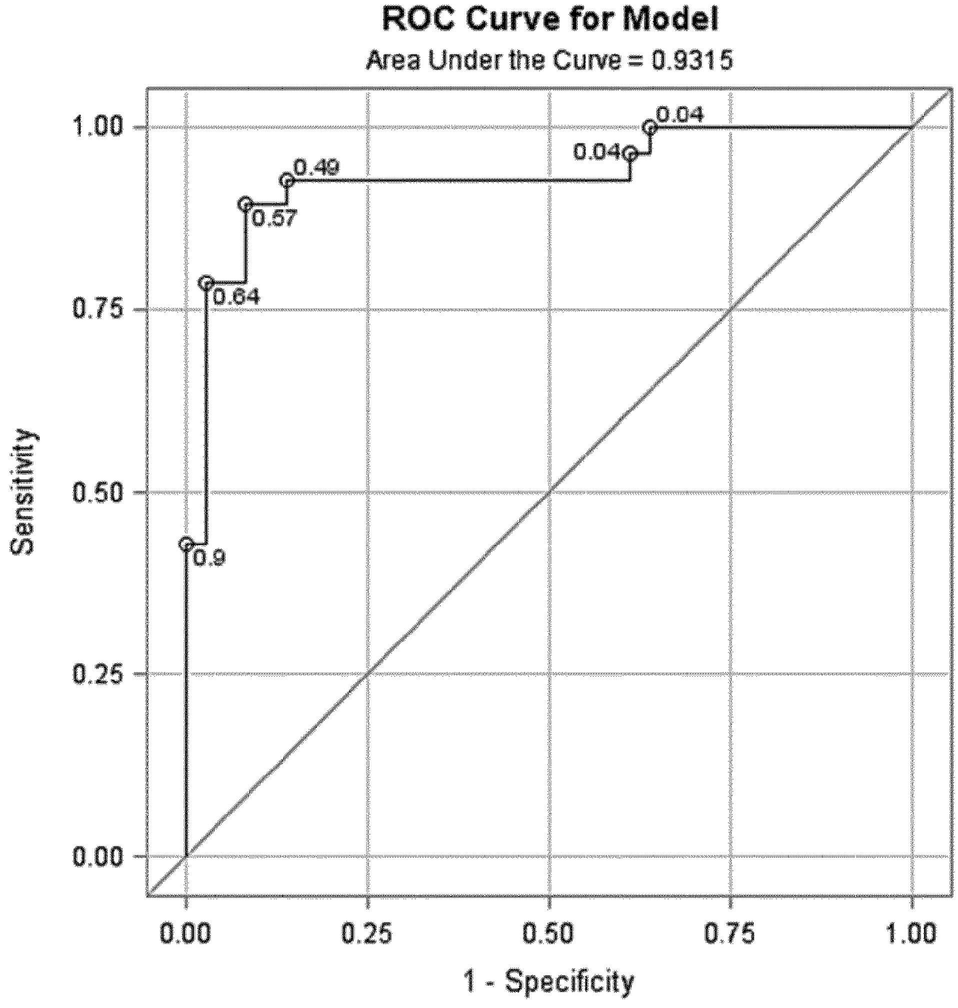


Fig.26

Recurrence score = exp( y)/(1 + exp( y)); in which y

$$y = -17.0971 + 17.3647 \times (hsa\text{-}miR\text{-}518b(Tumor)) + 0.7216 \times (hsa\text{-}miR\text{-}518b(Normal))$$
$$-13.2005 \times (hsa\text{-}miR\text{-}629(Tumor)) + 3.6886 \times (hsa\text{-}miR\text{-}629(Normal))$$
$$-1.7075 \times (hsa\text{-}miR\text{-}885\text{-}5p(Normal)) - 0.3346 \times (hsa\text{-}mir\text{-}139\text{-}3p(Tumor))$$
$$-0.1380 \times (hsa\text{-}mir\text{-}139\text{-}3p(Normal)) - 0.1915 \times (hsa\text{-}miR\text{-}223^{*}(Tumor))$$
$$-1.7493 \times \{(hsa\text{-}miR\text{-}518b(Tumor) \times (hsa\text{-}miR\text{-}629(Normal))\}$$
$$+1.3013 \times \{(hsa\text{-}miR\text{-}629(Tumor) \times (hsa\text{-}miR\text{-}629(Normal))\} ......[formula24]$$

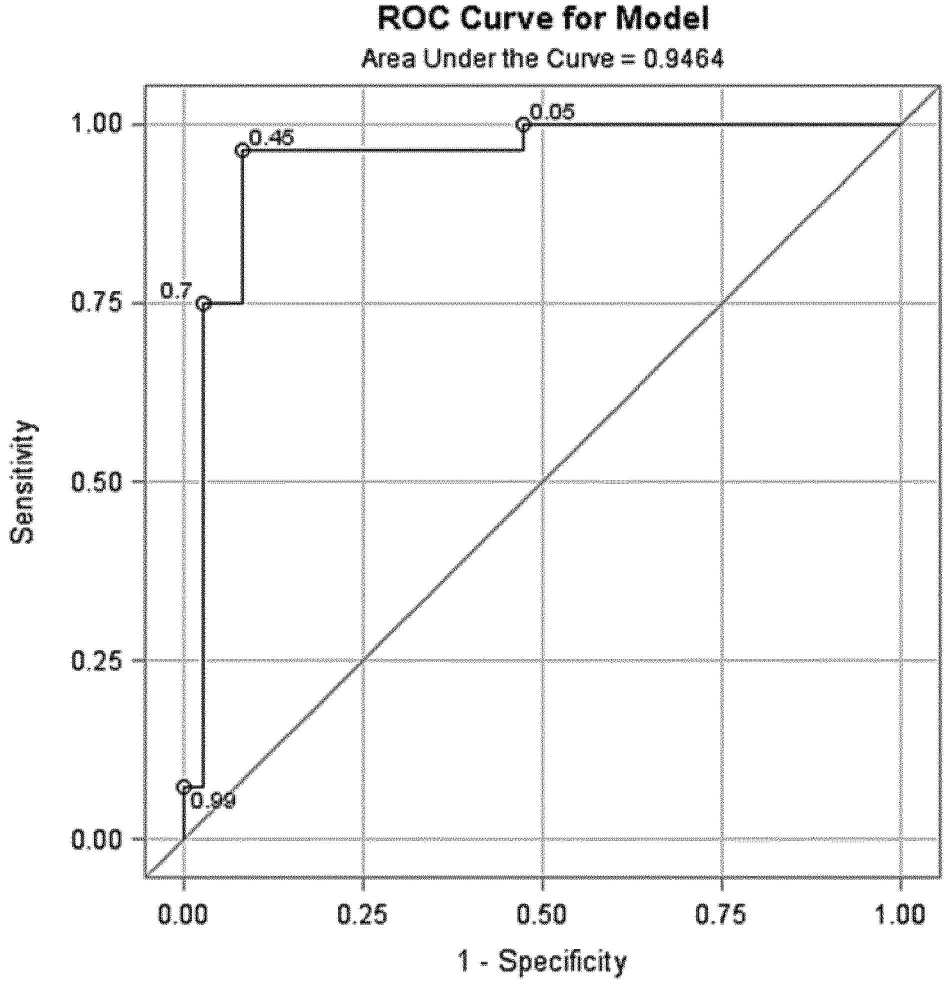


Fig.27

Recurrence score = exp( y)/(1 + exp( y)); in which y

$$y = 55.5037 + 16.5018 \times (hsa\text{-}miR\text{-}518b(Tumor)) + 1.5212 \times (hsa\text{-}miR\text{-}518b(Normal))$$
$$-18.5036 \times (hsa\text{-}miR\text{-}629(Tumor)) + 4.2862 \times (hsa\text{-}miR\text{-}629(Normal))$$
$$-2.4828 \times (hsa\text{-}miR\text{-}885\text{-}5p(Normal)) - 0.3196 \times (hsa\text{-}mir\text{-}139\text{-}3p(Tumor))$$
$$-0.4981 \times (hsa\text{-}mir\text{-}139\text{-}3p(Normal)) - 7.0990 \times (hsa\text{-}miR\text{-}223^{*}(Tumor))$$
$$-2.3513 \times \{(hsa\text{-}miR\text{-}518b(Tumor) \times (hsa\text{-}miR\text{-}629(Normal))\}$$
$$+1.8452 \times \{(hsa\text{-}miR\text{-}629(Tumor) \times (hsa\text{-}miR\text{-}629(Normal))\}$$
$$+0.5877 \times \{(hsa\text{-}miR\text{-}518b(Tumor)) \times (hsa\text{-}miR\text{-}223^{*}(Tumor))\} \ldots\ldots[\text{formula25}]$$

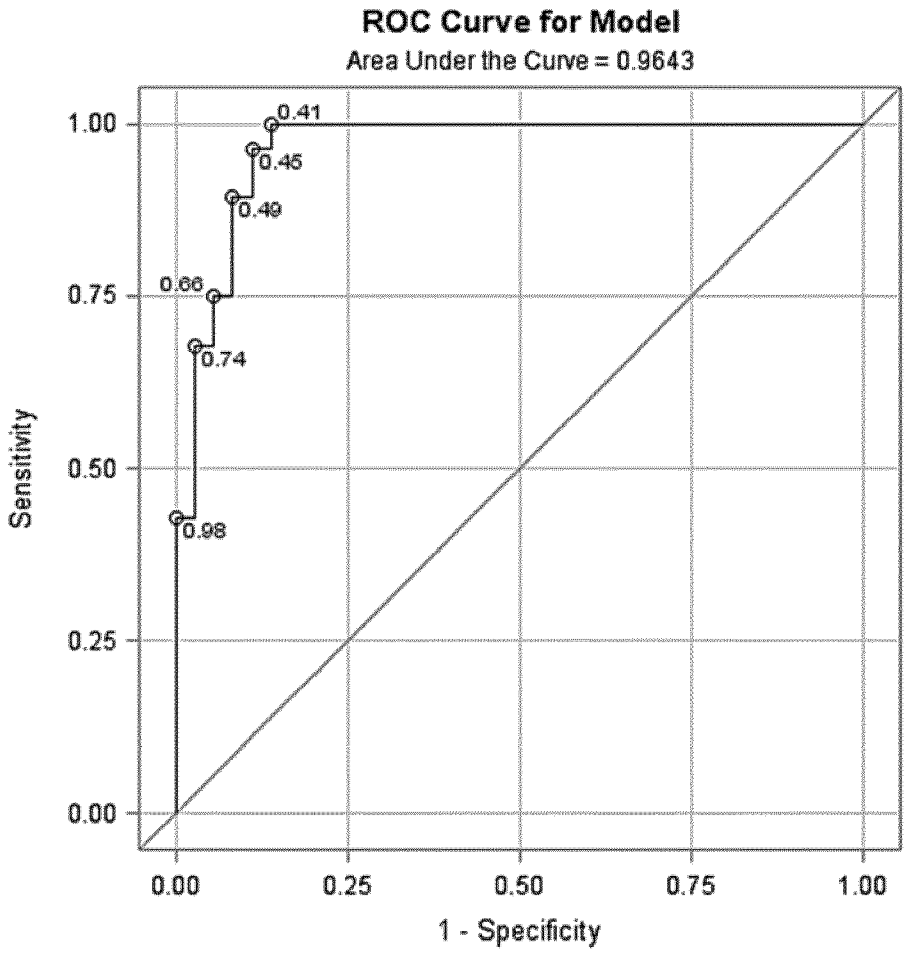


Fig.28

Recurrence score = exp( y)/(1 + exp( y)); in which y

$$y = 294.8 - 18.4197 \times (hsa\text{-}miR\text{-}518b(Tumor)) + 40.6962 \times (hsa\text{-}miR\text{-}518b(Normal))$$
$$-16.0046 \times (hsa\text{-}miR\text{-}629(Tumor)) - 33.3077 \times (hsa\text{-}miR\text{-}629(Normal)) - 3.4433 \times (hsa\text{-}miR\text{-}885\text{-}5p(Normal))$$
$$-0.5615 \times (hsa\text{-}mir\text{-}139\text{-}3p(Tumor)) - 1.8451 \times (hsa\text{-}mir\text{-}139\text{-}3p(Normal)) - 18.4236 \times (hsa\text{-}miR\text{-}223^{*}(Tumor))$$
$$-1.3689 \times \{(hsa\text{-}miR\text{-}518b(Tumor) \times (hsa\text{-}miR\text{-}629(Normal))\}$$
$$+3.8986 \times \{(hsa\text{-}miR\text{-}629(Tumor) \times (hsa\text{-}miR\text{-}629(Normal))\}$$
$$+1.4698 \times \{(hsa\text{-}miR\text{-}518b(Tumor)) \times (hsa\text{-}miR\text{-}223^{*}(Tumor))\}$$
$$-1.9122 \times \{(hsa\text{-}miR\text{-}518b(Normal)) \times (hsa\text{-}miR\text{-}629(Tumor))\} \text{......[formula26]}$$

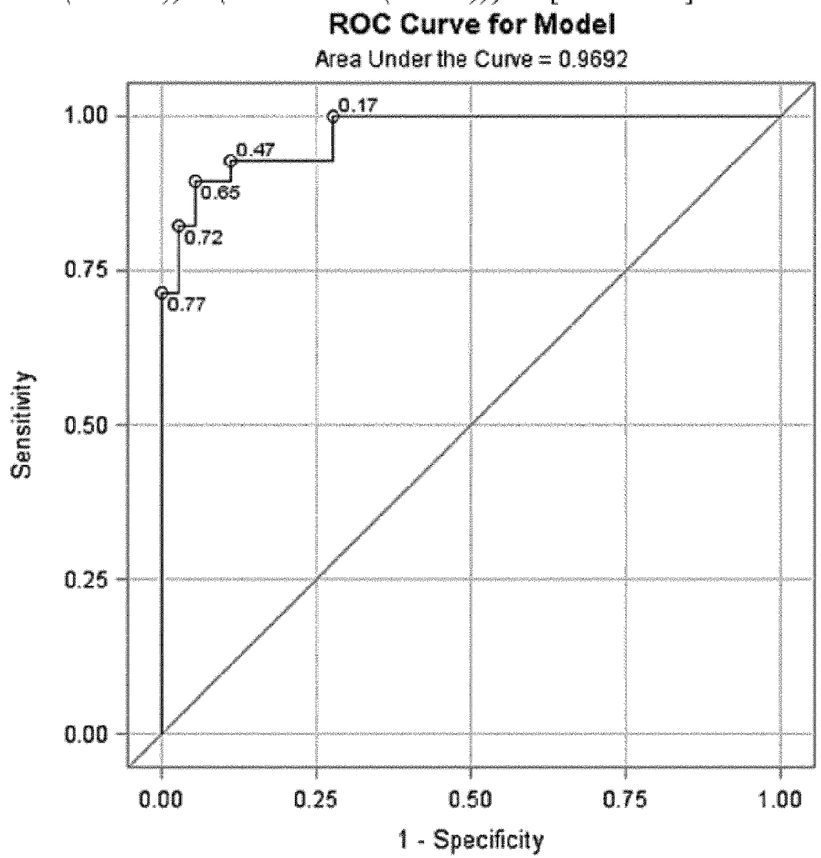


Fig.29

Recurrence score = exp( y)/(1 + exp( y)); in which y

$$y = 25.9125 - 0.6740 \times (hsa\text{-}miR\text{-}518b(Tumor)) + 0.5092 \times (hsa\text{-}miR\text{-}518b(Normal))$$
$$+ 0.3870 \times (hsa\text{-}miR\text{-}629(Tumor)) - 1.2266 \times (hsa\text{-}miR\text{-}629(Normal)) - 0.9633 \times (hsa\text{-}miR\text{-}885\text{-}5p(Normal))$$
$$-0.2863 \times (hsa\text{-}mir\text{-}139\text{-}3p(Tumor)) - 0.1075 \times (hsa\text{-}mir\text{-}139\text{-}3p(Normal))$$
$$-0.2587 \times (hsa\text{-}miR\text{-}223^{*}(Tumor)) + 0.0972 \times (hsa\text{-}miR\text{-}223^{*}(Normal)) \ldots\ldots[formula27]$$

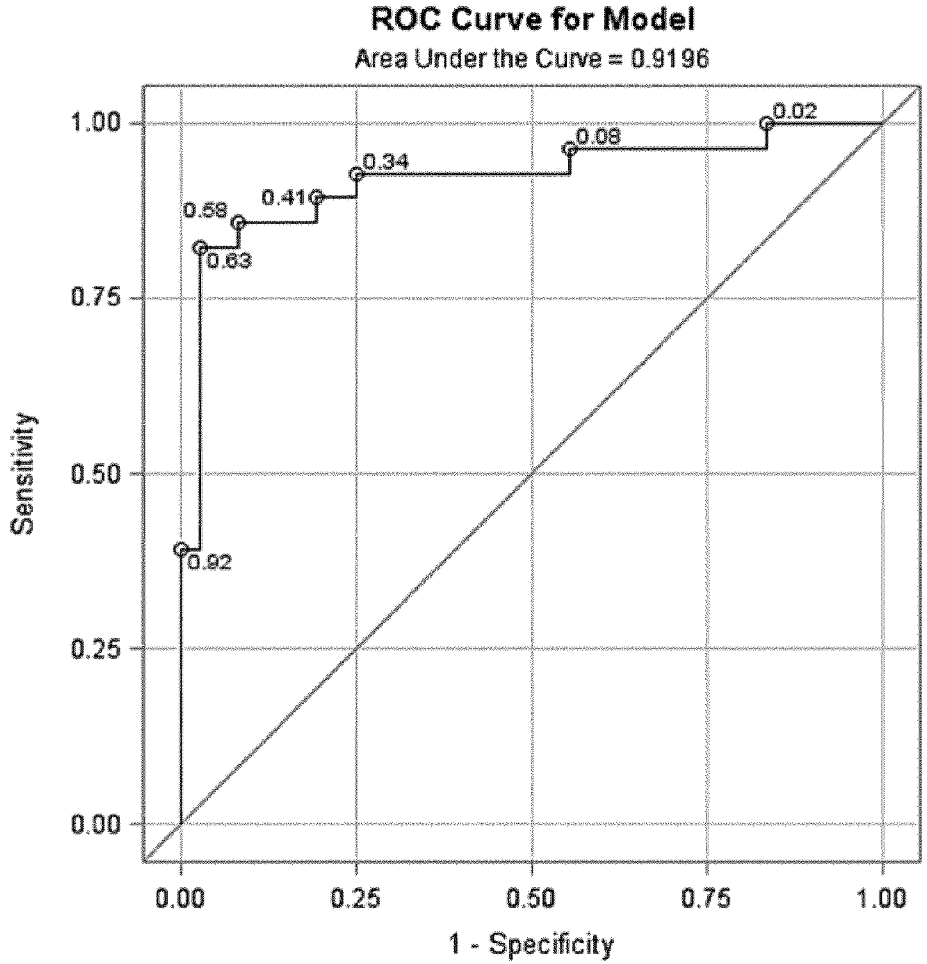


Fig.30

Recurrence score = exp( y)/(1 + exp( y)); in which y

$$y = -69.1934 + 7.7258 \times (hsa\text{-}miR\text{-}518b(Tumor)) + 0.7075 \times (hsa\text{-}miR\text{-}518b(Normal))$$
$$+ 0.3196 \times (hsa\text{-}miR\text{-}629(Tumor)) + 8.0023 \times (hsa\text{-}miR\text{-}629(Normal)) - 0.9692 \times (hsa\text{-}miR\text{-}885\text{-}5p(Normal))$$
$$-0.2522 \times (hsa\text{-}mir\text{-}139\text{-}3p(Tumor)) - 0.1119 \times (hsa\text{-}mir\text{-}139\text{-}3p(Normal)) - 0.2103 \times (hsa\text{-}miR\text{-}223^*(Tumor))$$
$$-0.0126 \times (hsa\text{-}miR\text{-}223^*(Normal)) - 0.8247 \times \{(hsa\text{-}miR\text{-}518b(Tumor)) \times (hsa\text{-}miR\text{-}629(Normal))\}$$ ......[formula28]

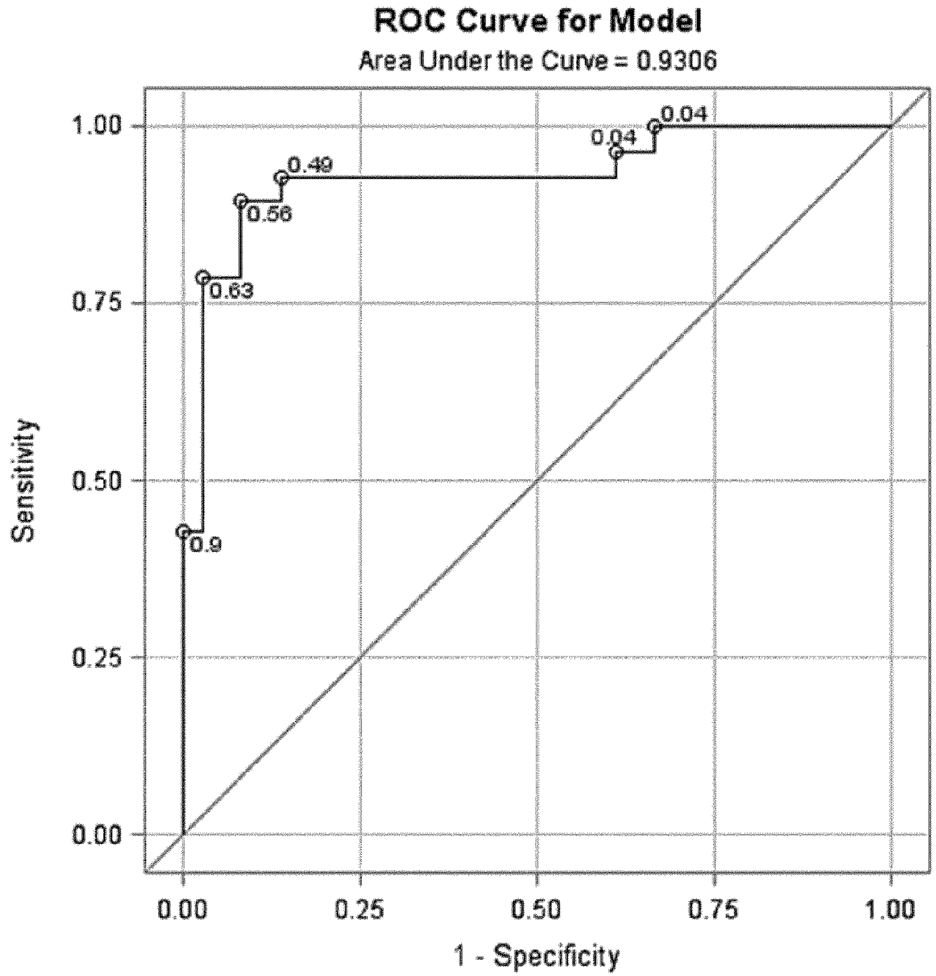


Fig.31

Recurrence score = exp( y)/(1 + exp( y)); in which y

$$y = -13.9497 + 17.4002 \times (hsa\text{-}miR\text{-}518b(Tumor)) + 0.6279 \times (hsa\text{-}miR\text{-}518b(Normal))$$
$$-13.4433 \times (hsa\text{-}miR\text{-}629(Tumor)) + 3.4325 \times (hsa\text{-}miR\text{-}629(Normal)) - 1.7349 \times (hsa\text{-}miR\text{-}885\text{-}5p(Normal))$$
$$-0.3360 \times (hsa\text{-}mir\text{-}139\text{-}3p(Tumor)) - 0.1433 \times (hsa\text{-}mir\text{-}139\text{-}3p(Normal))$$
$$-0.2594 \times (hsa\text{-}miR\text{-}223^{*}(Tumor)) + 0.0966 \times (hsa\text{-}miR\text{-}223^{*}(Normal))$$
$$-1.7485 \times \{(hsa\text{-}miR\text{-}518b(Tumor)) \times (hsa\text{-}miR\text{-}629(Normal))\}$$
$$+ 1.3255 \times \{(hsa\text{-}miR\text{-}629(Tumor)) \times (hsa\text{-}miR\text{-}629(Normal))\} \ldots\ldots [formula29]$$

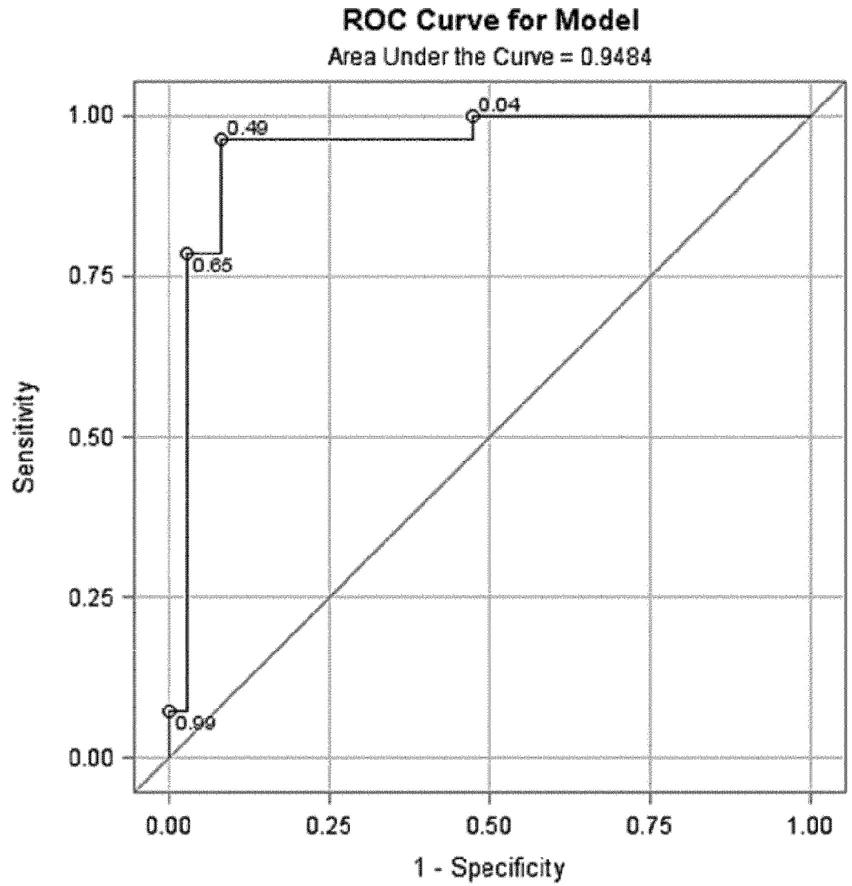


Fig.32

Recurrence score = exp( y)/(1 + exp( y)); in which y

$$y = 162.1 - 0.7534 \times (hsa\text{-}miR\text{-}518b(Tumor)) + 28.6992 \times (hsa\text{-}miR\text{-}518b(Normal))$$
$$-24.7347 \times (hsa\text{-}miR\text{-}629(Tumor)) + 2.4418 \times (hsa\text{-}miR\text{-}629(Normal)) - 19.8193 \times (hsa\text{-}miR\text{-}885\text{-}5p(Normal))$$
$$-0.4848 \times (hsa\text{-}mir\text{-}139\text{-}3p(Tumor)) - 0.3705 \times (hsa\text{-}mir\text{-}139\text{-}3p(Normal)) - 0.7596 \times (hsa\text{-}miR\text{-}223^*(Tumor))$$
$$-14.3213 \times (hsa\text{-}miR\text{-}223^*(Normal)) - 2.6334 \times \{(hsa\text{-}miR\text{-}518b(Tumor)) \times (hsa\text{-}miR\text{-}629(Normal))\}$$
$$+ 2.4241 \times \{(hsa\text{-}miR\text{-}629(Tumor)) \times (hsa\text{-}miR\text{-}629(Normal))\}$$
$$+ 1.5678 \times \{(hsa\text{-}miR\text{-}885\text{-}5p(Normal)) \times (hsa\text{-}mir\text{-}139\text{-}3p(Normal))\} \ldots\ldots[\text{formula30}]$$

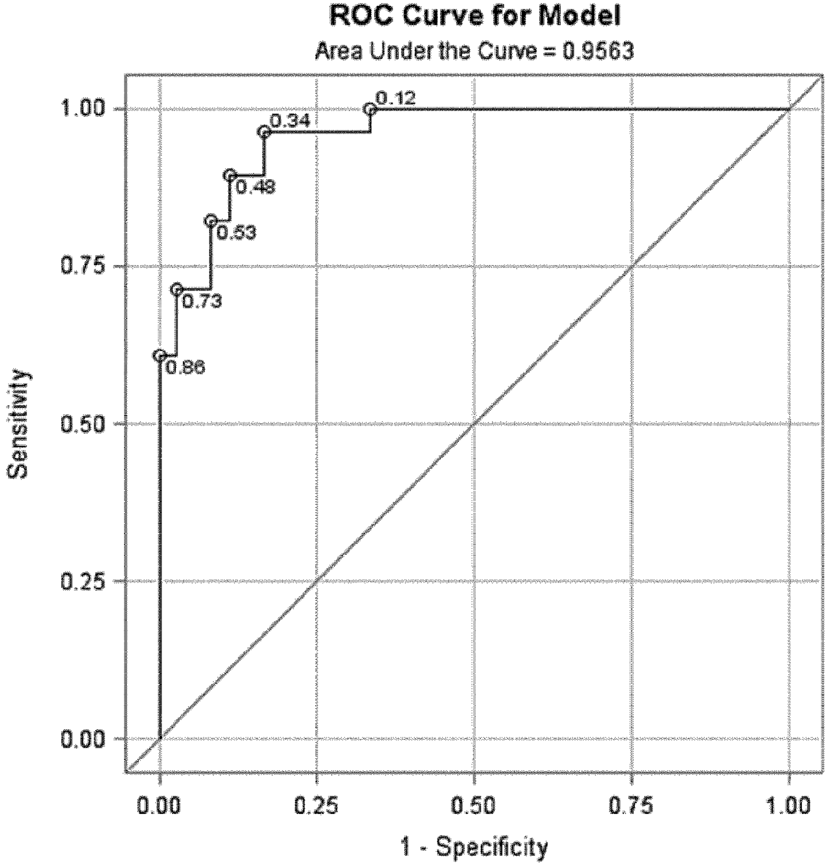


Fig.33

Recurrence score = exp( y)/(1 + exp( y)); in which y

$$y = 298.5 - 0.7477 \times (hsa\text{-}miR\text{-}518b(Tumor)) + 23.3527 \times (hsa\text{-}miR\text{-}518b(Normal))$$
$$-30.3301 \times (hsa\text{-}miR\text{-}629(Tumor)) + 0.3090 \times (hsa\text{-}miR\text{-}629(Normal)) - 19.6142 \times (hsa\text{-}miR\text{-}885\text{-}5p(Normal))$$
$$-0.4214 \times (hsa\text{-}mir\text{-}139\text{-}3p(Tumor)) - 0.8382 \times (hsa\text{-}mir\text{-}139\text{-}3p(Normal)) + 12.1727 \times (hsa\text{-}miR\text{-}223^{*}(Tumor))$$
$$-14.0675 \times (hsa\text{-}miR\text{-}223^{*}(Normal)) - 2.9762 \times \{(hsa\text{-}miR\text{-}518b(Tumor)) \times (hsa\text{-}miR\text{-}629(Normal))\}$$
$$+ 2.9549 \times \{(hsa\text{-}miR\text{-}629(Tumor)) \times (hsa\text{-}miR\text{-}629(Normal))\}$$
$$+ 1.5434 \times \{(hsa\text{-}miR\text{-}885\text{-}5p(Normal)) \times (hsa\text{-}mir\text{-}139\text{-}3p(Normal))\}$$
$$+ 0.9074 \times \{(hsa\text{-}miR\text{-}518b(Normal) \times (hsa\text{-}miR\text{-}223^{*}(Tumor))\} \ldots\ldots [formula31]$$

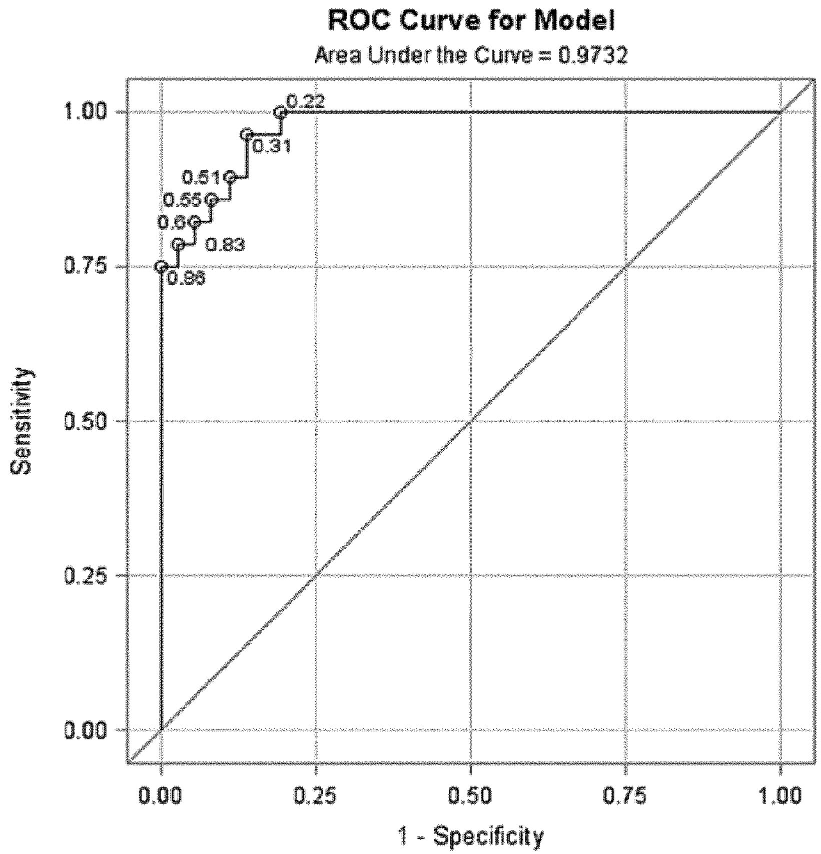


Fig.34

Recurrence score = exp( y)/(1 + exp( y)); in which y

$$y = 24.3035 + 0.5340 \times (hsa\text{-}mir\text{-}1224\text{-}5p(Tumor)) - 0.1910 \times (hsa\text{-}mir\text{-}1224\text{-}5p(Normal))$$
$$-0.7638 \times (hsa\text{-}miR\text{-}518b(Tumor)) + 0.4479 \times (hsa\text{-}miR\text{-}629(Tumor)) - 0.9289 \times (hsa\text{-}miR\text{-}629(Normal))$$
$$-0.6998 \times (hsa\text{-}miR\text{-}885\text{-}5p(Normal)) - 0.3048 \times (hsa\text{-}mir\text{-}139\text{-}3p(Tumor))$$
$$-0.1598 \times (hsa\text{-}mir\text{-}139\text{-}3p(Normal)) - 0.6600 \times (hsa\text{-}miR\text{-}223^{*}(Tumor))$$
$$+0.4940 \times (hsa\text{-}miR\text{-}223^{*}(Normal)) \ldots\ldots [formula32]$$

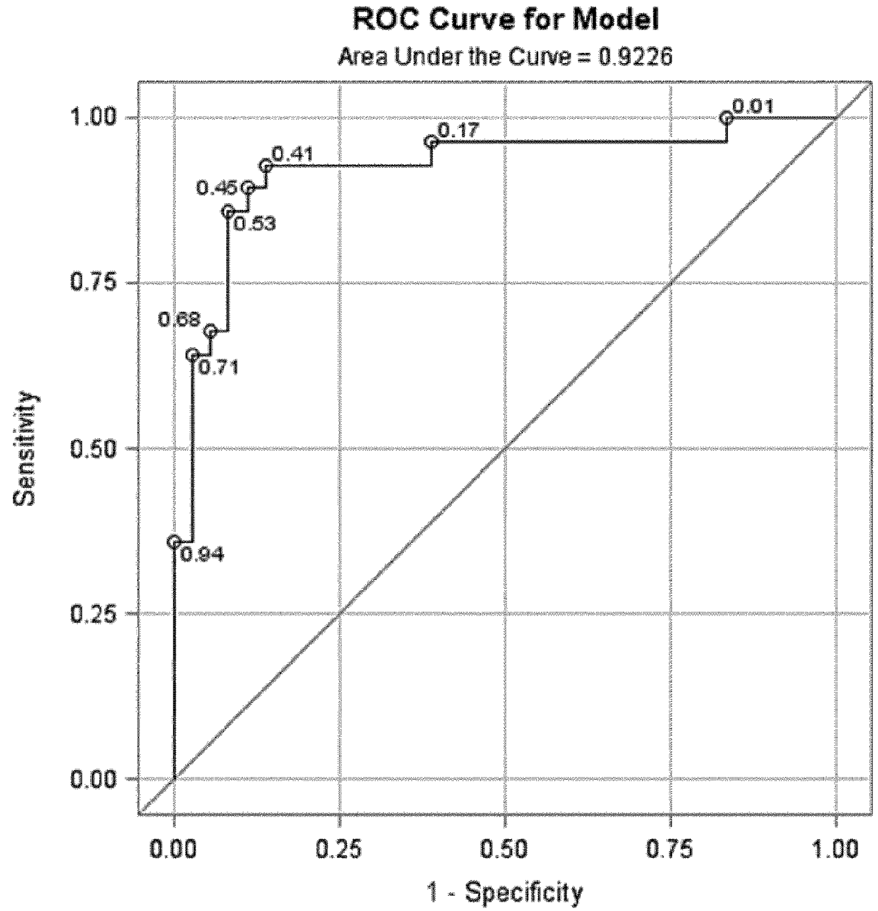


Fig.35

Recurrence score = exp( y)/(1 + exp( y)); in which y

$y = 167.7 - 13.2279 \times (hsa\text{-}mir\text{-}1224\text{-}5p(Tumor)) - 1.1091 \times (hsa\text{-}mir\text{-}1224\text{-}5p(Normal))$
$- 1.4007 \times (hsa\text{-}miR\text{-}518b(Tumor)) + 1.1036 \times (hsa\text{-}miR\text{-}629(Tumor)) - 1.1119 \times (hsa\text{-}miR\text{-}629(Normal))$
$- 15.0002 \times (hsa\text{-}miR\text{-}885\text{-}5p(Normal)) - 0.6073 \times (hsa\text{-}mir\text{-}139\text{-}3p(Tumor))$
$- 0.6369 \times (hsa\text{-}mir\text{-}139\text{-}3p(Normal)) - 1.3449 \times (hsa\text{-}miR\text{-}223^{*}(Tumor)) + 1.2204 \times (hsa\text{-}miR\text{-}223^{*}(Normal))$
$+ 1.5767 \times \{(hsa\text{-}mir\text{-}1224\text{-}5p(Tumor)) \times (hsa\text{-}miR\text{-}885\text{-}5p(Normal))\}$ ......[formula33]

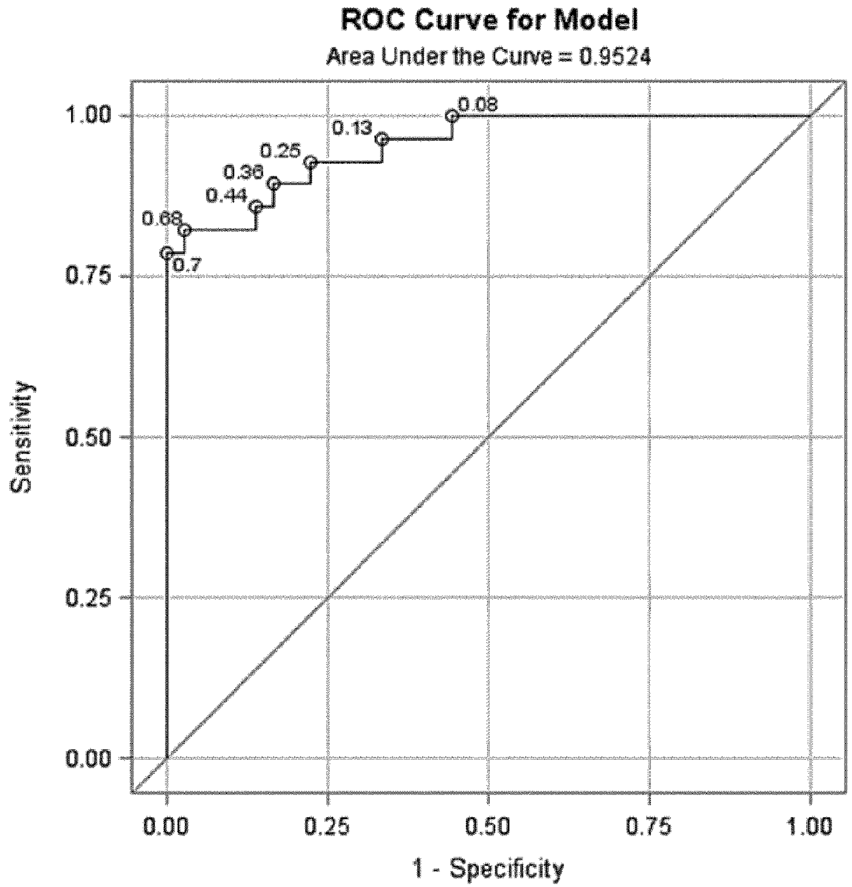


Fig.36

Recurrence score = exp( y)/(1 + exp( y)); in which y

$$y = 359.0 - 20.5097 \times (hsa\text{-}mir\text{-}1224\text{-}5p(Tumor)) - 2.1028 \times (hsa\text{-}mir\text{-}1224\text{-}5p(Normal))$$
$$-3.0483 \times (hsa\text{-}miR\text{-}518b(Tumor)) + 2.5706 \times (hsa\text{-}miR\text{-}629(Tumor)) - 2.4592 \times (hsa\text{-}miR\text{-}629(Normal))$$
$$-23.3841 \times (hsa\text{-}miR\text{-}885\text{-}5p(Normal)) - 6.4496 \times (hsa\text{-}mir\text{-}139\text{-}3p(Tumor))$$
$$-1.5971 \times (hsa\text{-}mir\text{-}139\text{-}3p(Normal)) - 11.6562 \times (hsa\text{-}miR\text{-}223^*(Tumor)) + 2.4651 \times (hsa\text{-}miR\text{-}223^*(Normal))$$
$$+ 2.5240 \times \{(hsa\text{-}mir\text{-}1224\text{-}5p(Tumor)) \times (hsa\text{-}miR\text{-}885\text{-}5p(Normal))\}$$
$$+ 0.5692 \times \{(hsa\text{-}mir\text{-}139\text{-}3p(Tumor)) \times (hsa\text{-}miR\text{-}223^*(Tumor))\} \text{......[formula34]}$$

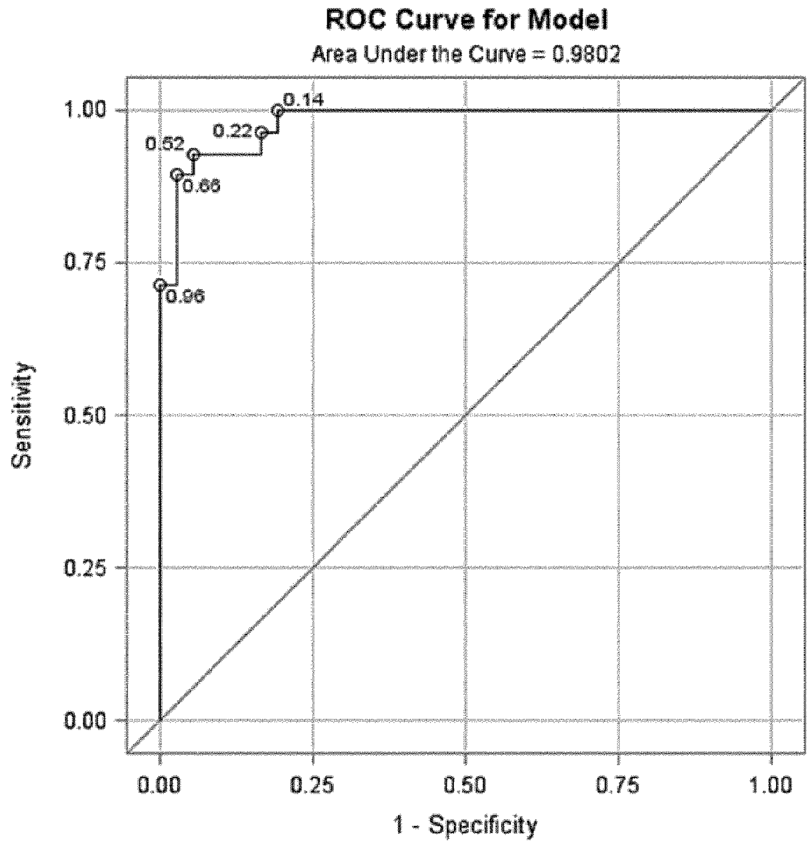


Fig.37

Recurrence score = exp( y)/(1 + exp( y)); in which y

$$y = 415.1 - 29.9501 \times (hsa\text{-}mir\text{-}1224\text{-}5p(Tumor)) + 7.6962 \times (hsa\text{-}mir\text{-}1224\text{-}5p(Normal))$$
$$-4.7711 \times (hsa\text{-}miR\text{-}518b(Tumor)) + 3.3794 \times (hsa\text{-}miR\text{-}629(Tumor)) - 2.3400 \times (hsa\text{-}miR\text{-}629(Normal))$$
$$-33.1847 \times (hsa\text{-}miR\text{-}885\text{-}5p(Normal)) - 8.9096 \times (hsa\text{-}mir\text{-}139\text{-}3p(Tumor))$$
$$-1.6504 \times (hsa\text{-}mir\text{-}139\text{-}3p(Normal)) - 6.1349 \times (hsa\text{-}miR\text{-}223*(Tumor)) + 1.8411 \times (hsa\text{-}miR\text{-}223*(Normal))$$
$$+3.5456 \times \{(hsa\text{-}mir\text{-}1224\text{-}5p(Tumor)) \times (hsa\text{-}miR\text{-}885\text{-}5p(Normal))\}$$
$$+0.7616 \times \{(hsa\text{-}mir\text{-}139\text{-}3p(Tumor)) \times (hsa\text{-}miR\text{-}223*(Tumor))\}$$
$$-0.9109 \times \{(hsa\text{-}mir\text{-}1224\text{-}5p(Normal) \times (hsa\text{-}miR\text{-}223*(Tumor))\} \text{......[formula35]}$$

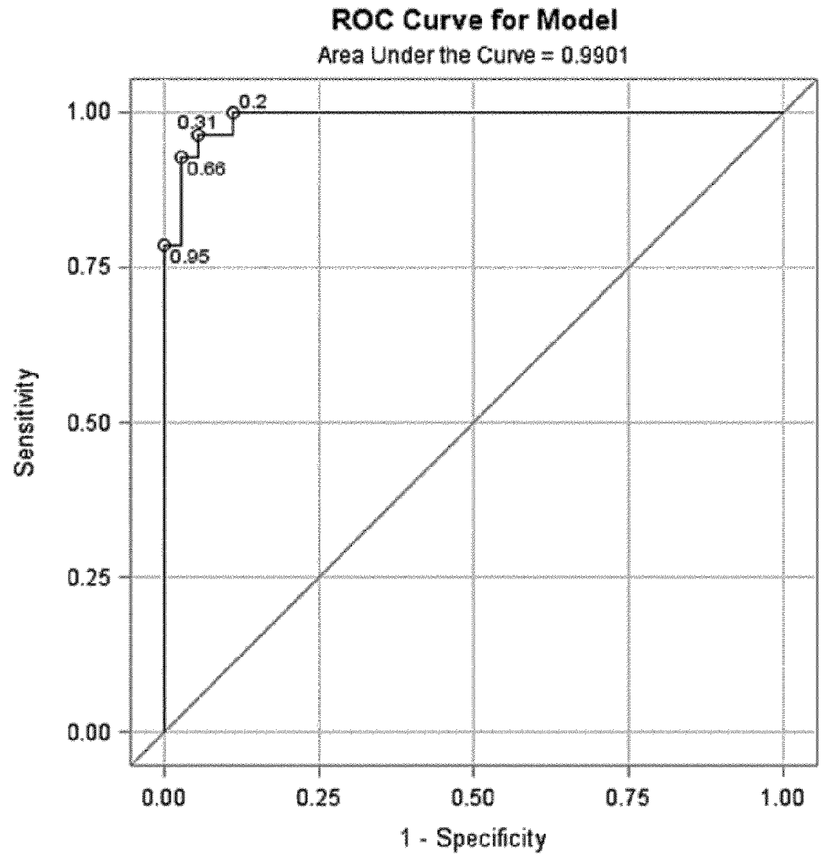


Fig.38

Recurrence score = exp( y)/(1 + exp( y)); in which y

$$y = 843.7 - 32.7889 \times (hsa\text{-}mir\text{-}1224\text{-}5p(Tumor)) - 4.2230 \times (hsa\text{-}mir\text{-}1224\text{-}5p(Normal))$$
$$-4.2232 \times (hsa\text{-}miR\text{-}518b(Tumor)) - 20.6187 \times (hsa\text{-}miR\text{-}629(Tumor)) - 30.0652 \times (hsa\text{-}miR\text{-}629(Normal))$$
$$-38.5813 \times (hsa\text{-}miR\text{-}885\text{-}5p(Normal)) - 8.9655 \times (hsa\text{-}mir\text{-}139\text{-}3p(Tumor)) - 2.8265 \times (hsa\text{-}mir\text{-}139\text{-}3p(Normal))$$
$$-16.9922 \times (hsa\text{-}miR\text{-}223^*(Tumor)) + 3.7414 \times (hsa\text{-}miR\text{-}223^*(Normal))$$
$$+ 4.0480 \times \{(hsa\text{-}mir\text{-}1224\text{-}5p(Tumor)) \times (hsa\text{-}miR\text{-}885\text{-}5p(Normal))\}$$
$$+ 0.8328 \times \{(hsa\text{-}mir\text{-}139\text{-}3p(Tumor)) \times (hsa\text{-}miR\text{-}223^*(Tumor))\}$$
$$+ 2.2931 \times \{(hsa\text{-}miR\text{-}629(Tumor)) \times (hsa\text{-}miR\text{-}629(Normal))\} \ldots\ldots [\text{formula36}]$$

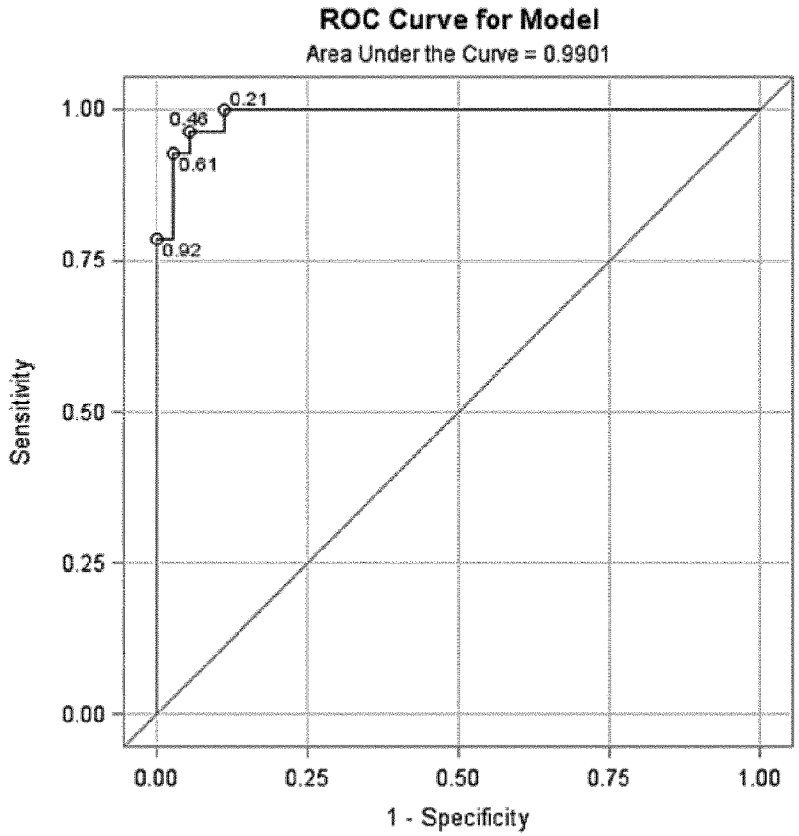


Fig.39

Recurrence score = exp( y)/(1 + exp( y)); in which y

$$y = 315.8 - 26.3641 \times (hsa\text{-}mir\text{-}1224\text{-}5p(Tumor)) - 3.1687 \times (hsa\text{-}mir\text{-}1224\text{-}5p(Normal))$$
$$-3.8282 \times (hsa\text{-}miR\text{-}518b(Tumor)) + 2.9126 \times (hsa\text{-}miR\text{-}629(Tumor)) + 9.4863 \times (hsa\text{-}miR\text{-}629(Normal))$$
$$-30.1097 \times (hsa\text{-}miR\text{-}885\text{-}5p(Normal)) - 6.9425 \times (hsa\text{-}mir\text{-}139\text{-}3p(Tumor)) - 2.0399 \times (hsa\text{-}mir\text{-}139\text{-}3p(Normal))$$
$$+ 0.5164 \times (hsa\text{-}miR\text{-}223^{*}(Tumor)) + 3.1883 \times (hsa\text{-}miR\text{-}223^{*}(Normal))$$
$$+ 3.2598 \times \{(hsa\text{-}mir\text{-}1224\text{-}5p(Tumor)) \times (hsa\text{-}miR\text{-}885\text{-}5p(Normal))\}$$
$$+ 0.6281 \times \{(hsa\text{-}mir\text{-}139\text{-}3p(Tumor)) \times (hsa\text{-}miR\text{-}223^{*}(Tumor))\}$$
$$-1.3465 \times \{(hsa\text{-}miR\text{-}629(Normal)) \times (hsa\text{-}miR\text{-}223^{*}(Tumor))\} \ldots\ldots [\text{formula37}]$$

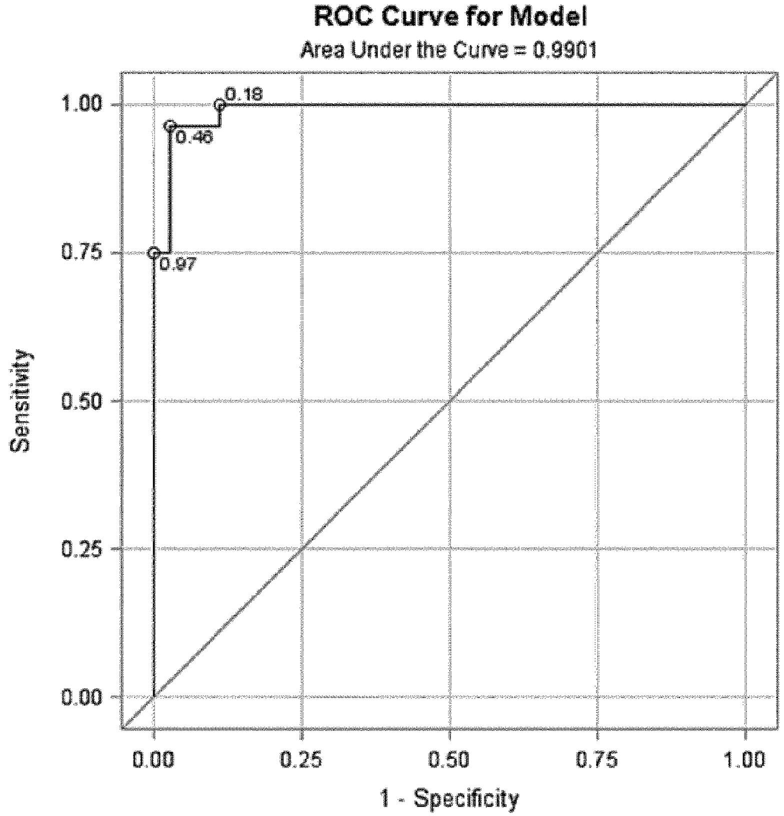


Fig.40

Recurrence score = exp( y)/(1 + exp( y)); in which y

$$y = 24.4235 + 0.4852 \times (hsa\text{-}mir\text{-}1224\text{-}5p(Tumor))\text{-}0.1668 \times (hsa\text{-}mir\text{-}1224\text{-}5p(Normal))$$
$$-0.7664 \times (hsa\text{-}miR\text{-}518b(Tumor)) + 0.4521 \times (hsa\text{-}miR\text{-}629(Tumor))\text{-}0.9759 \times (hsa\text{-}miR\text{-}629(Normal))$$
$$+ 0.1329 \times (hsa\text{-}miR\text{-}885\text{-}5p(Tumor))\text{-}0.7464 \times (hsa\text{-}miR\text{-}885\text{-}5p(Normal))$$
$$-0.3416 \times (hsa\text{-}mir\text{-}139\text{-}3p(Tumor))\text{-}0.1460 \times (hsa\text{-}mir\text{-}139\text{-}3p(Normal))\text{-}0.6731 \times (hsa\text{-}miR\text{-}223^{*}(Tumor))$$
$$+ 0.4968 \times (hsa\text{-}miR\text{-}223^{*}(Normal))......[formula38]$$

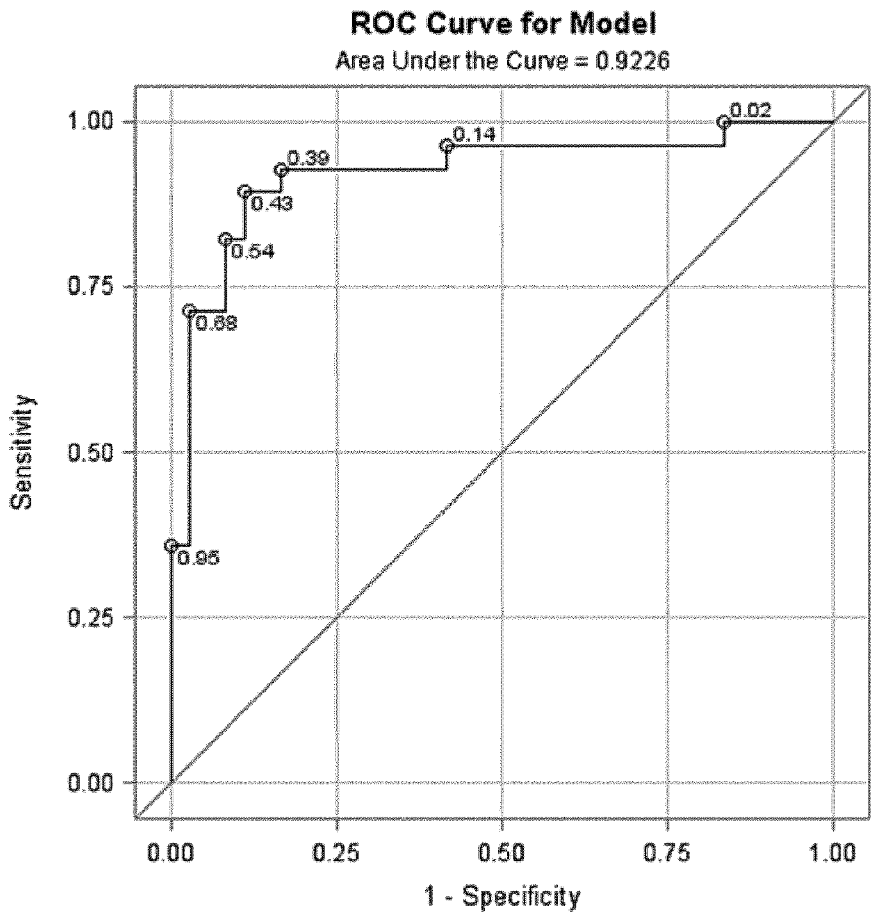


Fig.41

Recurrence score = exp( y)/(1 + exp( y)); in which y

$$y = 186.7 - 14.9237 \times (hsa\text{-}mir\text{-}1224\text{-}5p(Tumor)) - 1.3152 \times (hsa\text{-}mir\text{-}1224\text{-}5p(Normal)) - 1.4097 \times (hsa\text{-}miR\text{-}518b(Tumor)) + 1.1823 \times (hsa\text{-}miR\text{-}629(Tumor)) - 1.0889 \times (hsa\text{-}miR\text{-}629(Normal)) - 0.4604 \times (hsa\text{-}miR\text{-}885\text{-}5p(Tumor)) - 16.6973 \times (hsa\text{-}miR\text{-}885\text{-}5p(Normal)) - 0.5608 \times (hsa\text{-}mir\text{-}139\text{-}3p(Tumor)) - 0.7088 \times (hsa\text{-}mir\text{-}139\text{-}3p(Normal)) - 1.3307 \times (hsa\text{-}miR\text{-}223^{*}(Tumor)) + 1.2948 \times (hsa\text{-}miR\text{-}223^{*}(Normal)) + 1.7823 \times \{(hsa\text{-}mir\text{-}1224\text{-}5p(Tumor)) \times (hsa\text{-}miR\text{-}885\text{-}5p(Normal))\} \ldots\ldots [formula39]$$

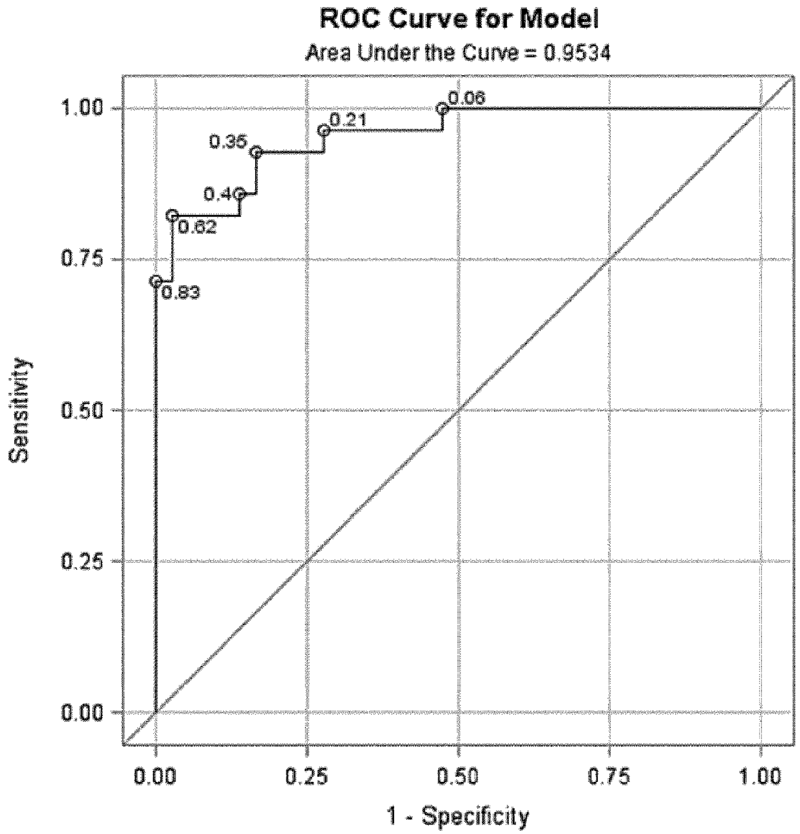


Fig.42

Recurrence score = exp( y)/(1 + exp( y)); in which y

$$y = 381.9 - 24.0811 \times (hsa\text{-}mir\text{-}1224\text{-}5p(Tumor)) - 2.4194 \times (hsa\text{-}mir\text{-}1224\text{-}5p(Normal))$$
$$-10.2279 \times (hsa\text{-}miR\text{-}518b(Tumor)) + 2.4929 \times (hsa\text{-}miR\text{-}629(Tumor))$$
$$-0.5979 \times (hsa\text{-}miR\text{-}629(Normal)) - 0.9393 \times (hsa\text{-}miR\text{-}885\text{-}5p(Tumor)) - 27.9006 \times (hsa\text{-}miR\text{-}885\text{-}5p(Normal))$$
$$-0.7966 \times (hsa\text{-}mir\text{-}139\text{-}3p(Tumor)) - 1.5331 \times (hsa\text{-}mir\text{-}139\text{-}3p(Normal)) - 10.0833 \times (hsa\text{-}miR\text{-}223^{*}(Tumor))$$
$$+ 2.4595 \times (hsa\text{-}miR\text{-}223^{*}(Normal)) + 2.9437 \times \{(hsa\text{-}mir\text{-}1224\text{-}5p(Tumor)) \times (hsa\text{-}miR\text{-}885\text{-}5p(Normal))\}$$
$$+ 0.6778 \times \{(hsa\text{-}miR\text{-}518b(Tumor)) \times (hsa\text{-}mir\text{-}139\text{-}3p(Normal))\} \ldots\ldots [formula40]$$

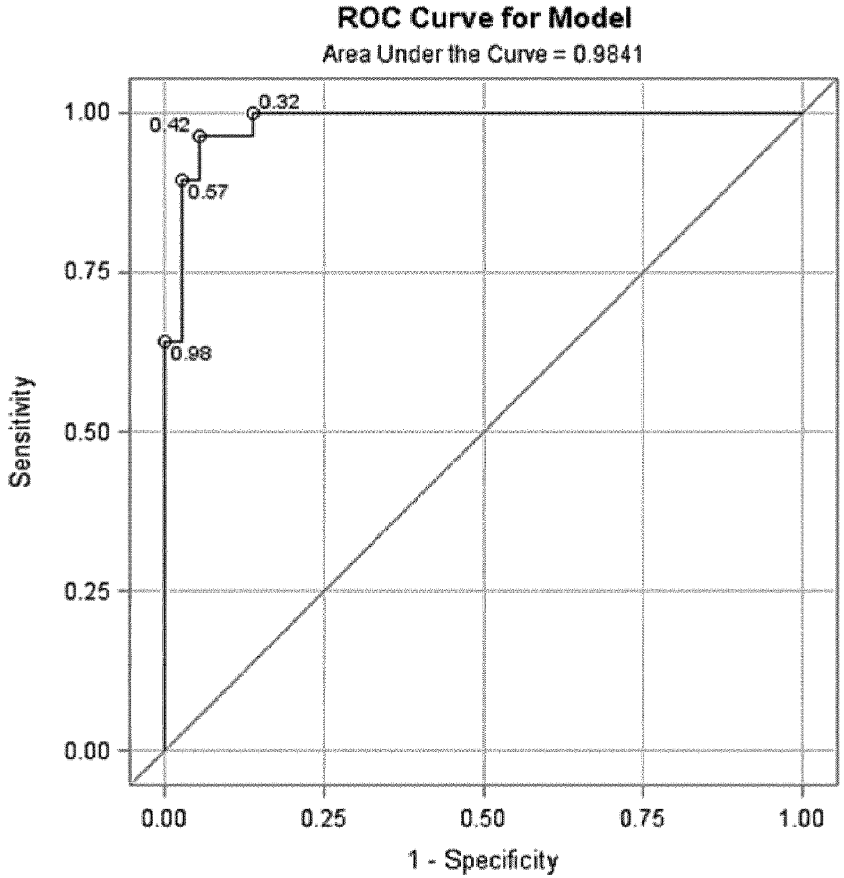


Fig.43

Recurrence score = exp( y)/(1 + exp( y)); in which y

$$y = 480.7 - 46.8405 \times (hsa\text{-}mir\text{-}1224\text{-}5p(Tumor)) - 2.1257 \times (hsa\text{-}mir\text{-}1224\text{-}5p(Normal))$$
$$-9.9400 \times (hsa\text{-}miR\text{-}518b(Tumor)) + 2.5093 \times (hsa\text{-}miR\text{-}629(Tumor)) - 1.0316 \times (hsa\text{-}miR\text{-}629(Normal))$$
$$-2.1232 \times (hsa\text{-}miR\text{-}885\text{-}5p(Tumor)) - 37.5329 \times (hsa\text{-}miR\text{-}885\text{-}5p(Normal)) - 1.0791 \times (hsa\text{-}mir\text{-}139\text{-}3p(Tumor))$$
$$+1.6236 \times (hsa\text{-}mir\text{-}139\text{-}3p(Normal)) - 1.7086 \times (hsa\text{-}miR\text{-}223^*(Tumor)) + 2.2057 \times (hsa\text{-}miR\text{-}223^*(Normal))$$
$$+4.0693 \times \{(hsa\text{-}mir\text{-}1224\text{-}5p(Tumor)) \times (hsa\text{-}miR\text{-}885\text{-}5p(Normal))\}$$
$$-0.2442 \times \{(hsa\text{-}miR\text{-}518b(Tumor)) \times (hsa\text{-}mir\text{-}139\text{-}3p(Normal))\}$$
$$+1.0647 \times \{(hsa\text{-}mir\text{-}1224\text{-}5p(Tumor)) \times (hsa\text{-}miR\text{-}518b(Tumor))\} \ldots\ldots [formula41]$$

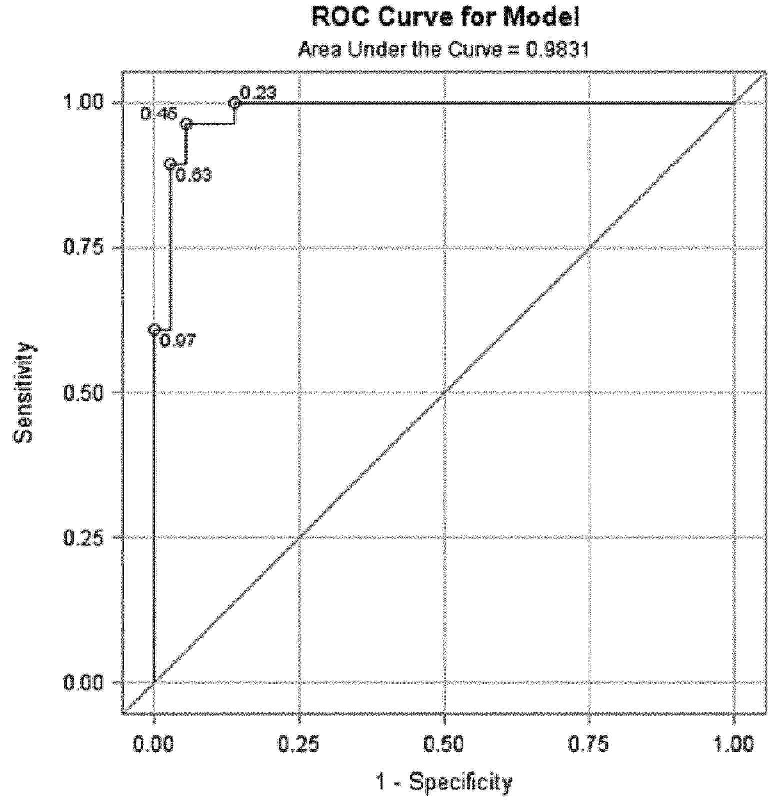


Fig.44

Recurrence score = exp( y)/(1 + exp( y)); in which y

$$y = 468.2 - 37.0352 \times (hsa\text{-}mir\text{-}1224\text{-}5p(Tumor)) - 3.1280 \times (hsa\text{-}mir\text{-}1224\text{-}5p(Normal))$$
$$-6.5985 \times (hsa\text{-}miR\text{-}518b(Tumor)) + 2.4534 \times (hsa\text{-}miR\text{-}629(Tumor)) - 0.3473 \times (hsa\text{-}miR\text{-}629(Normal))$$
$$-1.6419 \times (hsa\text{-}miR\text{-}885\text{-}5p(Tumor)) - 40.7892 \times (hsa\text{-}miR\text{-}885\text{-}5p(Normal)) - 12.2513 \times (hsa\text{-}mir\text{-}139\text{-}3p(Tumor))$$
$$+10.7013 \times (hsa\text{-}mir\text{-}139\text{-}3p(Normal)) - 2.0281 \times (hsa\text{-}miR\text{-}223{*}(Tumor)) + 2.7296 \times (hsa\text{-}miR\text{-}223{*}(Normal))$$
$$+4.3313 \times \{(hsa\text{-}mir\text{-}1224\text{-}5p(Tumor)) \times (hsa\text{-}miR\text{-}885\text{-}5p(Normal))\}$$
$$-1.0946 \times \{(hsa\text{-}miR\text{-}518b(Tumor)) \times (hsa\text{-}mir\text{-}139\text{-}3p(Normal))\}$$
$$+1.0057 \times \{(hsa\text{-}miR\text{-}518b(Tumor)) \times (hsa\text{-}mir\text{-}139\text{-}3p(Tumor))\} \text{......[formula42]}$$

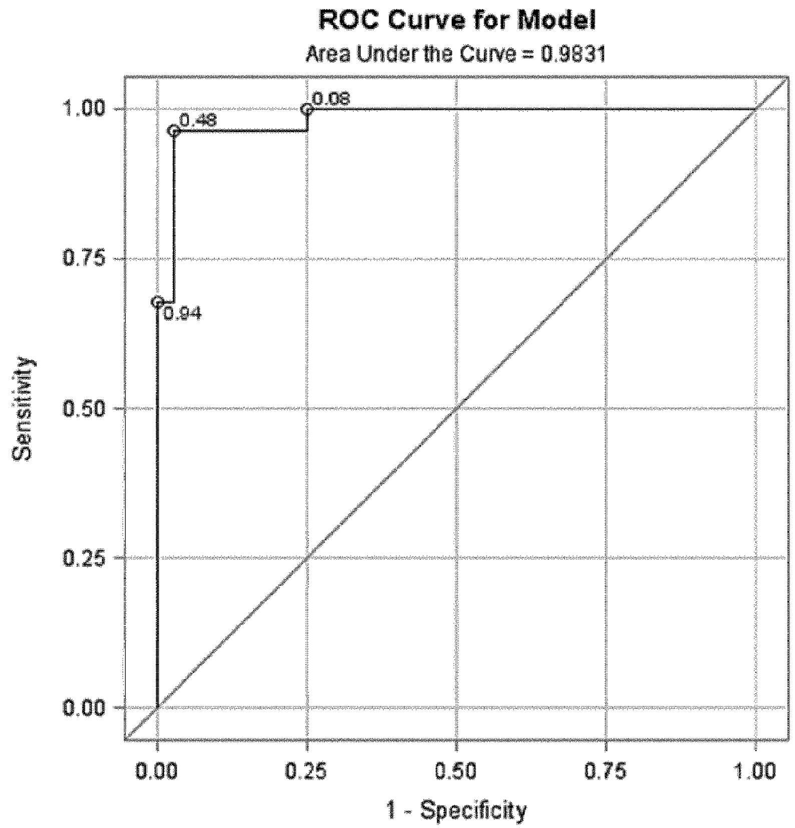


Fig.45

Recurrence score = exp( y)/(1 + exp( y)); in which y

$$y = 376.0 - 26.3925 \times (hsa\text{-}mir\text{-}1224\text{-}5p(Tumor)) - 2.7514 \times (hsa\text{-}mir\text{-}1224\text{-}5p(Normal))$$
$$-7.7972 \times (hsa\text{-}miR\text{-}518b(Tumor)) + 2.5951 \times (hsa\text{-}miR\text{-}629(Tumor))$$
$$-0.3460 \times (hsa\text{-}miR\text{-}629(Normal)) - 1.0957 \times (hsa\text{-}miR\text{-}885\text{-}5p(Tumor)) - 30.3785 \times (hsa\text{-}miR\text{-}885\text{-}5p(Normal))$$
$$-0.8151 \times (hsa\text{-}mir\text{-}139\text{-}3p(Tumor)) + 1.6837 \times (hsa\text{-}mir\text{-}139\text{-}3p(Normal)) - 10.3771 \times (hsa\text{-}miR\text{-}223^{*}(Tumor))$$
$$+ 2.5761 \times (hsa\text{-}miR\text{-}223^{*}(Normal)) + 3.2011 \times \{(hsa\text{-}mir\text{-}1224\text{-}5p(Tumor)) \times (hsa\text{-}miR\text{-}885\text{-}5p(Normal))\}$$
$$-0.2899 \times \{(hsa\text{-}miR\text{-}518b(Tumor)) \times (hsa\text{-}mir\text{-}139\text{-}3p(Normal))\}$$
$$+ 0.7126 \times \{(hsa\text{-}miR\text{-}518b(Tumor)) \times (hsa\text{-}mir\text{-}139\text{-}3p(Tumor))\} \ldots\ldots [formula43]$$

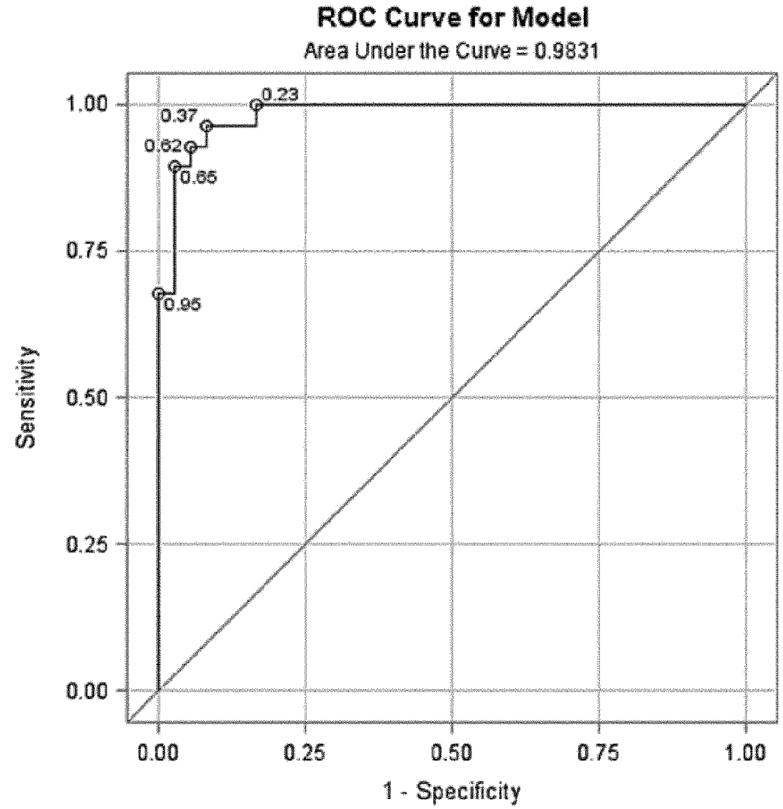


Fig.46

Recurrence score = exp( y)/(1 + exp( y)); in which y

$$y = 24.8947 + 0.5365 \times (hsa\text{-}mir\text{-}1224\text{-}5p(Tumor)) - 0.3340 \times (hsa\text{-}mir\text{-}1224\text{-}5p(Normal))$$
$$-1.0240 \times (hsa\text{-}miR\text{-}518b(Tumor)) + 0.5065 \times (hsa\text{-}miR\text{-}518b(Normal)) + 0.5491 \times (hsa\text{-}miR\text{-}629(Tumor))$$
$$-1.2018 \times (hsa\text{-}miR\text{-}629(Normal)) + 0.1196 \times (hsa\text{-}miR\text{-}885\text{-}5p(Tumor)) - 0.8256 \times (hsa\text{-}miR\text{-}885\text{-}5p(Normal))$$
$$-0.3039 \times (hsa\text{-}mir\text{-}139\text{-}3p(Tumor)) - 0.2134 \times (hsa\text{-}mir\text{-}139\text{-}3p(Normal))$$
$$-0.5905 \times (hsa\text{-}miR\text{-}223^{*}(Tumor)) + 0.3824 \times (hsa\text{-}miR\text{-}223^{*}(Normal)) \ldots\ldots [formula44]$$

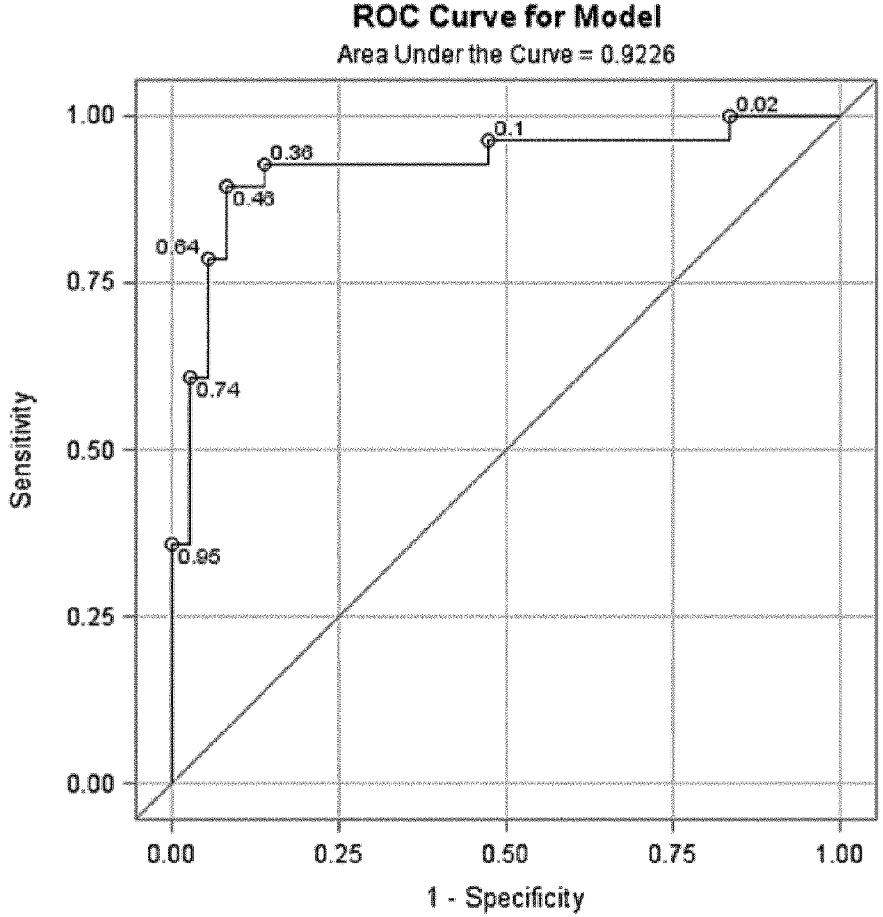


Fig.47

Recurrence score = exp( y)/(1 + exp( y)); in which y

$$y = 190.5 - 15.2253 \times (hsa\text{-}mir\text{-}1224\text{-}5p(Tumor)) - 1.6745 \times (hsa\text{-}mir\text{-}1224\text{-}5p(Normal))$$
$$-1.7881 \times (hsa\text{-}miR\text{-}518b(Tumor)) + 0.7531 \times (hsa\text{-}miR\text{-}518b(Normal))$$
$$+1.3768 \times (hsa\text{-}miR\text{-}629(Tumor)) - 1.5803 \times (hsa\text{-}miR\text{-}629(Normal)) - 0.5721 \times (hsa\text{-}miR\text{-}885\text{-}5p(Tumor))$$
$$-16.9701 \times (hsa\text{-}miR\text{-}885\text{-}5p(Normal)) - 0.4978 \times (hsa\text{-}mir\text{-}139\text{-}3p(Tumor)) - 0.7833 \times (hsa\text{-}mir\text{-}139\text{-}3p(Normal))$$
$$-1.2109 \times (hsa\text{-}miR\text{-}223^*(Tumor)) + 1.1800 \times (hsa\text{-}miR\text{-}223^*(Normal))$$
$$+1.8281 \times \{(hsa\text{-}mir\text{-}1224\text{-}5p(Tumor)) \times (hsa\text{-}miR\text{-}885\text{-}5p(Normal))\} \ldots\ldots [\text{formula45}]$$

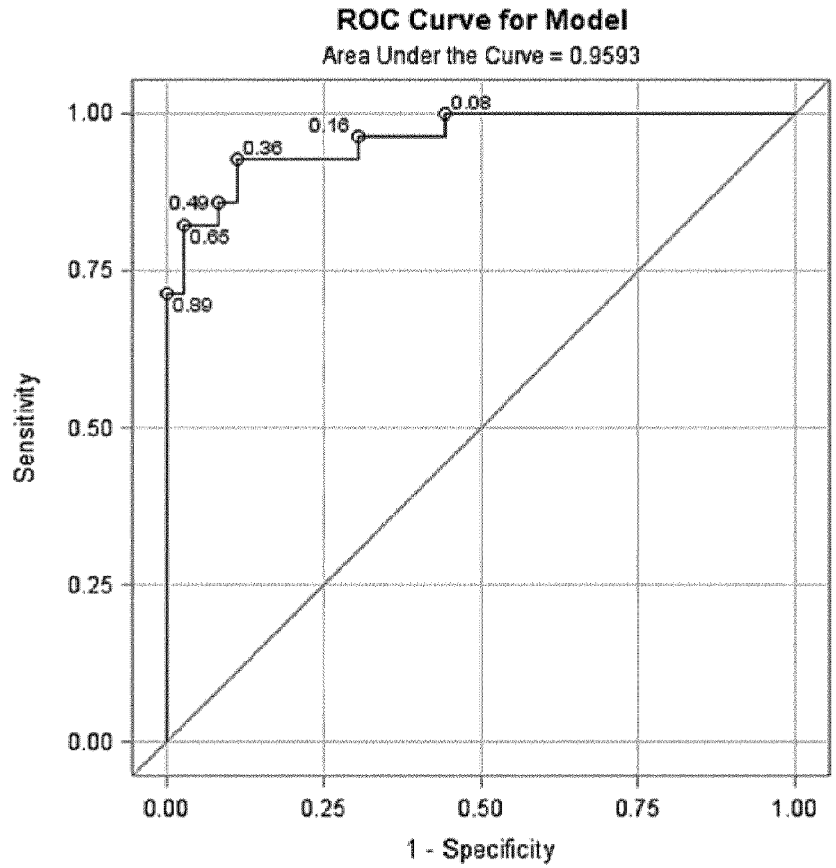


Fig.48

Recurrence score = exp( y)/(1 + exp( y)); in which y

$$y = 686.7 - 39.5482 \times (hsa\text{-}mir\text{-}1224\text{-}5p(Tumor)) - 3.4770 \times (hsa\text{-}mir\text{-}1224\text{-}5p(Normal))$$
$$-3.9820 \times (hsa\text{-}miR\text{-}518b(Tumor)) - 1.8738 \times (hsa\text{-}miR\text{-}518b(Normal))$$
$$+4.3659 \times (hsa\text{-}miR\text{-}629(Tumor)) - 3.2831 \times (hsa\text{-}miR\text{-}629(Normal)) \times -1.9722 \times (hsa\text{-}miR\text{-}885\text{-}5p(Tumor))$$
$$-43.7972 \times (hsa\text{-}miR\text{-}885\text{-}5p(Normal)) - 12.8521 \times (hsa\text{-}mir\text{-}139\text{-}3p(Tumor)) - 2.4551 \times (hsa\text{-}mir\text{-}139\text{-}3p(Normal))$$
$$-21.4547 \times (hsa\text{-}miR\text{-}223^*(Tumor)) + 4.2892 \times (hsa\text{-}miR\text{-}223^*(Normal))$$
$$+4.8087 \times \{(hsa\text{-}mir\text{-}1224\text{-}5p(Tumor)) \times (hsa\text{-}miR\text{-}885\text{-}5p(Normal))\}$$
$$+1.1145 \times \{(hsa\text{-}mir\text{-}139\text{-}3p(Tumor)) \times (hsa\text{-}miR\text{-}223^*(Tumor))\} \ldots\ldots [formula46]$$

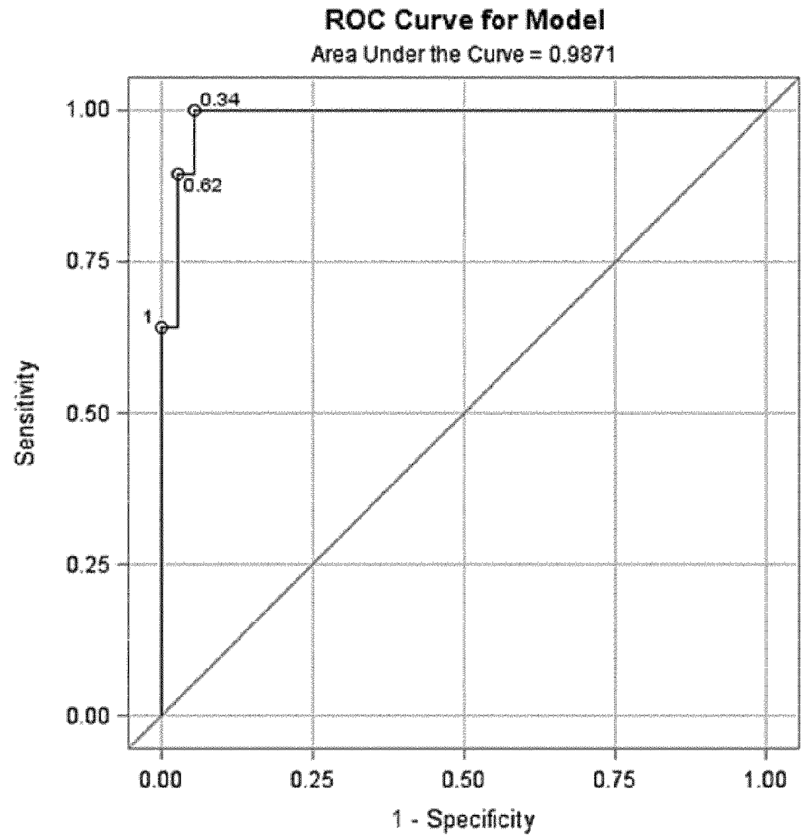


Fig.49

Recurrence score = exp( y)/(1 + exp( y)); in which y

$$y = 2.7536 - 0.1802 \times (hsa\text{-}mir_1224\text{-}5p(Tumor)) + 0.5144 \times (hsa\text{-}mir_1224\text{-}5p(Normal)) + 3.9232 \times (hsa\text{-}miR\text{-}518b(Tumor)) + 0.0165 \times (hsa\text{-}miR\text{-}629(Tumor)) - 1.0602 \times (hsa\text{-}miR\text{-}629(Normal)) + 0.2619 \times (hsa\text{-}miR\text{-}885\text{-}5p(Normal)) - 2.5254 \times (hsa\text{-}miR\text{-}223^*(Tumor)) - 0.0918 \times (hsa\text{-}miR\text{-}223^*(Normal)) - 1.2438 \times (hsa\text{-}mir\text{-}139\text{-}3p(Tumor)) - 2.1279 \times (hsa\text{-}mir\text{-}139\text{-}3p(Normal)) + 1.7237 \times (hsa\text{-}miR\text{-}1290(Normal)) \ldots\ldots formula 47$$

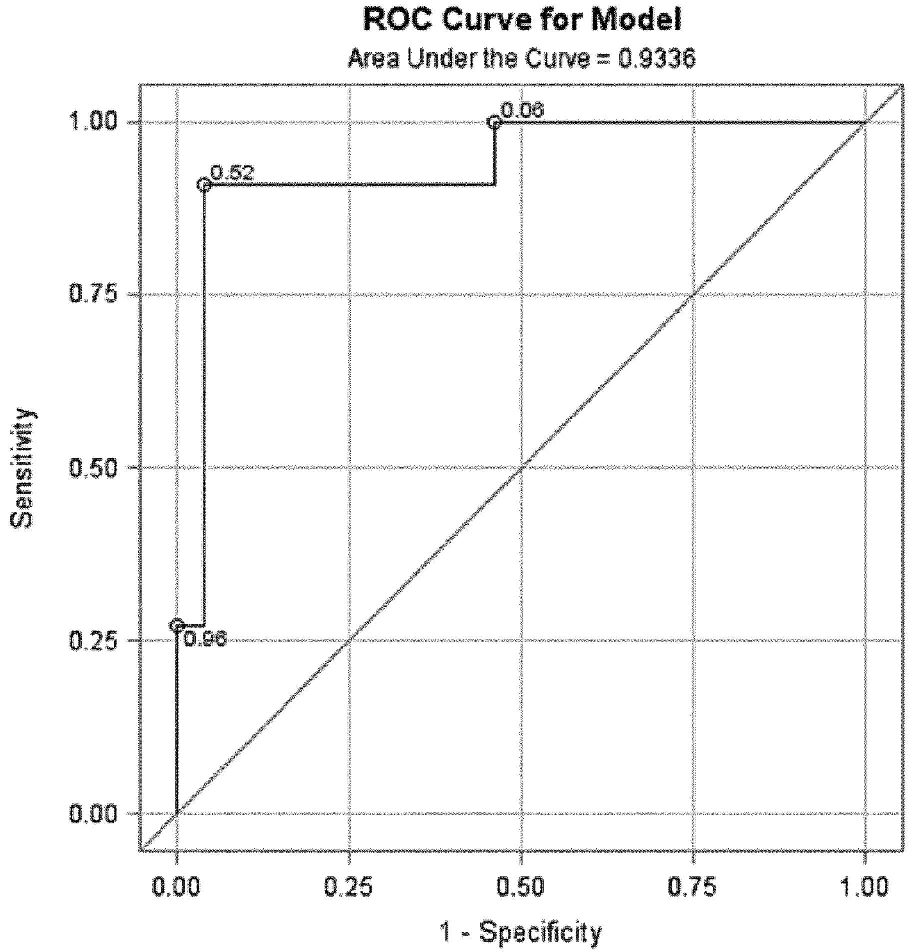


Fig.50

Recurrence score = exp( y)/(1 + exp( y)); in which y

$$y = 6.6549 - 0.386 \times (hsa\text{-}mir_1224\text{-}5p(Tumor)) - 0.076 \times (hsa\text{-}mir_1224\text{-}5p(Normal)) + 4.7078 \times (hsa\text{-}miR\text{-}518b(Tumor)) + 1.039 \times (hsa\text{-}miR\text{-}629(Tumor)) - 0.6502 \times (hsa\text{-}miR\text{-}629(Normal)) - 1.2053 \times (hsa\text{-}miR\text{-}885\text{-}5p(Normal)) - 2.7223 \times (hsa\text{-}miR\text{-}223^*(Tumor)) + 1.4863 \times (hsa\text{-}miR\text{-}223^*(Normal)) - 0.6587 \times (hsa\text{-}mir\text{-}139\text{-}3p(Tumor)) - 0.6084 \times (hsa\text{-}mir\text{-}139\text{-}3p(Normal)) - 2.5607 \times (hsa\text{-}miR\text{-}655(Tumor)) \quad --- formula48$$

**ROC Curve for Model**

Area Under the Curve = 0.9371

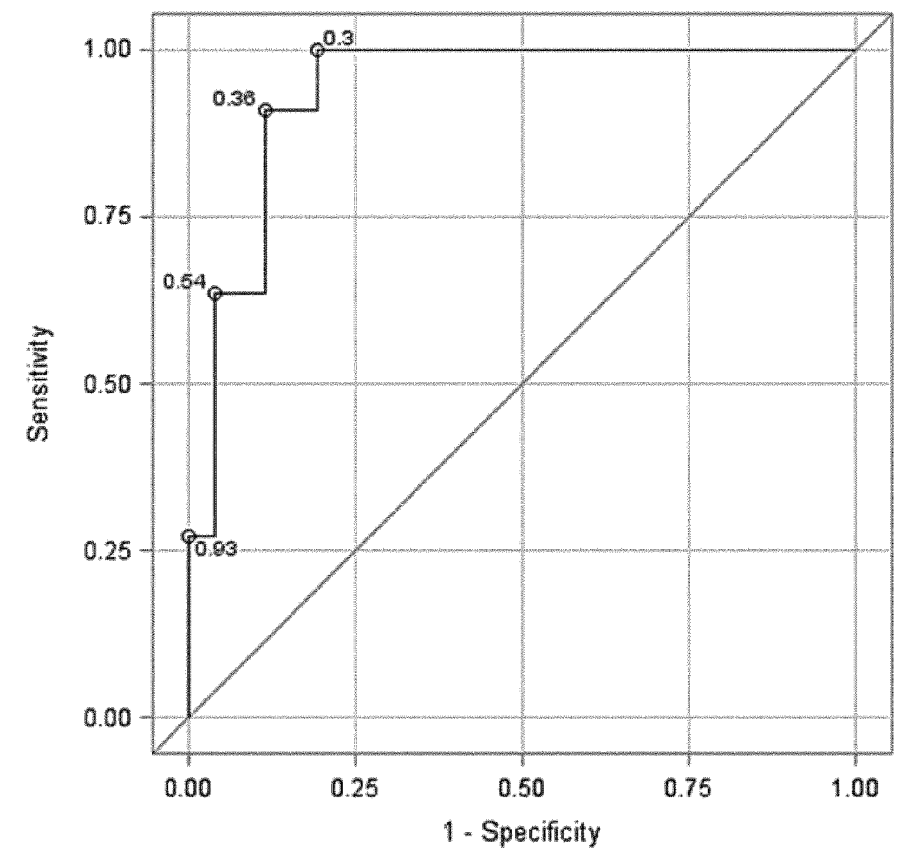


Fig.51

Recurrence score = exp( y)/(1 + exp( y)); in which y

$$y = 6.6705 - 0.3024 \times (hsa\text{-}mir_1224\text{-}5p(Tumor)) + 0.3688 \times (hsa\text{-}mir_1224\text{-}5p(Normal)) + 4.8481 \times (hsa\text{-}miR\text{-}518b(Tumor)) + 0.7677 \times (hsa\text{-}miR\text{-}629(Tumor)) - 0.9314 \times (hsa\text{-}miR\text{-}629(Normal)) - 0.2590 \times (hsa\text{-}miR\text{-}885\text{-}5p(Normal)) - 2.8268 \times (hsa\text{-}miR\text{-}223^*(Tumor)) + 0.5100 \times (hsa\text{-}miR\text{-}223^*(Normal)) - 1.1924 \times (hsa\text{-}mir\text{-}139\text{-}3p(Tumor)) - 1.8470 \times (hsa\text{-}mir\text{-}139\text{-}3p(Normal)) - 1.7363 \times (hsa\text{-}miR\text{-}655(Tumor)) + 1.1398 \times (hsa\text{-}miR\text{-}1290(Normal)) \ldots\ldots formula49$$

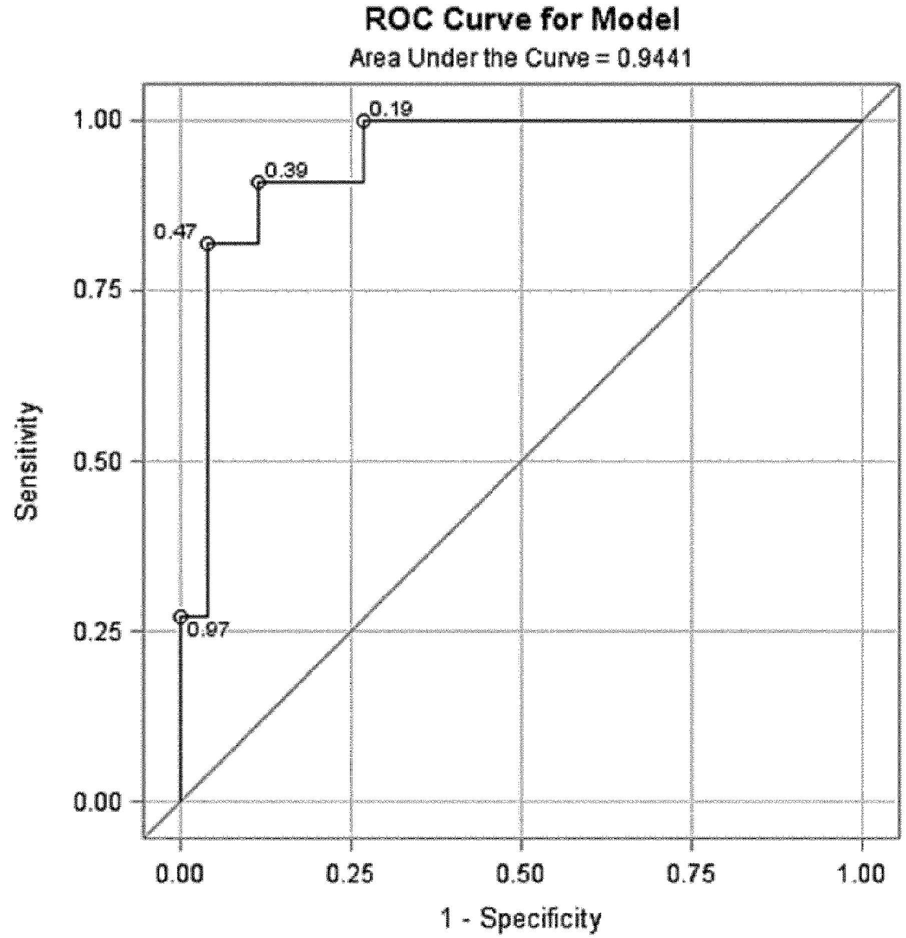


Fig.52

Recurrence score = exp( y)/(1 + exp( y)); in which y

$$y = 10.1195 - 0.3503 \times (hsa\text{-}mir_1224\text{-}5p(Tumor)) + 0.9442 \times (hsa\text{-}mir_1224\text{-}5p(Normal)) + 8.7184 \times (hsa\text{-}miR\text{-}518b(Tumor)) + 2.2476 \times (hsa\text{-}miR\text{-}629(Tumor)) - 1.4460 \times (hsa\text{-}miR\text{-}629(Normal)) - 0.2093 \times (hsa\text{-}miR\text{-}885\text{-}5p(Normal)) - 4.9632 \times (hsa\text{-}miR\text{-}223^*(Tumor)) + 0.5182 \times (hsa\text{-}miR\text{-}223^*(Normal)) - 2.5481 \times (hsa\text{-}mir\text{-}139\text{-}3p(Tumor)) - 2.6980 \times (hsa\text{-}mir\text{-}139\text{-}3p(Normal)) - 1.8716 \times (hsa\text{-}miR\text{-}655(Tumor)) + 1.9734 \times (hsa\text{-}miR\text{-}1290(Normal)) - 2.4953 \times (hsa\text{-}miR\text{-}450b\text{-}5p(Tumor)) \ldots\ldots fomula50$$

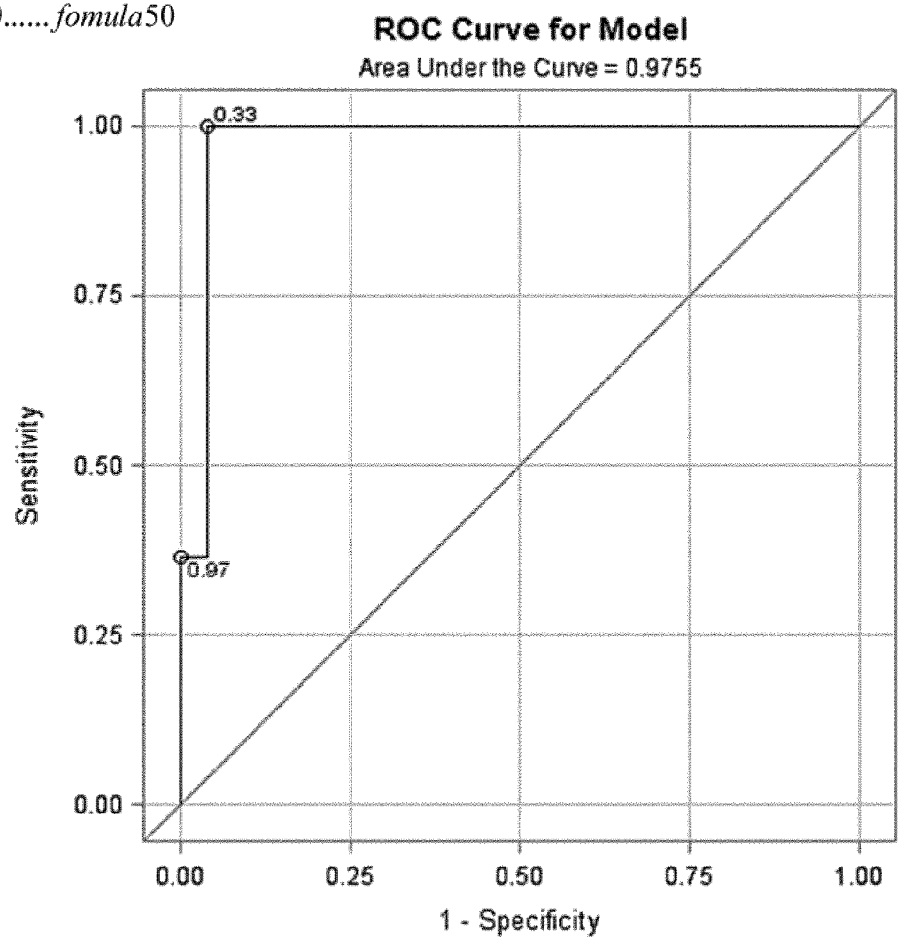


Fig.53

Recurrence score = exp( y)/(1 + exp( y)); in which y

$$y = -36.0574 - 0.1379 \times (hsa\text{-}mir_1224\text{-}5p(Tumor)) + 0.9103 \times (hsa\text{-}mir_1224\text{-}5p(Normal)) + 9.3755 \times (hsa\text{-}miR\text{-}518b(Tumor))$$
$$+ 1.8323 \times (hsa\text{-}miR\text{-}629(Tumor)) - 5.6923 \times (hsa\text{-}miR\text{-}629(Normal)) - 2.6627 \times (hsa\text{-}miR\text{-}885\text{-}5p(Normal))$$
$$-5.7424 \times (hsa\text{-}miR\text{-}223^*(Tumor)) + 0.3687 \times (hsa\text{-}miR\text{-}223^*(Normal)) - 0.0543 \times (hsa\text{-}mir\text{-}139\text{-}3p(Tumor))$$
$$+ 1.4675 \times (hsa\text{-}mir\text{-}139\text{-}3p(Normal)) - 5.4301 \times (hsa\text{-}miR\text{-}655(Tumor)) + 0.0991 \times (hsa\text{-}miR\text{-}1290(Normal))$$
$$+ 6.8188 \times (hsa\text{-}miR\text{-}450b\text{-}5p(Normal)) \ldots\ldots formula51$$

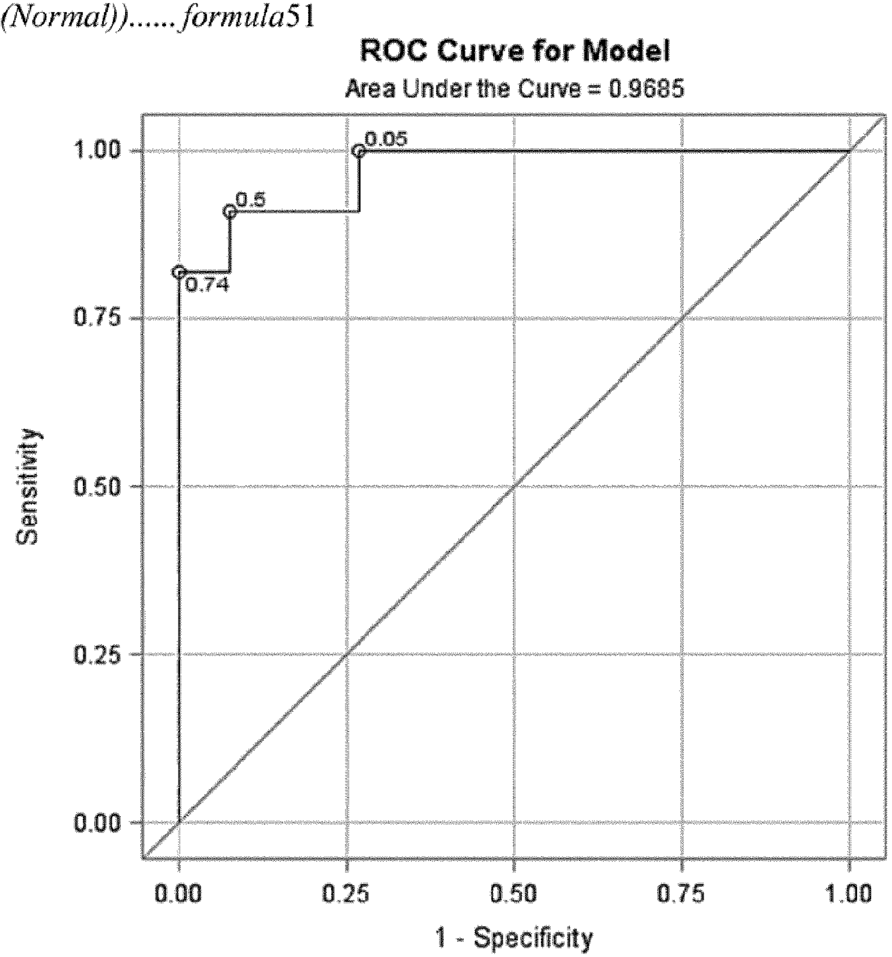


Fig.54

Recurrence score = exp( y)/(1 + exp( y)); in which y

$$y = 9.2339 + 24.1839 \times (hsa\text{-}miR\text{-}363^*) + 1.8440 \times (hsa\text{-}miR\text{-}1255b) + 89.2277 \times (hsa\text{-}miR\text{-}566) + 63.7366 \times (hsa\text{-}miR\text{-}1265) - 26.6886 \times (hsa\text{-}mir\text{-}127\text{-}5p) - 37.9903 \times (hsa\text{-}mir\text{-}338\text{-}5p) + 16.1643 \times (hsa\text{-}mir\text{-}550a^*) + 26.7510 \times (hsa\text{-}mir\text{-}588) - 13.2247 \times (hsa\text{-}mir\text{-}651) - 6.9598 \times (hsa\text{-}mir\text{-}223^*) - 52.5500 \times (hsa\text{-}mir\text{-}518b) - 1.6841 \times (hsa\text{-}mir\text{-}629) + 10.7469 \times (hsa\text{-}mir\text{-}885\text{-}5p) - 26.0437 \times (hsa\text{-}mir\text{-}139\text{-}3p) + 5.4643 \times (hsa\text{-}mir\text{-}1224\text{-}5p)$$

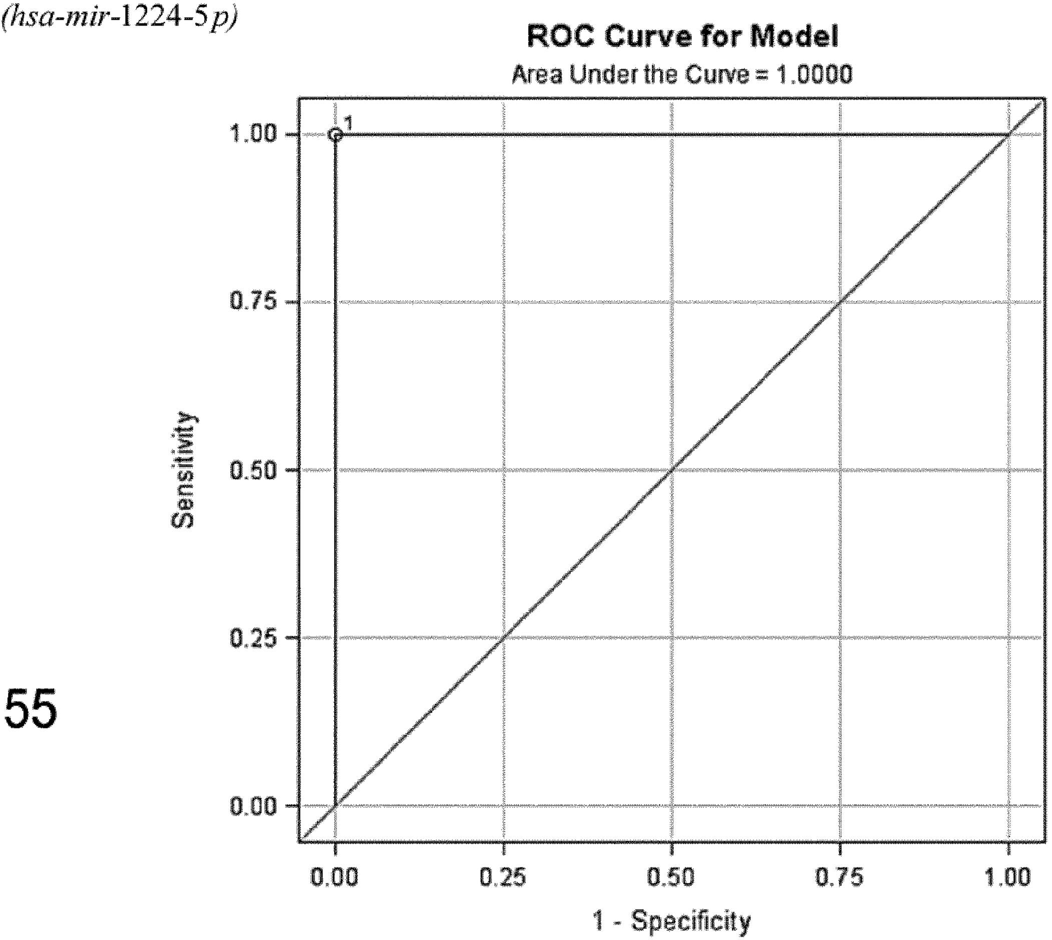


Fig.55

Recurrence score = exp( y)/(1 + exp( y)); in which y

$$y = -12.0054 - 83.8587 \times (hsa\text{-}miR\text{-}363^*) - 8.4239 \times (hsa\text{-}miR\text{-}1255b) + 104.5000 \times (hsa\text{-}miR\text{-}566) + 59.3283 \times (hsa\text{-}miR\text{-}1265) - 8.4693 \times (hsa\text{-}mir\text{-}127\text{-}5p) - 13.1045 \times (hsa\text{-}mir\text{-}338\text{-}5p) - 6.5275 \times (hsa\text{-}mir\text{-}550a^*) + 19.0586 \times (hsa\text{-}mir\text{-}588) + 2.1281 \times (hsa\text{-}mir\text{-}655) + 7.5022 \times (hsa\text{-}mir\text{-}1290) - 8.7410 \times (hsa\text{-}mir\text{-}450\text{-}5p)$$

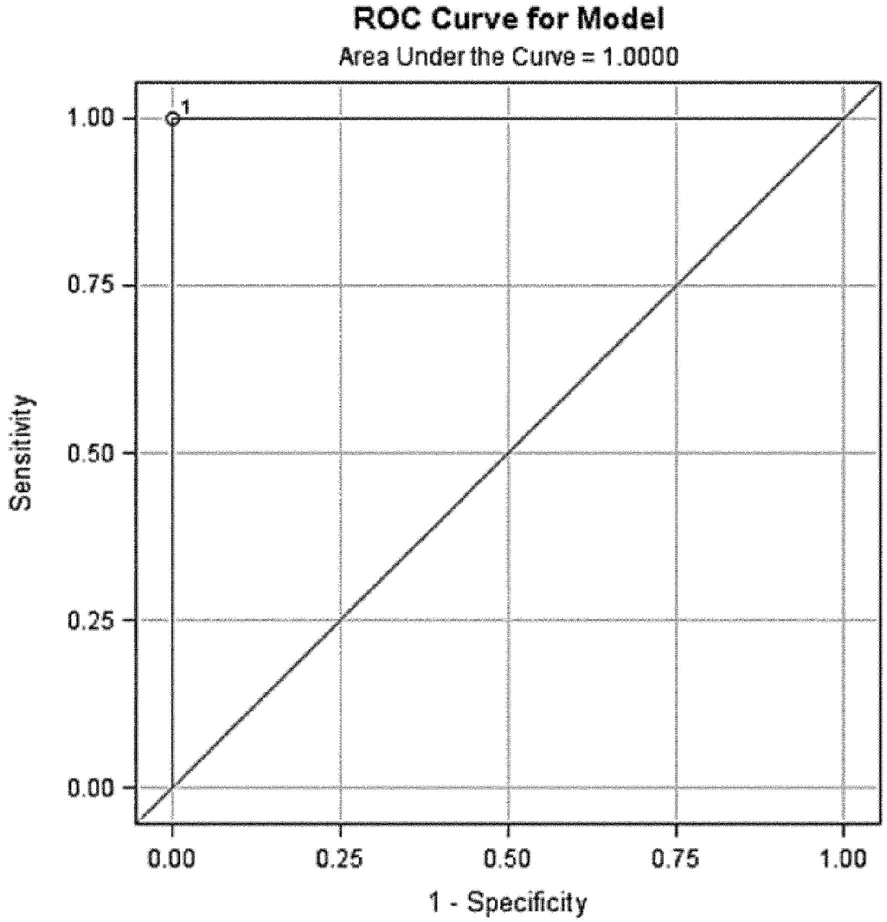


Fig.56

Recurrence score = exp( y)/(1 + exp( y)); in which y

$$y = 4.7039 + 18.0643 \times (hsa\text{-}miR\text{-}363^{*}) - 2.7715 \times (hsa\text{-}miR\text{-}1255b) + 44.9416 \times (hsa\text{-}miR\text{-}566)$$
$$+ 38.4025 \times (hsa\text{-}miR\text{-}1265) - 16.3826 \times (hsa\text{-}mir\text{-}127\text{-}5p) - 19.9281 \times (hsa\text{-}mir\text{-}338\text{-}5p)$$
$$+ 6.6695 \times (hsa\text{-}mir\text{-}550a^{*}) + 13.2860 \times (hsa\text{-}mir\text{-}588) - 1.3648 \times (hsa\text{-}mir\text{-}651) - 29.2228 \times (hsa\text{-}mir\text{-}518b)$$
$$- 2.6360 \times (hsa\text{-}mir\text{-}629) - 3.3134 \times (hsa\text{-}mir\text{-}885\text{-}5p) - 4.9860 \times (hsa\text{-}mir\text{-}139\text{-}3p)$$
$$+ 4.2604 \times (hsa\text{-}mir\text{-}1224\text{-}5p) - 3.7123 \times (hsa\text{-}mir\text{-}655)$$

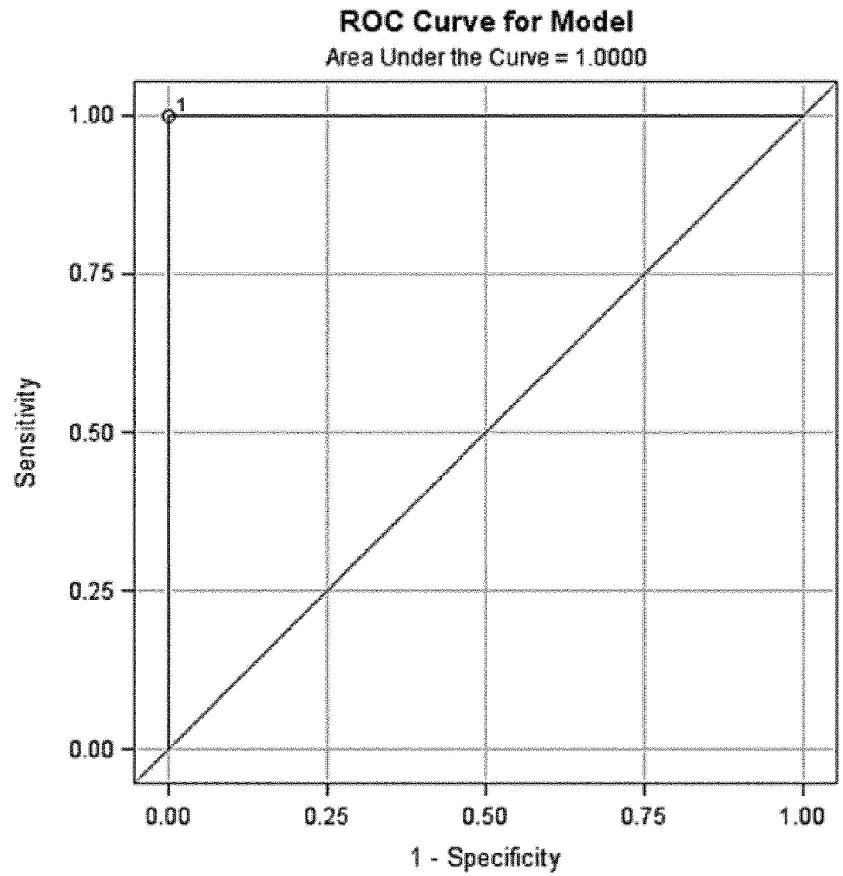


Fig.57

Recurrence score = exp( y)/(1 + exp( y)); in which y

$$y = 1.4780 + 42.1028 \times (hsa\text{-}miR\text{-}363^{*}) - 15.1928 \times (hsa\text{-}miR\text{-}1255b) + 51.9263 \times (hsa\text{-}miR\text{-}566) + 14.9704 \times (hsa\text{-}mir\text{-}651) - 11.2589 \times (hsa\text{-}mir\text{-}518b) - 2.0037 \times (hsa\text{-}mir\text{-}629) - 1.3359 \times (hsa\text{-}mir\text{-}885\text{-}5p) + 10.3658 \times (hsa\text{-}mir\text{-}139\text{-}3p) - 0.7143 \times (hsa\text{-}mir\text{-}1224\text{-}5p) - 19.3261 \times (hsa\text{-}mir\text{-}655)$$

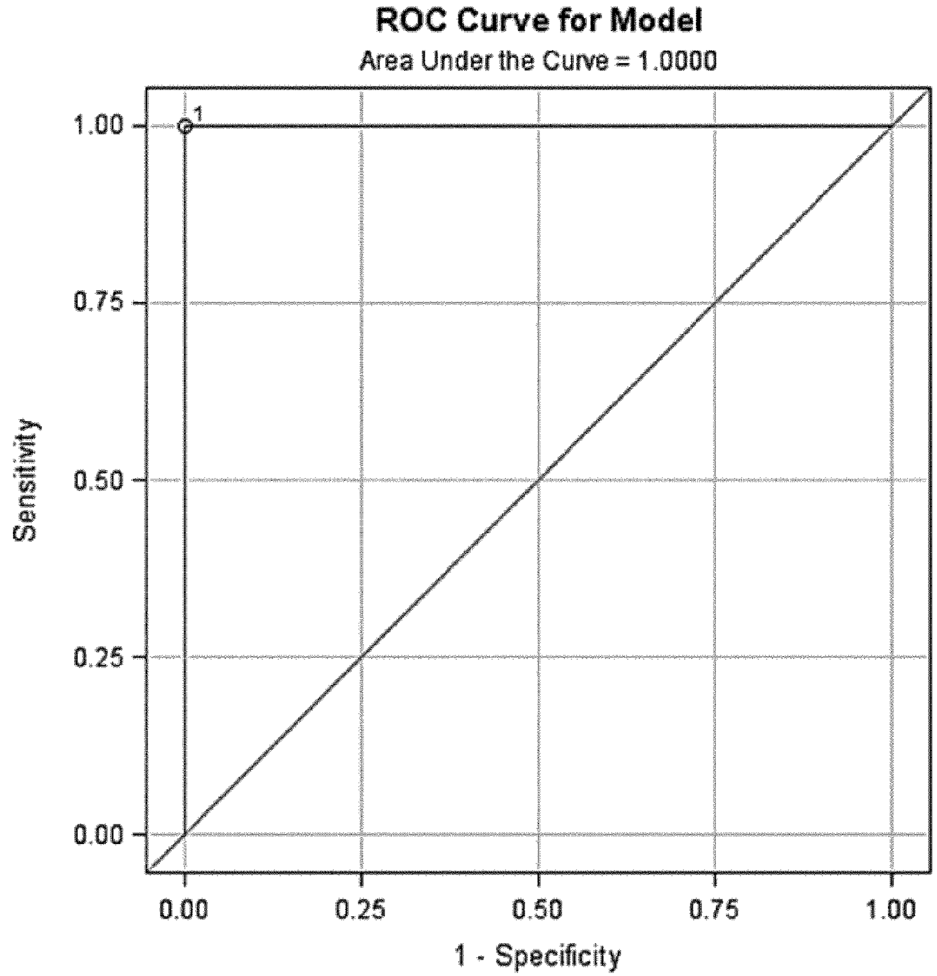


Fig.58

Recurrence score = exp( y)/(1 + exp( y)); in which y

$$y = -11.6353 - 8.4203 \times (hsa\text{-}miR\text{-}363^{*}) + 9.7013 \times (hsa\text{-}miR\text{-}1255b) + 87.6723 \times (hsa\text{-}miR\text{-}566) + 52.7557 \times (hsa\text{-}miR\text{-}1265) - 11.0997 \times (hsa\text{-}mir\text{-}127\text{-}5p) - 13.0881 \times (hsa\text{-}mir\text{-}338\text{-}5p) - 14.9692 \times (hsa\text{-}mir\text{-}655) + 6.4446 \times (hsa\text{-}mir\text{-}1290) - 0.0665 \times (hsa\text{-}mir\text{-}450\text{-}5p)$$

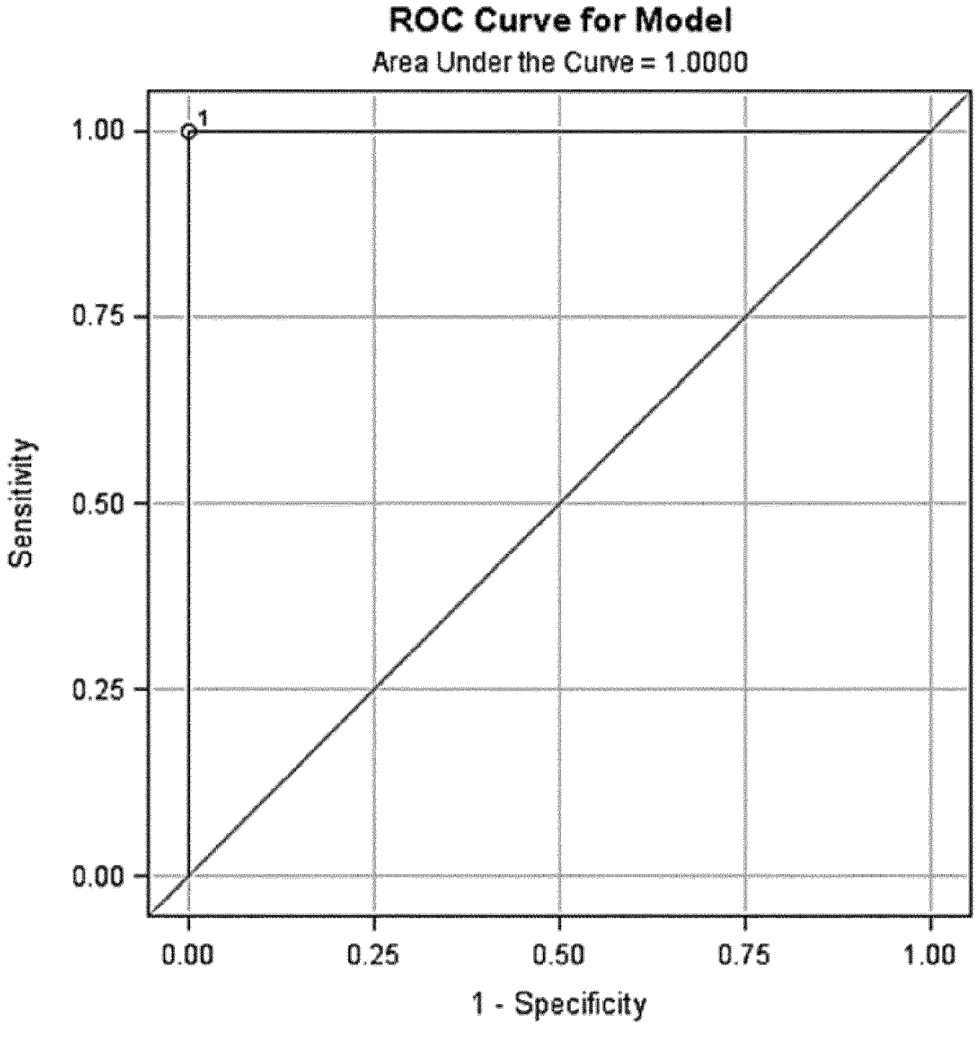


Fig.59

Recurrence score = exp( y)/(1 + exp( y)); in which y

$$y = -8.9870 - 9.6311 \times (hsa\text{-}mir\text{-}550a^{*}) + 34.8785 \times (hsa\text{-}mir\text{-}588) - 32.4082 \times (hsa\text{-}mir\text{-}518b) + 5.2230 \times (hsa\text{-}mir\text{-}629) - 7.6956 \times (hsa\text{-}mir\text{-}885\text{-}5p) + 13.0274 \times (hsa\text{-}mir\text{-}139\text{-}3p) - 4.9384 \times (hsa\text{-}mir\text{-}1224\text{-}5p)$$

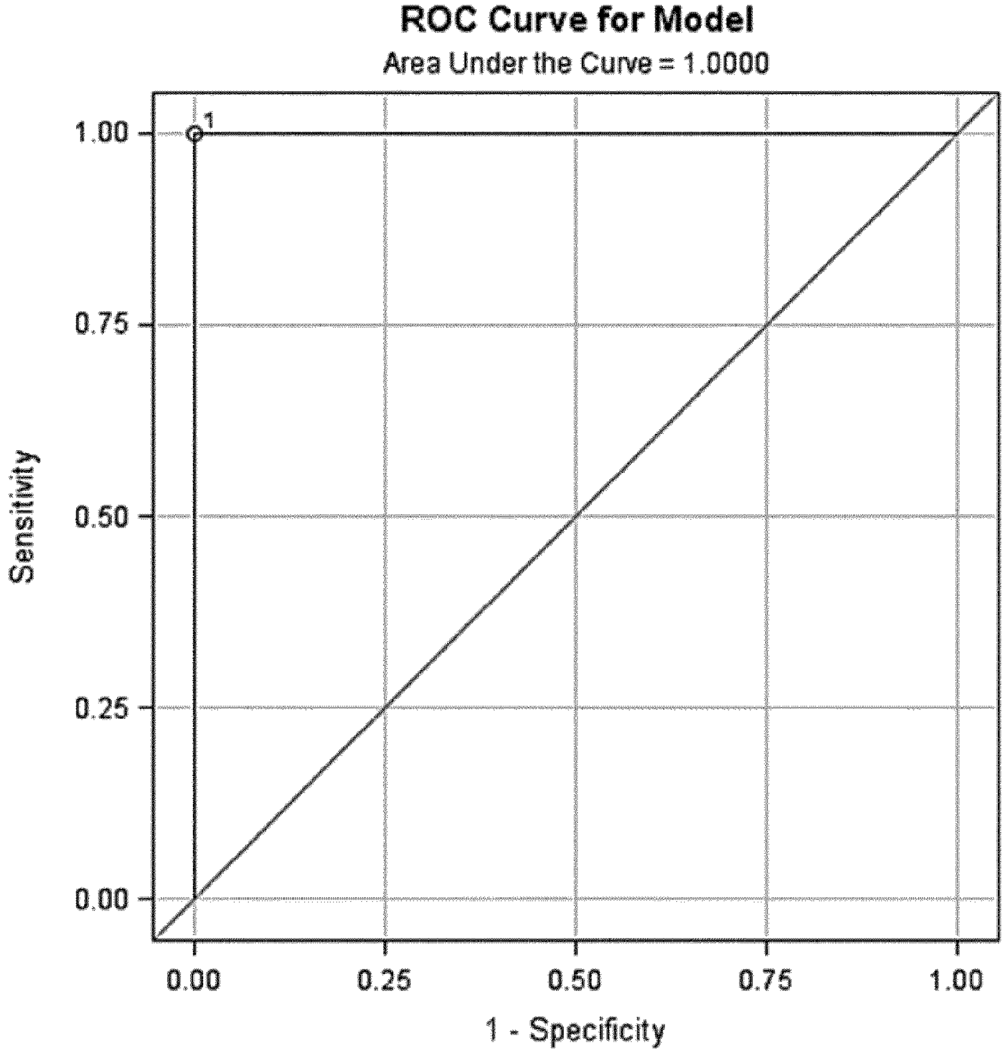


Fig.60

Europäisches Patentamt
European Patent Office
Office européen des brevets

# EUROPEAN SEARCH REPORT

Application Number
EP 14 16 8194

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 2010/058393 A2 (ROSETTA GENOMICS LTD [IL]; MOR RES APPLIC [IL]; LITHWICK YANAI GILA [I) 27 May 2010 (2010-05-27)<br>* claims 1-7, 21-26; examples 2-8 *<br>----- | 1-20 | INV. C12Q1/68<br>ADD. G06F19/18 |
| Y | WO 2009/111643 A2 (ASURAGEN INC [US]; MAMBO ELIZABETH [US]; DAVISON TIMOTHY S [US]; LEBOU)<br>11 September 2009 (2009-09-11)<br>* claims 1-58; examples 2,3; tables 5,6,7 *<br>* the whole document *<br>----- | 1-20 | |
| Y | WO 2009/140670 A2 (VERIDEX LLC [US]; RAPONI MITCH [US]; ARNDT GREG M [US]; DOSSEY LESLEY)<br>19 November 2009 (2009-11-19)<br>* the whole document *<br>----- | 1-20 | |
| Y | EP 2 133 431 A1 (JIANGSU MINGMA BIOTECH CO LTD [CN]) 16 December 2009 (2009-12-16)<br>* paragraphs [0012], [0021], [0022]; claims 28-51 *<br>* the whole document *<br>----- | 16-19 | TECHNICAL FIELDS SEARCHED (IPC)<br>C12Q |
| A | CHANG KAH HOONG ET AL: "MicroRNA expression profiling to identify and validate reference genes for relative quantification in colorectal cancer", BMC CANCER, BIOMED CENTRAL, LONDON, GB, vol. 10, no. 1, 29 April 2010 (2010-04-29), page 173, XP021075012, ISSN: 1471-2407, DOI: 10.1186/1471-2407-10-173<br>* the whole document *<br>----- | 1-20 | |

The present search report has been drawn up for all claims

1

| Place of search | Date of completion of the search | Examiner |
|---|---|---|
| Munich | 5 August 2014 | Schmitt, Anja |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 14 16 8194

This annex lists the patent family members relating to the patent documents cited in the above-mentioned European search report. The members are as contained in the European Patent Office EDP file on
The European Patent Office is in no way liable for these particulars which are merely given for the purpose of information.

05-08-2014

| Patent document cited in search report | Publication date | Patent family member(s) | Publication date |
|---|---|---|---|
| WO 2010058393 A2 | 27-05-2010 | NONE | |
| WO 2009111643 A2 | 11-09-2009 | EP 2268832 A2 | 05-01-2011 |
| | | US 2009233297 A1 | 17-09-2009 |
| | | US 2013029874 A1 | 31-01-2013 |
| | | WO 2009111643 A2 | 11-09-2009 |
| WO 2009140670 A2 | 19-11-2009 | CA 2725477 A1 | 19-11-2009 |
| | | EP 2283161 A2 | 16-02-2011 |
| | | JP 2011520454 A | 21-07-2011 |
| | | KR 20110015013 A | 14-02-2011 |
| | | WO 2009140670 A2 | 19-11-2009 |
| EP 2133431 A1 | 16-12-2009 | CN 101424640 A | 06-05-2009 |
| | | DK 2133431 T3 | 05-11-2012 |
| | | EP 2133431 A1 | 16-12-2009 |
| | | IL 195324 A | 24-03-2013 |
| | | US 2010173288 A1 | 08-07-2010 |
| | | US 2014121133 A1 | 01-05-2014 |
| | | WO 2009055979 A1 | 07-05-2009 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 12733946 A
- US 61432468 A


### Non-patent literature cited in the description


- **ASTLER V B ; COLLER F A.** *Ann Surg,* 1954, vol. 139, 846-52
- AJCC Cancer Staging Manual. Springer-Verlag, 2002
- AJCC Cancer Staging Manual. Springer, 2002
- **AUSUBEL et al.** Current Protocols of Molecular Biology. John Wiley and Sons, 1997
- **CHOMCZYNSKI ; SACCHI.** Single-step method of RNA isolation by acid guanidinium thiocyanate-phenol-chloroform extraction. *Anal Biochem,* April 1987, vol. 162 (1), 156-159
- **BARTEL, D.P.** MicroRNAs: genomics, biogenesis, mechanism, and function. *Cell,* 2004, vol. 116, 281-297
- **AMBROS, V.** The functions of animal microRNAs. *Nature,* 2004, vol. 431, 350-355
- **WEIS et al.** *Trends in Genetics,* 1992, vol. 8, 263-264
- **ALLAWI et al.** Quantitation of microRNAs using a modified Invader assay. *RNA,* 2004, vol. 10, 1309-1322
- **BARAD et al.** MicroRNA expression detected by oligonucleotide microarrays: system establishment and expression profiling in human tissues. *Genome Res,* 2004, vol. 14, 2486-2494
- **MISKA et al.** Microarray analysis of microRNA expression in the developing mammalian brain. *Genome Biol,* 2004, vol. 5, R68
- **BABAK et al.** Probing microRNAs with microarrays: tissue specificity and functional interference. *RNA,* 2004, vol. 10, 1813-1819
- **SCHMITTGEN et al.** A high-throughput method to monitor the expression of microRNA precursors. *Nucleic Acids Res,* 2004, vol. 32, e43
- **HELD et al.** *Genome Research,* 1996, vol. 6, 986-994
- **SCHENA et al.** *Proc. Natl. Acad. Sci. USA,* 1996, vol. 93 (2), 106-149
- **WILCOXON.** Individual comparisons by ranking methods. *Biometrics Bulletin,* 1945, vol. 1 (6), 80-83
- **SIEGEL.** Non-parametric statistics for behavioral sciences. McGraw-Hill, 1956, 75-83
- **KAPLAN ; MEIER.** Nonparametric estimation from incomplete observations. *J. Amer Statist Assn,* 1958, vol. 53, 457-481
- **COX.** Regression Models and Life-Tables. *J of the Royal Stat Soc, Series B (Methodological),* 1972, vol. 34 (2), 187-220